# FASCICULE DE BREVET EUROPEEN

(11) **EP 2 029 596 B1**
(45) Date de publication et mention de la délivrance du brevet: **24.02.2010**
(21) Numéro de dépôt: 07765943.1
(22) Date de dépôt: 23.05.2007
(51) Int. Cl.: C07D 471/08, C07D 211/18, C07D 207/09, C07D 215/08, C07D 213/54, A61K 31/4709, A61P 3/04, A61P 3/10, A61P 9/10, A61P 19/10, A61P 25/28

(54) **2-ALCOXY-3,4,5-TRIHYDROXY-ALKYLAMIDE-BENZOTHIAZEPINE, LEUR PRÉPARATION, COMPOSITIONS LES CONTENANT ET UTILISATION**
2-ALKOXY-3,4,5-TRIHYDROXY-ALKYLAMID-BENZOTHIAZEPINE, IHRE HERSTELLUNG UND VERWENDUNG SOWIE DIESE ENTHALTENDE ZUSAMMENSETZUNGEN
2-ALKOXY-3,4,5-TRIHYDROXY-ALKYLAMIDE-BENZOTHIAZEPINES, THE PREPARATION AND USE THEREOF, AND COMPOSITIONS CONTAINING THE SAME

(30) Priorité: 24.05.2006 FR 0604735
(43) Date de publication de la demande: 04.03.2009
(73) Titulaire: Sanofi-Aventis, 75013 Paris (FR)
(72) Inventeur: ZHANG, Jidong, F-92160 Antony (FR); BENEDETTI, Yannick, F-92160 Antony (FR); NARDI, Frédérico, F-92160 Antony (FR); COMMERCON, Alain, F-92160 Antony (FR)
(74) Mandataire: Le Pennec, Magali
(86) Numéro de dépôt international: PCT/FR2007/000867
(87) Numéro de publication internationale: WO 2007/135294

(56) Documents cités:
- WO-A-01/85697
- WO-A-98/35941
- WO-A-2005/014574
- WO-A2-2006/056696
- US-A- 5 283 241
- US-B1- 6 239 127
- KINDER F R ET AL: "SYNTHESIS AND ANTITUMOR ACTIVITY OF ESTER-MODIFIED ANALOGUES OF BENGAMIDE B" JOURNAL OF MEDICINAL CHEMISTRY, AMERICAN CHEMICAL SOCIETY. WASHINGTON, US, vol. 44, 2001, pages 3692-3699, XP002310388 ISSN: 0022-2623
- MORTON G C ET AL: "Novel solid-phase synthesis of 1,5-benzothiazepine-4-one derivatives" TETRAHEDRON LETTERS, ELSEVIER, AMSTERDAM, NL, vol. 41, no. 17, avril 2000 (2000-04), pages 3029-3033, XP004196718 ISSN: 0040-4039
- JOURNAL OF MEDICINAL CHEMISTRY., vol. 28, no. 10, 1985, pages 1517-1521, XP002412023 USAMERICAN CHEMICAL SOCIETY. WASHINGTON.

## Description

La présente invention concerne notamment des 2-alcoxy-3,4,5-trihydroxy-alkylamide-benzothiazépines, leur préparation, des compositions les contenant, et leur utilisation comme médicament.

Plus particulièrement, et selon un premier aspect, l'invention concerne des 2-alcoxy-3,4,5-trihydroxy-alkylamide-benzothiazépines utiles comme agents anticancéreux.

Des 2-méthoxy-3,4,5-trihydroxy-alkylamides ont été décrits dans US 6239127, US 20010044433 A1, WO 01/85697, WO 00/29382, US 5283241, WO98/35941, W02005/014574, US 4831135, EP 687673, et US 2002128474 A1. Ces documents divulguent essentiellement des analogues et dérivés de bengamide, un produit naturel isolé d'une éponge marine, *Jaspis coriacea* porteurs d'un noyau azépinone.

Ces mêmes produits ont été décrits dans la littérature : J. Org. Chem. (1986), 51 (23), 4494-7 ; J. Org. Chem. (2001), 66(5), 1733-41 ; J. Med. Chem. 2001, 44, 3692-9 ; Tetrahedron Letters (2000), 41 (17), 3029-3033 ; J. Med. Chem. 1985, 28 (10) 1517-1521.

Le problème que se propose de résoudre la présente invention est d'obtenir de nouveaux produits présentant une activité anticancéreuse. En plus du maintien d'une activité anticancéreuse, certains de ces nouveaux produits peuvent également présenter des propriétés avantageuses en relation avec leur activité pharmacologique, telle que leur pharmacocinétique, biodisponibilité, solubilité, stabilité, toxicité, absorption ou métabolisme.

Le document intercalaire WO2006/056696 décrit des bengamide porteurs d'un noyau benzothiazépinon de la même famille qui sont exclus de la présente invention par un disclaimer.

La présente invention a pour objet les produits répondant à la formule générale (I) suivante : 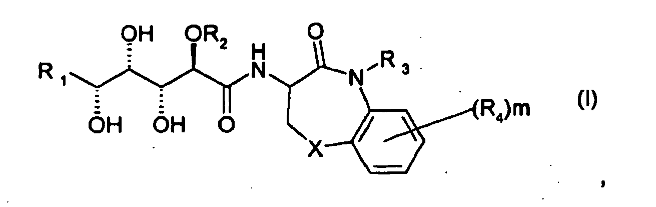 dans laquelle :
a) R₁₂ est le groupe -C(R₆)=C(R₇)(R₈) dans lequel R₆, R₇, et R₈ sont indépendamment sélectionnés parmi H, (C1-C6)alkyle, cycloalkyle, hétérocyclyle, aryle, hétéroaryle;
b) R₂ est méthyle;
c) R₃ est sélectionné dans le groupe constitué par H, méthyle, un groupe phénylméthyle ou un groupe (3,5-difluoro-phényl)-méthyle;
d) R₄ est indépendamment sélectionné dans le groupe constitué par H, F, Cl, Br, phényle, cyanophényle, trifluorométhyle, méthoxy, phénoxy ou bien 2 substituants R₄, liés à 2 carbones adjacents du phényle, forment ensemble un cycle pyrazine.
e) m a pour valeur 0, 1, 2, 3, ou 4 ;
f) X est choisi parmi S ou SO;
à la condition que lorsque R₁ est (E) -CH=CH-C(CH₃)₃, R₂ est méthyle, X est S et m est 0, alors R₃ n'est pas un atome d'hydrogène, un groupe (3,5-difluoro-phényl)-méthyle ou un groupe -CH₂-CH=CH₂.

Plus préférentiellement, R₁ est choisi parmi (E) -CH=CH-CH(CH₃)(C₂H₅), (E) - CH=CH-CH(CH₃)₂, (E) -CH=CH-C(CH₃)₃, ou encore parmi (E) -C(CH₃)=CH-CH(CH₃)(C₂H₅), (E) -C(CH₃)=CH-CH(CH₃)₂, et (E) -C(CH₃)=CH-C(CH₃)₃.

Plus préférentiellement, R1 est choisi parmi (E) -CH=CH-C₅H₉, (E) -CH=CH-C₆H₁₁, (E) -CH=CH-(CH₂)₃₋CH₃, (E) -CH=CH-C₆H₅ où le phényle est éventuellement substitué par un atome de fluor.

Parmi les objets de la présente invention, un premier groupe est **caractérisé en ce que** X est S. Un second groupe est **caractérisé en ce que** X est SO.

Parmi les objets de la présente invention, un troisième groupe est **caractérisé en ce que** R₃ est indépendamment choisi parmi : méthyle, un groupe phénylméthyle ou un groupe (3,5-difluoro-phényl)-méthyle. Un quatrième groupe est **caractérisé en ce que** R₃ est H.

Un cinquième groupe est **caractérisé en ce que** m est 0.

De manière préférée, l'invention concerne les produits exemplifiés dans le tableau 1.

Selon un autre aspect, l'invention concerne les procédés de préparation des produits de formule générale (I) ou (I'). Les produits de formule générale (I') sont des précurseurs, éventuellement actifs, des produits de formule générale (I). Les produits de formule générale (I) sont obtenus à partir des produits de formule générale (I') par des procédés décrits ou par une ou plusieurs réactions classiques pour l'homme du métier telle que par exemple une cyclopropanation, une oxydation ou une séparation chirale. Notamment, les produits des exemples 1 et 2 sont obtenus par oxydation du produit 6 de formule générale (I').

Les produits de formule générale (I) ou (I') peuvent être obtenus par hydrolyse d'un produit de formule générale (II) : 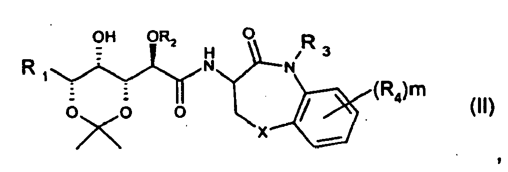 dans lequel R₁, R₂, R₃, R₄, X et m sont tels que définis précédemment,.

Les produits de formule générale (II) peuvent être obtenus par réaction d'un produit de formule générale (III) : 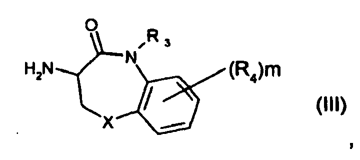 dans lequel R₃, R₄ , X et m sont tels que définis précédemment, avec un produit de formule générale (IV) : 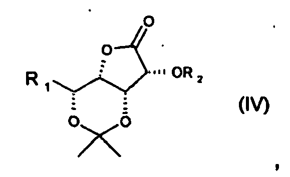 dans lequel R₁, R₂ sont tels que définis précédemment.

Les produits de formule générale (I) ou (I') peuvent également être obtenus par réaction d'un produit de formule générale (III) tel que défini précédemment, avec un produit de formule générale (V) : 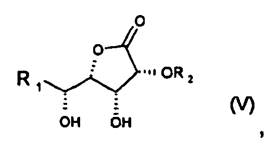 dans lequel R₁, R₂ sont tels que définis précédemment.

Les produits de formule générale (V) peuvent être obtenus par hydrolyse d'un produit de formule générale (IV) : 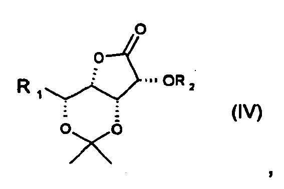 dans lequel R₁, R₂ sont tels que définis précédemment. Des produits de formule générale (V) pour lesquels R₁ représente -CH=CH-R'₁ peuvent encore être obtenus par hydrolyse d'un produit de formule générale (VII) : 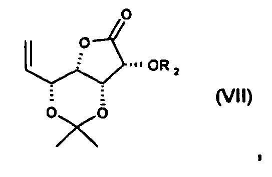 dans lequel R₂ est tel que défini précédemment, afin d'obtenir un produit de formule générale (VI) : 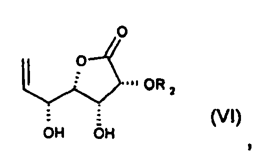 dans lequel R₂ est tel que défini précédemment, qui subit une métathèse afin d'obtenir un produit de formule générale (V) : 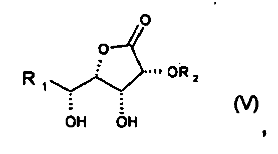 pour lesquels R₁ représente -CH=CH-R'₁ et R'₁ représente un groupe (C1-C6)alkyle, cycloalkyle, hétérocyclyle, aryle ou hétéroaryle.

Les produits de formule générale (VII) peuvent être obtenus par double déshydratation d'un produit de formule générale (VIII) : 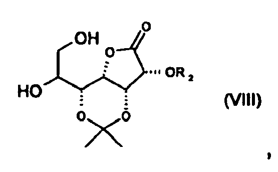 dans lequel R₂ est tel que défini précédemment.

Les produits de formule générale (I') et (II) tels que définis précédemment, à l'exception de ceux pour lesquels R₁ est (E) -CH=CH-C(CH₃)₃, R₂ est méthyle, X est S, m est 0, et R₃ est un atome d'hydrogène, un groupe (3,5-difluoro-phényl)-méthyle ou un groupe -CH₂-CH=CH₂, sont un objet de la présente invention.

Les produits de formule générale (III) pour lesquels X est S, R₃ est H, méthyle, phénylméthyle ou (3,5-difluorophényl)-méthyle et R₄ est F, Cl, Br, phényle, cyanophényle, trifluorométhyle, méthoxy ou phénoxy, ou bien m a pour valeur 0, à l'exception de ceux pour lesquels X est S, m est 0, et R₃ est un atome d'hydrogène ou un groupe (3,5-difluoro-phényl)-méthyle et à l'exception de ceux pour lesquels X est S, R₃ est phénylméthyle et R4 est trifluorométhyle ou méthoxy, sont un objet de la présente invention.

Les produits de formule générale (IV) et (V) pour lesquels R₂ est méthyle et R1 est -(E) -CH=CH-C₅H₉ ou -(E) -CH=CH-C₆H₅ où le phényle est substitué par un atome de fluor sont un objet de la présente invention.

Les produits de formule générale (VI) pour lesquels R₂ est méthyle sont un objet de la présente invention. Les produits de formule générale (VII) pour lesquels R₂ est méthyle sont un objet de la présente invention.

Les produits selon la présente invention peuvent exister à l'état de bases, de sels d'additions avec des acides, de solvats, d'hydrates ou de prodrogues.

Les produits selon l'invention peuvent être sous forme non chirale, ou racémique, ou enrichie en un stéréo-isomère, ou enrichie en un énantiomère ; et peuvent éventuellement être salifiés. Les produits pour lesquels le carbone lié à l'amine exocyclique est de configuration (R) sont préférés.

Un produit conforme à l'invention pourra être utilisé pour la fabrication d'un médicament utile pour prévenir ou traiter un état pathologique, en particulier un cancer.

Les produits de la présente invention peuvent également être utilisés pour la fabrication d'un médicament utile pour prévenir ou traiter un état pathologique dans lequel une néovascularisation ou angiogenèse se fait de façon inappropriée c'est-à-dire dans les cancers en général et dans des cancers particuliers tels que le sarcome de Kaposi ou l'hémoangiome infantile, mais aussi dans l'arthrite rhumatoïde, l'osthéoarthrite et/ou ses douleurs associées, les maladies inflammatoires de l'intestin telle que la recto-colite hémorragique ou la maladie de Crohn's, les pathologies de l'oeil telle que la dégénérescence maculaire liée à l'âge, les rétinopathies diabétiques, l'inflammation chronique, le psoriasis.

L'angiogenèse est un processus de génération de nouveaux vaisseaux capillaires à partir des vaisseaux pré-existants. L'angiogenèse tumorale (formation de néovaisseaux sanguins), indispensable à la croissance tumorale, est également un des facteurs essentiels de la dissémination métastatique (Oncogene. 2003 May 19;22(20):3172-9 ; Nat Med. 1995 Jan;1(1):27-31.).

La présente invention concerne aussi les compositions thérapeutiques contenant un composé selon l'invention, en association avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront de préférence injectables et, de ce fait, auront une formulation acceptable pour une telle utilisation.

Des voies d'administration par injection acceptables incluent les voies intraveineuse, intra-péritonéale, intramusculaire, et sous cutanée, la voie intraveineuse étant préférée.

La dose administrée des composés de l'invention sera adaptée par le praticien en fonction de la voie d'administration du patient et de l'état de ce dernier.

Les composés de la présente invention peuvent être administrés seuls ou en mélange avec d'autres anticancéreux. Parmi les associations possibles on peut citer :
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoides (paclitaxel et docétaxel)
- les anthracyclines comme notamment la doxorubicine, la daunorubicine, l'idarubicine, l'épirubicine, la mitoxantrone, la losoxantrone
- les topoisomérases des groupes I et II telles que l'étoposide, le teniposide, l'amsacrine, l'irinotecan, le topotecan et le tomudex
- les fluoropyrimidines telles que le 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine telles que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques
- les agents antivasculaires tels que les dérivés de la combretastatine ou de la colchicine et leurs prodrogues.
- les agents antivasculaires tels que les dérivés de la combretastatine, par exemple la CA4P, des chalcones ou de la colchicine, par exemple le ZD6126, et leurs prodrogues.
- Les inhibiteurs de kinases tels que l'ertonilib ou l'imatinib.
- Les agents biothérapeutiques comme les anticorps tels que le rituximab, le bevacizumab, le cetuximab, le trastuzumab ou l'alemtuzumab.
- Les inhibiteurs du protéasome tel que le bortesomib.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

### Définitions

Le terme « halogène » fait référence à un élément choisi parmi F, CI, Br, et I.

Le terme « alkyle » fait référence à un substituant hydrocarboné saturé, linéaire ou ramifié, ayant de 1 à 12 atomes de carbone. Les substituants méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, hexyle, 1-méthylpentyle, 2-méthylpentyle, 1-éthylbutyle, 2-éthylbutyle, 3,3-diméthylbutyle, heptyle, 1-éthylpentyle, octyle, nonyle, décyle, undécyle, et dodécyle sont des exemples de substituant alkyle.

Le terme « alcényle » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alcynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tetrahydronapht-5-yle ; et 1,2,3,4-tetrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle ; oxadiazolyle ; thiadiazolyle ; tetrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle; cyclooctyle; bicyclo[2.2.2]octyle ; adamantyle ; et perhydronapthyte sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Concernant le phényle fusionné, lorsque m a pour valeur zéro on entend qu'il s'agit d'un phényle non substitué (ou substitué par 4 atomes d'hydrogène), et lorsque m a pour valeur 1, 2, 3 ou 4, on entend qu'1, 2, 3 ou 4 atomes d'hydrogènes sont substitués par un substituant R₄.

Les avantages de l'invention seront plus particulièrement illustrés par les exemples suivants :

### Abréviations :

**Ac** acétate ; **Bn** benzyle ; **°C** degré Celsius ; **cat.** catalyseur; **CCM** chromatographie sur couche mince ; **CCP** chromatographie sur colonne préparative ; **cm** centimètre ; 8 déplacement chimique ; **d** doublet ; **dd** doublet de doublets ; **DMF** diméthylformamide ; **DMSO-d⁶** diméthylsulfoxyde deutéré ; **dt** doublet de triplets ; **éq.** équivalent ; **ES+/-** electrospray (modes positif / négatif) ; **Et** éthyle ; **g** gramme ; **h** heure ; **Hz** hertz ; **IC₅₀** constante d'inhibition à 50% d'activité ; **iPr** isopropyle ; **j,** jour ; **J** constante de couplage ; **LCMS** chromatographie liquide couplée à une spectrométrie de masse; **m** multiplet ; **Me** méthyle ; **mg** milligramme ; **MHz** mégahertz ; **mL** millilitre ; **µL** microlitre ; **mm** millimètre ; µ**m** micromètre ; mmol millimole ; **mn** minute ; **N** mol.L⁻¹; **PF** point de fusion ; **Ph** phényle ; ppm parties par million ; **q** quadruplet ; **Rdt** rendement ; **Rf** rapport frontal ; **RMN ¹H** résonance magnétique nucléaire du proton ; s singulet ; **sl** singulet large ; **t** triplet ; **TA** température ambiante; **tBu** tertiobutyle ; **TFA** acide trifluoroacétique ; **THF** tetrahydrofuranne ; **t_{R}** temps de rétention ; **U.V.** ultraviolet ; **V** volt.

**Ex1:** N-[(1R,3R)-5-(3,5-difluoro-benzyl)-1,4-dioxo-2,3,4,5-tetrahydro-1,5-benzothiazépin-3-yl]-((6E)-(2R,3R,4S,5R)-3,4,5-trihydroxy-2-méthoxy-8,8-diméthyl-non-6-énamide et **Ex2**: N-[(1S,3R)-5-(3,5-difluoro-benzyl)-1,4-dioxo-2,3,4,5-tetrahydro-1,5-benzothiazépin-3-yl]-((6E)-(2R,3R,4S,5R)-3,4,5-trihydroxy-2-méthoxy-8,8-diméthyl-non-6-énamide

### Etape 1: Préparation du (3R)-[5-(3,5-difluoro-benzyl)-4-oxo-2,3,4,5-tetrahydro-1,5-benzothiazépin-3-yl]-carbamate de tert-butyle (2)

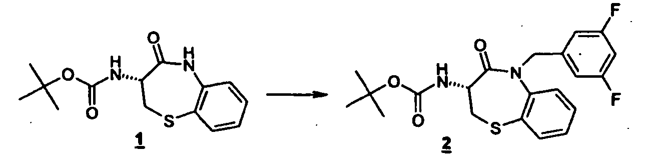

Dans un ballon de 100 mL, sous agitation et atmosphère d'argon, contenant 40 mL de THF et 1,3 g de **1** (4,4 mmol), on introduit à TA 177 mg d'hydrure de sodium en suspension à 60% dans l'huile (4,42 mmol). On agite le milieu pendant 1h, puis ajoute 1,8 g (8,83 mmol) de bromure de 3,5-difluorobenzyl. On laisse le milieu sous agitation pendant une nuit. On ajoute 100 mL d'AcOEt, lave la phase organique avec 2 fois 100 mL d'eau. La phase organique est séchée sur MgSO₄, filtrée puis évaporée à sec. On obtient 3 g d'une huile jaune, qui donne après chromatographie sur une cartouche de silice (120 g) en éluant avec un mélange Heptane/AcOEt (*en gradient : AcOEt 12 à 100%*) 1,59 g de produit **2**.
RMN ¹H (400 MHz, DMSO-d₆), δ(ppm): 1,35 (s, 9H) ; 3,11 (t, J = 11,5 Hz, 1H); 3,48 (dd, J = 7,0 et 11,5 Hz, 1H); 4,15 (m, 1H) ; 5,03 (d, J = 16,0 Hz, 1H) ; 5,13 (d, J = 16,0 Hz, 1H) ; de 6,98 à 7,10 (m, 3H) ; 7,28 (t large, J = 8,0 Hz, 1H) ; 7,39 (d large, J = 8,0 Hz, 1H) ; de 7,47 à 7,53 (m, 2H) ; 7,63 (d large, J = 7,5 Hz, 1H).

### Etape 2: Préparation du chlorhydrate de (3R)-3-amino-5-(3,5-difluoro.benzyl)-2,3-dihvdro-5H-1,5-benzothiazéain-4-one (3)

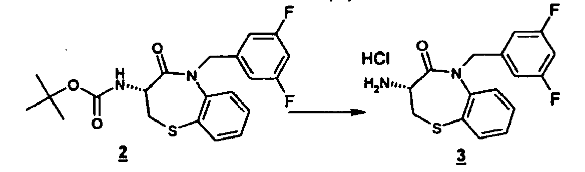

On reprend 1,59 g de **2** (3,78 mmol) dans un ballon de 100 mL et on ajoute 25 mL d'une solution d'acide chlorhydrique dans le dioxane (4M). On agite le milieu pendant 5h à TA sous argon. Après évaporation du solvant, on obtient 1,44 g d'amine **3** sous forme de chlorhydrate qui est utilisé directement pour l'étape suivante.
RMN ¹H (400 MHz, DMSO-d₆), δ(ppm): 3,25 (t, J = 11,5 Hz, 1H) ; 3,75 (dd, J = 7,0 et 11,5 Hz, 1H) ; 3,94 (dd, J = 7,0 et 11,5 Hz, 1H) ; 5,09 (d, J = 16,0 Hz, 1H) ; 5,24 (d, J = 16,0 Hz, 1H) ; de 6,97 à 7,15 (m, 3H) ; 7,33 (m, 1H) ; de 7,48 à 7,59 (m, 2H) ; 7,68 (dd, J = 1,5 et 7,5 Hz, 1H) ; 8,63 (s large, 3H).

### Etape 3: Préparation du (3R,4R,5S)-4-hydroxy-5-((2E)-(2R)-1-hydroxy-4,4-diméthyl-pent-2-ényl)-3-méthoxy-dihydro-furan-2-one (5)

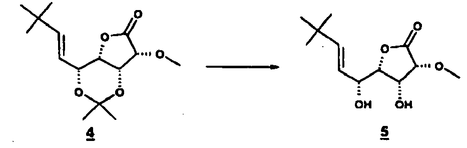

Dans un ballon de 250 mL conteant 40 mL d'eau et 3,6 g de **4** (qui peut être préparé selon les modes opératoires décrites dans Org. Process Res. Dev. 2003, *7*(6), 856-865) en suspension, on ajoute 17 mL de TFA en solution dans 10 mL d'eau. On agite le milieu pendant 1,5 h à TA puis dilue le milieu avec 290 mL d'eau, congèle et lyophilise. On obtient 4 g d'une huile qui cristallise dans 20 mL d'éther isopropylique à TA. Après essorage, lavage à l'éther isopropylique, et séchage sous vide à 40°C, on obtient 2,46 g de produit attendu **5** (cristaux blanc).
PF : 123°C.
IC : m/z = 262 MNH₄⁺.
RMN ¹H (400 MHz, DMSO-d₆), δ(ppm): 1,00 (s, 9H) ; 3,41 (s, 3H) ; 3,93 (dd, J = 2,5 et 9,0 Hz, 1H) ; de 4,22 à 4,31 (m, 3H) ; 5,19 (d, J = 5,0 Hz, 1H) ; 5,42 (dd, J = 5,0 et 16,0 Hz, 1H) ; 5,43 (d, J = 4,5 Hz, 1H) ; 5,87 (d, J = 16,0 Hz, 1H).
IR (KBr): 3239; 2964; 2914; 1701; 1499; 1312; 1253; 1047 & 751 cm⁻¹.

### Etape 4: Préparation du N-[(3R)-5-(3,5-difluoro-benzyl)-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide (6)

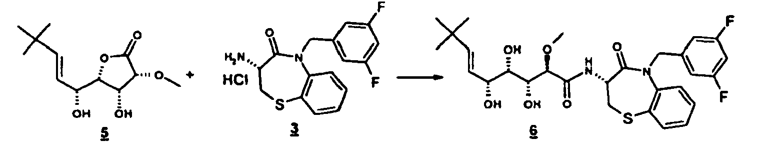

On introduit successivement dans 2,0 mL de THF dans un tube Wheaton, sous agitation et sous atmosphère d'argon, 99 mg de **5** (405 µmol), 362 mg de **3** (1.0 mmol), 135 mg de 2-éthylhexanoate de sodium (0,81 mmol). On agite à TA pendant 24 h. On ajoute 3 mL d'acétate d'éthyle au milieu réactionnel. On lave successivement avec 3 mL d'une solution de HCl (1N), puis 5 mL d'une solution saturée de NaHCO₃ et 3 mL d'eau. La phase organique est séchée sur sulfate de magnésium anhydre, filtrée puis évaporée à sec. On obtient 300 mg d'un solide marron, qui est chromatographié sur une cartouche de silice (10g, éluant Heptane/AcOEt- en *gradient : AcOEt 25 à 100%*). On recueille 123 mg de produit attendu **6**.
ES : 565(+)=(M+H)(+); 547(+)=(M+H)(+) -H₂O.
RMN ¹H (400 MHz, DMSO-d₆), δ(ppm): 0,95 (s, 9H) ; 3,20 (s, 3H) ; 3,21 (t, J = 12,0 Hz, 1H) ; de 3,32 à 3,47 (m masqué, 1H) ; 3,48 (dd, J = 7,0 et 12,0 Hz, 1H) ; 3,50 (m partiellement masqué, 1H) ; 3,69 (d, J = 8,0 Hz, 1H) ; 3,92 (m, 1H) ; 4,30 (m large, 2H) ; 4,45 (m, 1H) ; 4,54 (d, J = 4,5 Hz, 1H) ; 5,03 (d, J = 16,0 Hz, 1H) ; 5,21 (d, J = 16,0 Hz, 1H) ; 5,28 (dd, J = 7,0 et 16,0 Hz, 1H) ; 5,61 (d large, J = 16,0 Hz, 1H) ; de 7,00 à 7,10 (m, 3H) ; 7,29 (m, 1H) ; de 7,45 à 7,55 (m, 2H) ; 7,61 (dd, J = 1,5 et 7,5Hz, 1H) ; 8,49 (d, J = 8,0 Hz, 1H)

### Etape 4 Préparation de N-[(1R,3R)-5-(3,5-difluoro-benzyl)-1,4-dioxo-2,3,4,5-tetrahydro-1,5-benzothiazépin-3-yl]-((6E)-(2R,3R,4S,5R)-3,4,5-trihydroxy-2-méthoxy-8,8-diméthyl-non-6-énamide (Ex1) et N-[(1S,3R)-5-(3,5-difluoro-benzyl)-1,4-dioxo-2,3,4,5-tetrahydro-1,5-benzothiazépin-3-yl]-((6E)-(2R,3R,4S,5R)-3,4,5-trihydroxy-2-méthoxy-8,8-diméthyl-non-6-énamide (Ex2)

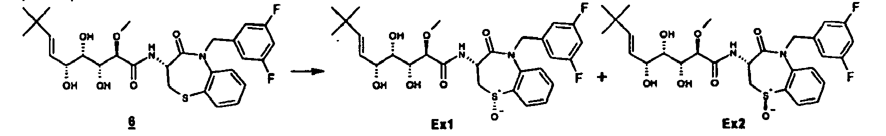

On introduit dans un tube Wheaton, sous agitation et sous atmosphère d'argon, 100 mg de **6** (177 µmol), 1 mL d'hexafluoro-2-propanol et 35 µl d'eau oxygénée à 33%. On maintient l'agitation à TA pendant 5h. On rajoute encore 70 µl d'eau oxygénée, laisse le milieu pendant 24 h. On ajoute 3 mL d'une solution de Na₂SO₃ au milieu réactionnel. On extrait 2 fois avec 3 mL de CH₂Cl₂. La phase organique est séchée sur sulfate de magnésium anhydre, filtrée puis évaporée à sec. On obtient 65 mg de produit brut, qui est chromatographié sur une plaque de silice préparative (*éluant* CH₂Cl₂/MeOH : *90*/*10*). On recueille 11,5 mg de produit attendu **Ex1** et 28,5 mg de produit attendu **Ex2.**
ES: m/z = 579 (M-H)⁻.
**Ex1:□** RMN ¹H (400 MHz, DMSO-d₆), δ(ppm): 0,95 (s, 9H) ; 3,22 (s, 3H) ; de 3,26 à 3,32 (m masqué, 1H) ; 3,38 (dd, J = 11,0 et 14,5 Hz, 1H) ; 3,52 (m, 1H) ; 3,71 (d, J = 8,0 Hz, 1H) ; 3,84 (dd, J = 7,5 et 14,5 Hz, 1H) ; 3,93 (m, 1H) ; 4,25 (d, J = 7,0 Hz, 1H) ; 4,32 (d, J = 5,5 Hz, 1H) ; 4,55 (d, J = 4,5 Hz, 1H) ; 4,67 (m, 1H) ; 4,75 (d, J = 16,5 Hz, 1H) ; 5,24 (d, J = 16,5 Hz, 1H) ; 5,29 (dd, J = 7,0 et 16,0 Hz, 1H) ; 5,61 (d, J = 16,0 Hz, 1H) ; 7,07 (tt, J = 2,0 et 9,5 Hz, 1H) ; 7,15 (m, 2H) ; 7,34 (d, J = 7,5 Hz, 1H) ; 7,42 (t, J = 7,5 Hz, 1H) ; de 7,63 à 7,68 (m, 2H) ; 8,59 (d, J = 7,5 Hz, 1H).
**Ex2:** RMN ¹H (400 MHz, DMSO-d₆), δ(ppm): 0,95 (s, 9H) ; de 3,15 à 3,32 (m masqué, 2H) ; 3,22 (s, 3H) ; 3,49 (m, 1H) ; 3,69 (d, J = 8,0 Hz, 1H) ; 3,92 (m, 1H) ; 4,17 (t, J = 11,5 Hz, 1H) ; 4,30 (m, 2H) ; 4,44 (m, 1H) ; 4,55 (d, J = 4,5 Hz, 1H) ; 4,94 (d, J = 15,5 Hz, 1H) ; 5,19 (d, J = 15,5 Hz, 1H) ; 5,28 (dd, J = 7,0 et 16,0 Hz, 1H) ; 5,61 (d, J = 16,0 Hz, 1H) ; 6,96 (m, 2H) ; 7,13 (tt, J = 2,0 et 9,5 Hz, 1H) ; 7,53 (m, 1H) ; de 7,62 à 7,73 (m, 3H) ; 8,54 (d, J = 7,5 Hz, 1H).

### Ex3: N-[(3R)-5-benzyl-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide

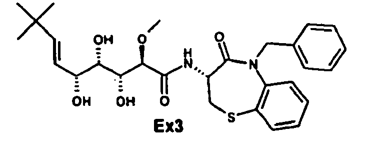

### Etape 1: Préparation du (3R)-[5-benzyl-4-oxo-2,3,4,5-tetrahydro-1,5-benzothiazépin-3-yl]-carbamate de tert-butyle (7)

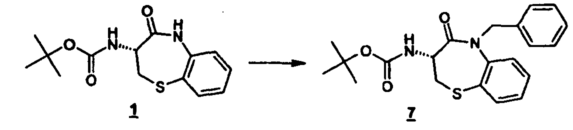

Dans un ballon de 50 mL, sous agitation et atmosphère d'argon, contenant 20 mL de THF et 315 mg de **1** (1,07 mmol), on introduit à TA 42 mg d'hydrure de sodium en suspension à 60% dans l'huile (1,07 mmol). On agite le milieu pendant 1h, et puis ajoute 183 mg (1,07 mmol) de bromure de benzyl. On laisse le milieu sous agitation pendant une nuit, on ajoute 30 mL d'AcOEt, lave la phase organique avec 50 mL d'eau. La phase organique est séchée sur MgSO₄, filtrée puis évaporée à sec. On obtient 0,4 g d'une huile translucide, qui donne après chromatographie sur une cartouche de silice (10 g) en éluant avec un mélange CH₂Cl₂/MeOH (*en gradient : MeOH 1 à 10%*) 0,28 g de produit **7.**
RMN ¹H (300 MHz, DMSO-d₆), δ(ppm):: 1,35 (s, 9H) ; 3,09 (t, J = 11,5 Hz, 1H) ; 3,45 (dd, J = 7,0 et 11,5 Hz, 1H) 4,15 (m, 1H); 4,97 (d, J = 15,5 Hz, 1H) ; 5,20 (d, J = 15,5 Hz, 1H) ; de 7,13 à 7,30 (m, 6H) ; 7,39 (d, J = 8,0 Hz, 1H) ; 7,47 (m, 2H) ; 7,58 (d, J = 7,5 Hz, 1H).

### Etape 2: Préparation du chlorhydrate de (3R)-amino-5-benzyl-2,3-dihydro-5H-1,5-benzothiazépin-4-one (8)

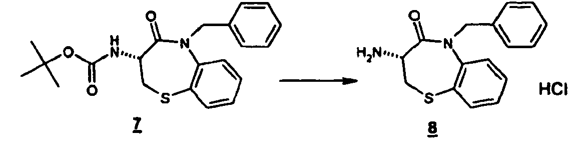

On reprend 0,28 g de **7** (0,73 mmol) dans un ballon de 50 mL et on ajoute 6 mL d'une solution d'acide chlorhydrique dans le dioxane (4M). On agite le milieu pendant 5 h à TA sous argon. Après évaporation du solvant, et trituration avec un mélange de CH₂Cl₂ et éther isopropylique, après essorage on obtient 0,24 g d'amine **8** (meringue jaune) sous forme de chlorhydrate qui est utilisé directement dans l'étape suivante.
RMN ¹H (300 MHz, DMSO-d₆), δ(ppm): 3,22 (t, J = 11,5 Hz, 1H) ; 3,73 (dd, J = 7,0 et 11,5 Hz, 1H) ; 3,91 (dd, J = 7,0 et 11,5 Hz, 1H) ; 4,97 (d, J = 15,5 Hz, 1H) ; 5,33 (d, J = 15,5 Hz, 1H) ; de 7,17 à 7,34 (m, 6H) ; de 7,48 à 7,59 (m, 2H) ; 7,62 (d, J = 7,5 Hz, 1H) ; 8,60 (s large, 3H).
IR ( KBr): 2923; 2613; 1680; 1471; 1453; 1261; 1203; 774; 743; 698; 629 & 458 cm⁻¹

### Etape 3: Préparation du N-((3R)-5-benzyl-4-oxo-2,3,4,5-tetrahydro-1,5-benzothiazépin-3-yl)-(R)-2-[(4R,5S,6R)-6-((1E)-3,3-diméthyl-but-1-ényl)-5-hydroxy-2,2-diméthyl-1,3-dioxinan-4-yl]-2-méthoxy-acétamide (9)

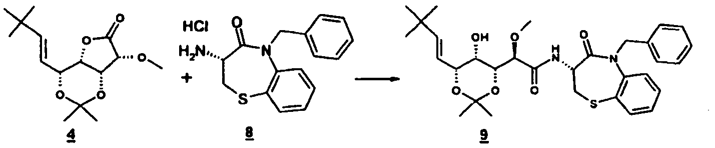

On introduit successivement dans un tube Wheaton, sous agitation et sous atmosphère d'argon, 50 mg de **4** (176 µmol), 113 mg de **8** (0,35 mmol), 73 mg de 2-éthylhexanoate de sodium (0,44 mmol) dans 1 mL de THF. On maintient l'agitation à TA pendant 24h. On ajoute 3 mL d'acétate d'éthyle au milieu réactionnel. On lave successivement avec 3 mL d'une solution de HCl (1N), puis 3 mL d'une solution saturée de NaHCO₃ et 3 mL d'eau. La phase organique est séchée sur sulfate de magnésium anhydre, filtrée puis évaporée à sec. Le brut est chromatographié sur une cartouche de silice (12 g, éluant CH₂Cl₂/MeOH- *en gradient : MeOH 1 à 10%*), on recueille 100 mg de produit attendu **9**.
RMN ¹H (400 MHz, DMSO-d₆), δ(ppm): 0,98 (s, 9H) ; 1,22 (s, 3H) ; 1,28 (s, 3H) ; 3,15 (t, J = 11,5 Hz, 1H) ; de 3,22 à 3,34 (m masqué, 1H) ; 3,23 (s, 3H) ; 3,47 (m, 1H) ; 3,80 (d, J = 9,0 Hz, 1H) ; 3,90 (d, J = 9,0 Hz, 1H) ; 4,25 (d, J = 7,0 Hz, 1H) ; 4,36 (d, J = 8,0 Hz, 1H) ; 4,47 (m, 1H) ; 4,96 (d, J = 15,5 Hz, 1H) ; 5,24 (d, J = 15,5 Hz, 1H) ; 5,44 (dd, J = 7,0 et 16,0 Hz, 1H) ; 5,67 (d, J = 16,0 Hz, 1H) ; de 7,14 à 7,30 (m, 6H) ; 7,52 (m, 2H) ; 7,61 (d, J = 8,0 Hz, 1H) ; 8,52 (d, J = 8,5 Hz, 1H).

### Etape 4: Préparation du N-[(3R)-5-benzyl-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide (Ex3)

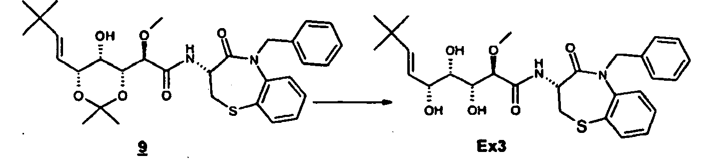

Dans un ballon de 20ml, on mélange 101 mg de **9** (178 µmol) dans 0,85 mL de THF et 1,78 mL d'acide chlorhydrique 1 N, sous agitation et sous argon. L'agitation est maintenue 5 heures à TA. Puis, on refroidit la solution à 0°C. On neutralise à pH 7,0 avec de la soude 1N. On extrait 2 fois avec 5 mL d'AcOEt. Les phases organiques sont réunies, séchées sur sulfate de magnésium et filtrées puis amenées à sec. On obtient 66 mg de produit brut, qui après purification sur une plaque de silice préparative (éluant CH₂Cl₂/MeOH : 90/10) donne 16,5 mg de produit attendu **Ex3.**
IC : m/z = 546 MNH₄⁺. ; m/z = 529 MH⁺.
RMN ¹H (400 MHz, DMSO-d₆), δ(ppm):: 0,95 (s, 9H) ; 3,17 (t partiellement masqué, J = 11,5 Hz, 1H) ; 3,21 (s, 3H) ; de 3,25 à 3,33 (m masqué, 1H) ; de 3,40 à 3,53 (m, 2H) ; 3,69 (d, J = 8,0 Hz, 1H) ; 3,93 (m, 1H) ; 4,32 (m, 2H) ; 4,47 (m, 1H) ; 4,54 (d large, J = 4,5 Hz, 1H) ; 4,94 (d, J = 15,5 Hz, 1H) ; de 5,25 à 5,32 (m, 2H) ; 5,61 (d, J = 16,0 Hz, 1H) ; de 7,14 à 7,30 (m, 6H) ; de 7,45 à 7,54 (m, 2H) ; 7,59 (d, J = 7,5 Hz, 1H) ; 8,40 (d, J = 8,5 Hz, 1H).

### Ex4: N-[(3R)-5-méthyl-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide

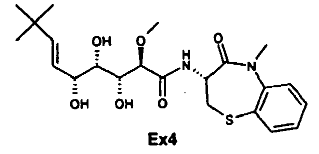

### Etape 1: Préparation du (3R)-[5-méthyl-4-oxo-2,3,4,5-tetrahydro-1,5-benzothiazépin-3-yl]-carbamate de tert-butyle (10)

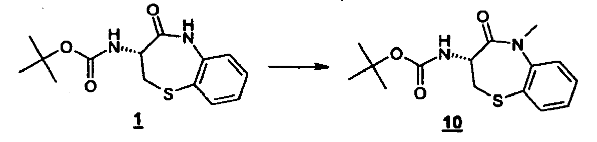

Dans un ballon de 100 mL, sous agitation et atmosphère d'argon, contenant 15 mL de THF et 1,5 g de **1** (5,1 mmol), on introduit à TA 224 mg d'hydrure de sodium en suspension à 60% dans l'huile (5,6 mmol). On agite le milieu pendant 1 h, et puis ajoute 0,80 g (5,6 mmol) d'iodure de méthyle. On laisse le milieu sous agitation pendant 1,5 h, on ajoute 50 mL de CH₂Cl₂, lave la phase organique avec 2 fois 30 mL d'eau et 1 fois 30 ml d'une solution saturée en NaCl. La phase organique est séchée sur MgSO₄, filtrée puis évaporée à sec. Le brut est chromatographié sur une cartouche de silice (120 g) avec un mélange Heptane/AcOEt (*75*/*25*) donnant 1,31 g de produit **10.** PF: 118°C+ /- 2°C.
ES: m/z = 309 MH⁺.
RMN ¹H (400 MHz, DMSO-d₆), δ(ppm): 1,32 (s, 9H) ; 3,01 (t, J = 11,5 Hz, 1H) ; 3,29 (s, 3H) ; 3,41 (dd J = 7,0 et 11,5 Hz, 1H) ; 4,07 (m, 1H) 7,25 (d , J = 9,0 Hz, 1H) ; 7,30 (m, 2H) ; 7,57 (m, 2H) ; 7,63 (d, J = 7,5 Hz, 1H).

### Etape 2: Préparation de chlorhydrate de (3R)-amino-5-méthyl-2,3-dihydro-5H-1,5-benzothiazépin-4-one (11)

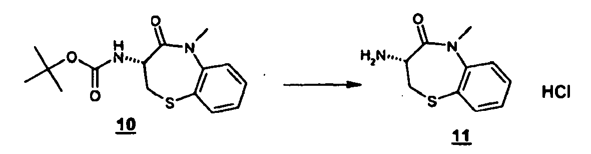

On reprend 1,01 g de **10** (3,28 mmol) dans un ballon de 100 mL et on ajoute 20 mL d'une solution d'acide chlorhydrique dans le dioxane (4M). On agite le milieu pendant 2 h à TA sous argon. Après évaporation du solvant, trituration avec l'éther, et essorage, on obtient 0,83 g d'amine **11** (poudre jaune claire) sous forme de chlorhydrate qui est utilisé directement pour l'étape suivante.
IE: m/z = 208 M^{+.}.
RMN ¹H (400 MHz, DMSO-d₆), δ(ppm): 3,15 (t, J = 11,5 Hz, 1H) ; 3,35 (s, 3H) ; 3,67 (dd, J = 7,0 et 11,5 Hz, 1H) ; 3,85 (dd J = 7,0 et 11,5 Hz, 1H) ; 7,36 (m, 1H) ; 7,56 à 7,63 (m, 2H) ; 7,69 (m, 1H) ; 8,41 (s large, 3H).

### Etape 3: Préparation du N-[(3R)-5-méthyl-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide (Ex4)

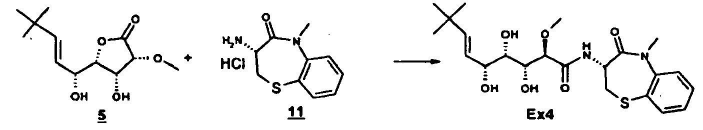

On introduit successivement dans un ballon de 30 mL, sous agitation et sous atmosphère d'argon, 293 mg de **5** (1,2 mmol), 441 mg de **11**(1,8 mmol) , 399 mg de 2-éthylhexanoate de sodium (2,4 mmol) dans 10 mL de THF. On maintient l'agitation à TA pendant 94 h. On ajoute 20 mL de CH₂Cl₂ au milieu réactionnel. On lave avec 15 mL d'une solution de HCl (0,5 N), puis 10 mL d'eau. La phase organique est séchée sur sulfate de magnésium anhydre, filtrée puis évaporée à sec. Le brut est chromatographié sur une cartouche de silice (25 g, éluant. CH₂Cl₂/isopropanol *: 95*/*5*). On recueille 345 mg de produit attendu **Ex4**.
ES : m/z = 451 (M-H)⁻.
IR (KBr): 3396; 2958; 1659; 1585; 1517; 1475; 1394; 1110; 975 & 766 cm⁻¹ RMN ¹H (300 MHz, DMSO-d₆), δ(ppm): 0,95 (s, 9H) ; 3,07 (t, J = 11,5 Hz, 1H) ; 3,20 (s, 3H) ; 3,29 (m partiellement masqué, 4H) ; 3,43 (dd, J = 6,5 et 11,5 Hz, 1H) ; 3,49 (m, 1H) ; 3,66 (d, J = 8,0 Hz, 1H) ; 3,91 (m, 1H) ; 4,32 (m, 2H) ; 4,41 (m, 1H) ; 4,52 (d large, J = 4,5 Hz, 1H) ; 5,28 (dd, J = 7,0 et 16,0 Hz, 1H) ; 5,61 (d large, J = 16,0 Hz, 1H) ; 7,32 (m, 1H) ; 7,56 (m, 2H) ; 7,65 (d, J = 7,5 Hz, 1H) ; 8,22 (d, J = 8,0 Hz, 1H).

### Ex5 N-((3R)-9-chloro-4-oxo-7-trifluorométhyl-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide

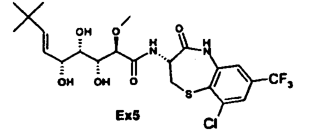

### Etape 1: Préparation de l'acide (2R)-2-tert-butoxycarbonylamino-3-(2-chloro-6-nitro-4-trifluorométhyl-phenylsulfanyl)-propionique (13)

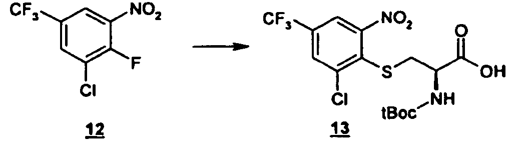

Dans un tricol contenant 0,91 g de L-Boc-Cys-OH (4,1 mmol), 7,2 mL d'eau et 1,0 g de NaHCO₃ (2,89 mmol), on introduit goutte à goutte une solution de **12** (1,0g, 4,1 mmol) dans 8,6 mL d'éthanol. On porte le milieu au reflux pendant 2 h, et concentre l'éthanol. On lave la phase aqueuse avec 25 mL d'éther, une fois la phase éthérée décantée, on rajoute 25 mL d'AcOEt, et amène la phase aqueuse jusqu'à pH2-3 avec du HCl (1N). Après agitation et décantation la phase aqueuse est extraite de nouveau avec 25 mL d'AcOEt. Les phases organiques sont réunies, séchées sur MgSO4, filtrées puis évaporées à sec. On obtient 2,0 g de produit attendu **13** (huile jaune) qui est utilisé directement pour le stade suivant.

### Etape 2: Préparation de l'acide (2R)-(2-amino-6-chloro-4-trifluorométhyl-phenylsulfanyl)-2-tert-butoxycarbonylamino-propionique (14)

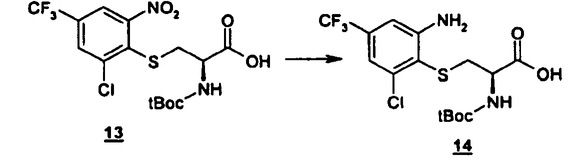

Dans un autoclave contenant 1,85g de **13** (4,2 mmol), 186 mg de Pd/C (10%) et 45 mL de MeOH, on hydrogène sous 5 bars pendant 5,5 h à 20°C. Après filtration sur célite du catalyseur, et évaporation du solvant, on obtint 1,45 g de produit **14** attendu qui est utilisé directement pour le stade suivant. RMN ¹H (300 MHz, DMSO-d₆), δ(ppm):1,36 (s large, 9H) ; 3,01 (m, 1H) ; 3,10 (dd, J = 4,5 et 13,5 Hz, 1H) ; 3,98 (m, 1H) ; 6,25 (s large, 2H) ; 6,94 (d, J = 2,0 Hz, 1H) ; 7,00 (d, J = 2,0 Hz, 1H) ; 7,14 (d large, J = 8,0 Hz, 1H) ; 12,8 (m très étalé, 1H).

### Etape 3: Préparation du ((3R)-9-chloro-4-oxo-7-trifluorométhyl-2,3,4,5-tetrahydro-1,5-benzothiazépin-3-yl)-carbamate de tert-butyle (15)

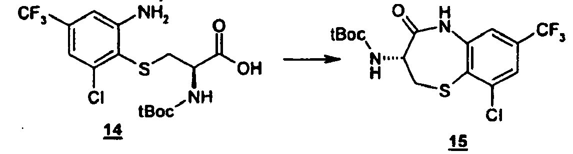

Dans un tricol de 500 mL contenant 1,35 g de **14** (3,3 mmol), 50 mL de CH₂Cl₂ et 0,325 g de TEA (3,3 mmol), on introduit, à 0°C, une solution d'EDCI (0,624, 3,3 mmol, chlorhydrate de 1-(3-diméthylaminopropyl)-3-éthylcarbodiimide) et 1-hydroxybenzotriazole (0,44g, 3,3 mmol) dans 200 mL de CH₂Cl₂. On laisse le milieu réactionnel revenir à TA, agite pendant 24 h. On lave le milieu avec 2 fois 200 mL d'eau. La phase organique est séchée sur MgSO₄, filtrée et évaporée au sec. Le brut est chromatographié sur une cartouche de silice (70 g) avec éluant CH₂Cl₂/MeOH (*gradient : MeOH 0* à *5%*). On obtient 0,44 g de produit attendu **15** (meringue beige).
RMN ¹H (300 MHz, DMSO-d₆), δ(ppm): 1,34 (s, 9H) ; 3,24 (t, J = 11,5 Hz, 1H) ; 3,58 (dd, J = 7,0 et 11,5 Hz, 1H) ; 4,12 (m, 1H) ; 7,34 (d, J = 8,0 Hz, 1H) ; 7,43 (s large, 1H) ; 7,84 (s large, 1H) ; 10,3 (s, 1H).

### Etape 4: Préparation de chlorhydrate de (3R)-3-amino-9-chloro-7-trifluorométhyl-2,3-dihydro-5H-1,5-benzothiazépin-4-one (16)

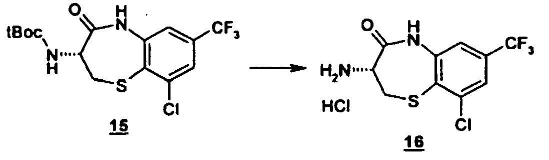

Dans un ballon de 25 mL contenant 582 mg de **15** (1,47 mmol), on ajoute 11 mL d'une solution d'acide chlorhydrique dans le dioxane (4M). On agite pendant 4 h à TA sous argon. Un précipité blanc se forme, qui est essoré, lavé avec 3 mL de dioxane puis 5 mL d'éther isopropylique. On obtient ainsi 450 mg d'amine **16** sous forme de chlorhydrate.
RMN ¹H (400 MHz, DMSO-d₆), δ(ppm): 3,39 (t, J = 11,5 Hz, 1H) ; 3,79 (dd, J = 7,0 et 11,5 Hz, 1H) ; 4,18 (dd, J = 7,0 et 11,5 Hz, 1H) ; 7,43 (d, J = 2,0 Hz, 1H) ; 7,91 (d, J = 2,0 Hz, 1H) ; 8,57 (s large, 3H) ; 10,9 (s, 1H).

### Etape 5 : Préparation du N-((3R)-9-chloro-4-oxo-7-trifluorométhyl-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide (Ex5)

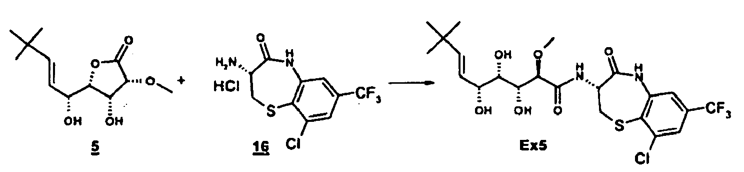

On introduit successivement dans un ballon de 25 mL, sous agitation et sous atmosphère d'argon, 210 mg de **5** (0,43 mmol), 287 mg de **16** (0,86 mmol), 179 mg de 2-éthylhexanoate de sodium (1,1 mmol) dans 2,5 mL de THF. On maintient l'agitation à TA pendant 24 h. On ajoute 10 mL d'acétate d'éthyle au milieu réactionnel. On lave successivement avec 2 fois 10 mL d'une solution de HCl (1 N). La phase organique est séchée sur sulfate de magnésium anhydre, filtrée puis évaporée à sec. Le brut est chromatographié sur une cartouche de silice (20g, éluant CH₂Cl₂/MeOH : *en gradient MeOH 0* à *10%*). On recueille 176 mg de produit attendu **Ex5.**
ES : m/z = 539 (M-H)⁻.
RMN ¹H (400 MHz, DMSO-d₆), δ(ppm): 0,95 (s, 9H) ; 3,19 (s, 3H) ; de 3,27 à 3,38 (m masqué, 2H) ; 3,49 (m, 1H) ; 3,58 (dd, J = 7,0 et 11,5 Hz, 1H) ; 3,67 (d, J = 8,0 Hz, 1H) ; 3,93 (m, 1H) ; 4,27 (d, J = 7,5 Hz, 1H) ; 4,31 (d, J = 5,5 Hz, 1H) ; 4,45 (m, 1H) ; 4,55 (d, J = 4,5 Hz, 1H) ; 5,28 (dd, J = 7,0 et 16,0 Hz, 1H) ; 5,62 (d, J = 16,0 Hz, 1H) ; 7,42 (d, J = 2,0 Hz, 1H) ; 7,86 (d, J = 2,0 Hz, 1H) ; 8,35 (d, J = 7,5 Hz, 1H) ; 10,45 (s, 1H).

### Ex6: N-((3R)-9-bromo-4-oxo-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide

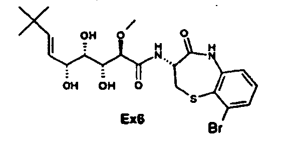

### Etape 1: Préparation de l'acide (3R)-(2-bromo-6-nitro-phenylsulfanyl)-2-tert-butoxycarbonylamino-propionique (18)

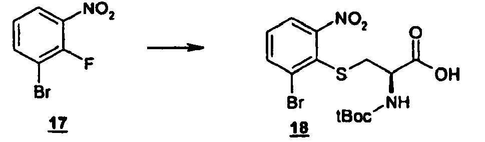

Dans un tricol contenant 3,02 g de L-Boc-Cys-OH (13,6 mmol), 24 mL d'eau et 3,31 g de NaHCO₃ (39,4 mmol), on introduit goutte à goutte une solution de **17** (3,0 g, 13,6 mmol) dans 28,5 mL d'éthanol. On porte le milieu au reflux pendant 2 h, puis évapore l'éthanol. On lave ensuite la phase aqueuse avec 50 mL d'éther, une fois la phase éthérée décantée, on rajoute 25 mL d'AcOEt et amène la phase aqueuse jusqu'à pH2-3 avec du HCl (1 N). Après agitation et décantation la phase aqueuse est extraite de nouveau avec 50 mL d'AcOEt. Les phases organiques sont réunies, séchées sur MgSO₄, filtrées et enfin évaporées à sec. On obtient 5,92 g de produit attendu **18** qui est utilisé directement pour le stade suite.
RMN ¹H (400 MHz, DMSO-d₆), δ(ppm): 1,36 (s, 9H) ; 3,12 (dd, J = 9,5 et 13,0 Hz, 1H) ; 3,32 (dd, J = 4,5 et 13,0 Hz, 1H) ; 3,93 (m, 1H) ; 7,09 (d, J = 8,5 Hz, 1H) ; 7,55 (t, J = 7,5 Hz, 1H) ; 7,90 (d large, J = 7,5 Hz, 1H) ; 8,05 (d large, J = 7,5 Hz, 1H) ; 12,7 (m étalé, 1H)_{.}

### Etape 2: Préparation de l'acide (3R)-3-(2-amino-6-bromo-phenylsulfanyl)-2-tert-butoxycarbonylamino-propionique (19)

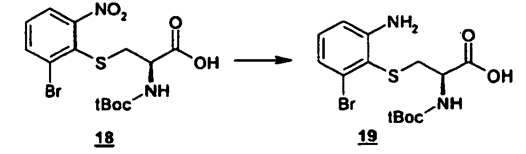

Dans un autoclave contenant 5,92 g de **18** (14,1 mmol), 882 mg de Pd/C (10%) et 100 mL de MeOH, on hydrogène sous 5 bars pendant 5 h à 20°C. Après filtration du catalyseur sur célite. On évapore le solvant et obtient 5,1 g de produit attendu **19** qui est utilisé directement pour l'étape suivante. RMN ¹H (400 MHz, DMSO-d₆), δ(ppm): 1,37 (s, 9H) ; 2,92 (dd, J = 9,5 et 13,0 Hz, 1H) ; 3,02 (dd, J = 4,5 et 13,0 Hz, 1H) ; 3,95 (m, 1H) ; 6,72 (d large, J = 8,0 Hz, 1H) ; 6,83 (d large, J = 8,0 Hz, 1H) ; 6,96 (t, J = 8,0 Hz, 1H) ; 7,22 (m masqué, 1H) ; 12,75 (m étalé, 1H).

### Etape 3: Préparation du ((3R)-9-bromo-4-oxo-2,3,4,5-tetrahydro-1,5-benzothiazépin-3-yl)-carbamate de tert-butyle (20)

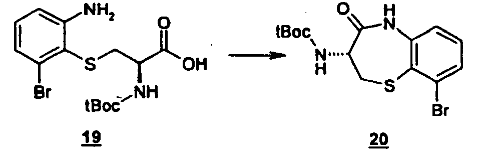

Dans un tricol de 500 mL contenant 5,02 g de **19** (12,8 mmol), 150 mL de CH₂Cl₂ et 1,30 g de TEA (12,8 mmol), on introduit, à 0°C, une solution d'EDCI (2,46, 12,8 mmol, chlorhydrate de 1-(3-diméthylaminopropyl)-3-éthylcarbodiimide) et 1-hydroxybenzotriazole (1,73 g, 12,8 mmol) dans 65 mL de CH₂Cl₂. On laisse revenir à TA le milieu réactionnel et poursuit l'agitation pendant 24 h. On lave le milieu avec 2 fois 500 mL d'eau. La phase organique est séchée sur MgSO₄, filtrée puis évaporée à sec. Le brut est chromatographié sur une cartouche de silice (300 g) avec éluant CH₂Cl₂/MeOH (*en gradient : MeOH 0 à 5%)*. On obtient 2,91 g de produit attendu **20** (meringue beige).
RMN ¹H (400 MHz, DMSO-d₆), δ(ppm): 1,33 (s, 9H) ; 3,13 (t, J = 12,0 Hz, 1H) ; 3,52 (dd, J = 7,0 et 12,0 Hz, 1H) ; 4,04 (m, 1H) ; 7,17 (d large, J = 8,0 Hz, 1H) ; 7,28 (d, J = 8,0 Hz, 1H) ; 7,34 (t, J = 8,0 Hz, 1H) ; 7,59 (d large, J = 8,0 Hz, 1H) ; 10,15 (s, 1H).

### Etape 4: Préparation du chlorhydrate de (3R)-3-amino-9-bromo-2,3-dihydro-5H-1,5-benzothiazépin-4-one (21)

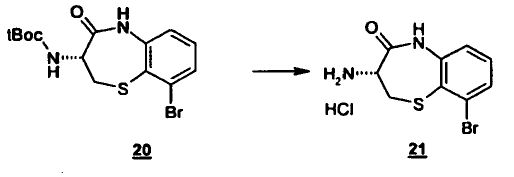

Dans un ballon de 25 mL contenant 500 mg de **20** (1,34 mmol), on ajoute 10 mL d'une solution d'acide chlorhydrique dans le dioxane (4M). On agite durant 4 h à TA sous argon. Un précipité blanc se forme, qui est essoré, lavé avec 3 mL de dioxane, puis 5 mL d'éther isopropylique. On obtient ainsi 386 mg d'amine **21** (solide beige) sous forme de chlorhydrate.
RMN ¹H (300 MHz, DMSO-d₆), δ(ppm):: 3,29 (t, J = 11,5 Hz, 1H) ; 3,78 (dd, J = 7,0 et 11,5 Hz, 1H) ; 3,96 (m, 1H) ; 7,19 (dd, J = 1,5 et 8,0 Hz, 1H) ; 7,38 (t, J = 8,0 Hz, 1H) ; 7,64 (dd, J = 1,5 et 8,0 Hz, 1H) ; 8,53 (s large, 3H) ; 10,75 (s, 1H).

### Etape 5: Préparation du N-((3R)-9-bromo-4-oxo-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide (Ex6)

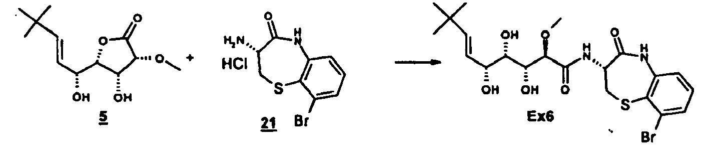

On introduit successivement dans un ballon de 25 mL, sous agitation et sous atmosphère d'argon, 80 mg de **5** (327 µmol), 101 mg de **21** (327 µmol), 163 mg de 2-éthylhexanoate de sodium (0,98 mmol) dans 2,0 mL de THF. On maintient l'agitation à TA pendant 24 h. On ajoute 5 mL d'acétate d'éthyle au milieu réactionnel. On lave successivement avec 5 mL d'une solution de HCl (1 N), 5 mL d'une solution saturé de NaHCO₃, et 5 mL d'eau. La phase organique est séchée sur sulfate de magnésium anhydre, filtrée puis évaporée à sec. Le brut est chromatographié sur une cartouche de silice (5 g, éluant CH₂Cl₂/MeOH *en gradient : MeOH 1 à 10%*). On recueille 95 mg de produit attendu **Ex6.**
ES: m/z = 515 (M-H)⁻.
RMN ¹H (400 MHz, DMSO-d₆), δ(ppm): 0,96 (s, 9H) ; de 3,17 à 3,32 (m partiellement masqué, 2H) ; 3,20 (s, 3H) ; 3,49 (m, 1H); 3,53 (dd partiellement masqué, J = 7,0 et 11,5 Hz, 1H) ; 3,68 (d, J = 8,0 Hz, 1H) ; 3,93 (m, 1H) ; 4,30 (m, 2H) ; 4,38 (m, 1H) ; 4,57 (d, J = 4,5 Hz, 1H) ; 5,29 (dd, J = 7,0 et 16,0 Hz, 1H) ; 5,62 (d, J = 16,0 Hz, 1H) ; 7,18 (d large, J = 7,5 Hz, 1H) ; 7,35 (t, J = 7,5 Hz, 1H) ; 7,61 (d large, J = 7,5 Hz, 1H) ; 8,29 (d, J = 7,5 Hz, 1H) ; 10,3 (s, 1H).

### Ex7: N-((3R)-8-chloro-4-oxo-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide

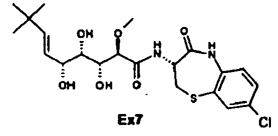

### Etape 1: Préparation de l'acide (3R)-3-(3-chloro-6-nitro-phenylsulfanyl)-2-tert-butoxycarbonylamino-propionique (23)

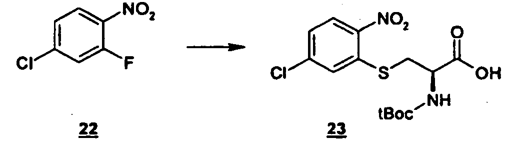

Dans un tricol contenant 1,26 g de L-Boc-Cys-OH (5,70 mmol), 10 mL d'eau et 1,38 g de NaHCO₃ (16,5 mmol), on introduit goutte à goutte une solution de **22** (1,0 g, 5,7 mmol) dans 12 mL d'éthanol. On porte le milieu au reflux pendant 6 h. On évapore l'éthanol. On lave ensuite la phase aqueuse avec 30 mL d'éther, une fois la phase éthérée décantée, on acidifie la phase aqueuse jusqu'à pH2-3 avec du HCl (1 N) puis on extrait avec 2 fois 30 ml de CH₂Cl₂. Les phases organiques sont réunies séchées sur MgSO₄, filtrées puis évaporées à sec. On obtient 2,1 g de produit attendu **23** (meringue jaune) qui est utilisé pour le stade suivant.
RMN ¹H (400 MHz, DMSO-d₆), δ(ppm): 1,35 (s, 9H) ; 3,24 (dd, J = 10,0 et 13,5 Hz, 1H) ; 3,59 (dd, J = 4,0 et 13,5 Hz, 1H) ; 4,13 (m, 1H) ; 7,29 (d large, J = 9,0 Hz, 1H) ; 7,48 (dd, J = 2,0 et 9,0 Hz, 1H) ; 7,72 (d, J = 2,0 Hz, 1H) ; 8,20 (d, J = 9,0 Hz, 1H) ; 13,0 (m étalé, 1H).

### Etape 2: Préparation de l'acide (3R)-3-(2-amino-4-chloro-phenylsulfanyl)-2-tert-butoxycarbonylamino-propionique (24)

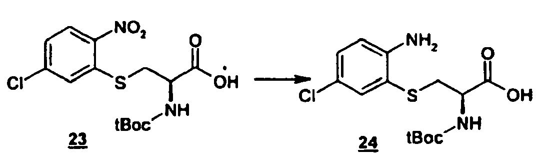

Dans un tricol contenant 2,1 g de **23** (5,55 mmol), 0,59g de chlorure d'ammonium et 70 mL de MeOH, on ajoute 7,3 g de Zinc (0,11 mol). On agite le milieu réactionnel à TA pendant 2 h et puis on chauffe à 75°C pendant 2h. On filtre sur célite, lave avec 20 ml de MeOH bouillant. On évapore à sec, le résidu brut est repris dans 50 mL d'eau puis extrait avec 2 fois 50 mL de CH₂Cl₂. Les phases organiques réunies sont séchées sur MgS04, filtrées et évaporées à sec pour donner 1,62 g de produit attendu **24** (meringue crème) qui est utilisé pour le stade suivant.
RMN ¹H (400 MHz, DMSO-d₆), δ(ppm): 1,36 (s, 9H) ; 2,93 (dd, J = 8,5 et 13,0 Hz, 1H) ; 3,12 (dd, J = 4,0 et 13,0 Hz, 1H) ; 3,93 (m, 1H) ; 5,49 (s large, 2H) ; 6,59 (d large, J = 8,0 Hz, 1H) ; 6,70 (d, J = 8,5 Hz, 1H) ; 7,04 (dd, J = 2,5 et 8,5 Hz, 1H) ; 7,26 (d, J = 2,5 Hz, 1H).

### Etape 3: Préparation du (3R)-8-chloro-4-oxo-2,3,4,5-tetrahydro-1,5-benzothiazépin-3-yl)-carbamate de tert-butyle (25)

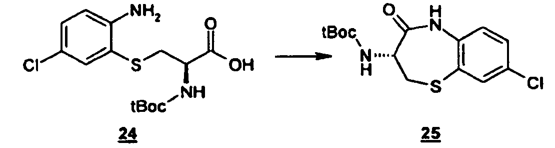

Dans un ballon de 100 mL contenant 1,62 g de **24** (4,67 mmol) et 30 mL de DMF, on ajoute 2,07 g d'hexafluorure de benzotriazol-1-yloxytris(diméthylamino)-phosphonium (4,67 mmol) et 1,96 g de NaHCO₃. Le milieu réactionnel est agité pendant 24 h à TA. On ajoute 70 mL d'AcOEt, lave en suite avec 50 mL de HCl (1 N), 50 mL d'une solution saturée en NaHCO₃ et 50 mL d'eau. La phase organique est séchée sur MgSO₄, filtrée et évaporée à sec. Le brut est chromatographié sur une cartouche de silice (40 g) avec éluant Heptane/AcOEt (*en gradient AcOEt : 12 à 50* %*)*. On obtient 1,14 g de produit attendu **25** (meringue jaune pâle).
RMN ¹H (400 MHz, DMSO-d₆), δ(ppm): 1,33 (s, 9H) ; 3,10 (t, J = 11,5 Hz, 1H) ; 3,54 (dd, J = 7,0 et 11,5 Hz, 1H) ; 4,08 (m, 1H) ; 7,16 (d, J = 8,5 Hz, 1H) ; 7,27 (d, J = 8,5 Hz, 1H) ; 7,51 (dd, J = 2,5 et 8,5 Hz, 1H) ; 7,64 (d, J = 2,5 Hz, 1H) ; 10,1 (s, 1H).

### Etape 4: Préparation du chlorhydrate de (3R)-3-amino-8-chloro-2,3-dihydro-5H-1,5-benzothiazépin-4-one (26)

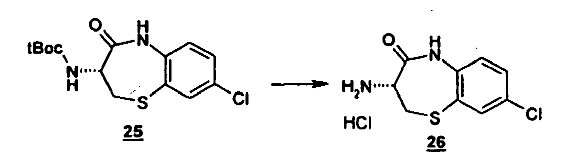

Dans un ballon de 25 mL contenant 500 mg de **25** (1,52 mmol), on ajoute 10 mL d'une solution d'acide chlorhydrique dans le dioxane (4M). On agite pendant 4 h à TA sous argon. Un précipité blanc se forme, qui est essoré, lavé avec 5 mL de dioxane puis 5 mL d'éther isopropylique, on obtient après séchage sous vide 510 mg d'amine **26** ( sous forme de chlorhydrate).
RMN ¹H (400 MHz, DMSO-d₆), δ(ppm): 3,26 (t, J = 11,5 Hz, 1H) ; 3,81 (dd, J = 7,0 et 11,5 Hz, 1H) ; 3,97 (dd, J = 7,0 et 11,5 Hz, 1H) ; 7,20 (d, J = 8,5 Hz, 1H) ; 7,55 (dd, J = 2,5 et 8,5 Hz, 1H) ; 7,70 (d, J = 2,5 Hz, 1H) ; 8,55 (s large, 3H) ; 10,65 (s, 1H).

### Etape 5: Préparation du N-((3R)-8-chloro-4-oxo-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide (Ex7)

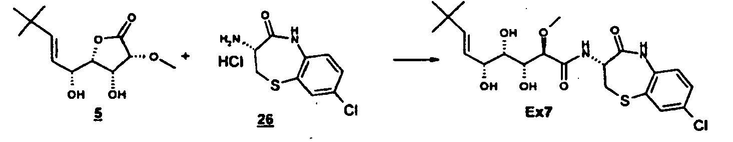

On introduit successivement dans un ballon de 25 mL, sous agitation et sous atmosphère d'argon, 87 mg de **5** (352 µmol), 187 mg de **26** (704 µmol), 146 mg de 2-éthylhexanoate de sodium (0,88 mmol) dans 2,0 mL de THF. On maintient l'agitation à TA pendant 24 h. On ajoute 5 mL d'acétate d'éthyle au milieu réactionnel. On lave successivement avec 5 mL d'une solution de HCl (1 N), 5 mL d'une solution aqueuse saturée de NaHCO₃ et 5 mL d'eau. La phase organique est séchée sur sulfate de magnésium anhydre, filtrée puis évaporée à sec. Le brut est chromatographié sur une cartouche de silice (25 g, éluant CH₂Cl₂/MeOH : *en gradient MeOH : 1 à 10%*). On recueille 70 mg de produit attendu **Ex7.**
ES: m/z = 495 MNa⁺ ; m/z = 473 M H⁺.
RMN ¹H (300 MHz, DMSO-d₆), δ(ppm): 0,96 (s, 9H) ; 3,18 (t partiellement masqué, J = 11,5 Hz, 1H) ; 3,21 (s, 3H) ; de 3,25 à 3,34 (m masqué, 1H) ; 3,49 (m, 1H) ; 3,56 (dd, J = 7,0 et 11,5 Hz, 1H) ; 3,67 (d, J = 8,0 Hz, 1H) ; 3,91 (m, 1H) ; 4,29 (m large, 2H) ; 4,41 (m, 1H) ; 4,53 (m large, 1H) ; 5,28 (dd, J = 7,0 et 16,0 Hz, 1H) ; 5,62 (d, J = 16,0 Hz, 1H) ; 7,17 (d, J = 8,5 Hz, 1H) ; 7,52 (dd, J = 2,0 et 8,5 Hz, 1H) ; 7,67 (d, J = 2,0 Hz, 1H) ; 8,24 (d large, J = 8,0 Hz, 1H) ; 10,2 (s, 1H).

### Ex8: (6E)-(2R,3R,4S,5R)-8-éthyl-3,4,5-trihydroxy-2-méthoxy-N-((3R)-5-méthyl-4-oxo-2,3,4,5-tetrahydro-1,5-benzothiazépin-3-yl)-déc-6-énamide

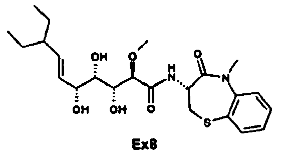

### Etape 1: Préparation du (4R,4aS,7R,7aR)-7-méthoxy-2,2-diméthyl-4-vinyl-tetrahydro-furo[3,2-d]-1,3-dioxin-6-one (28)

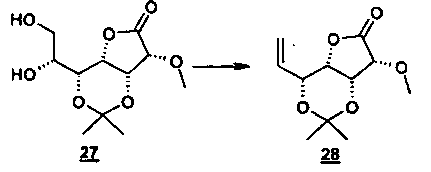

Dans un ballon de 4000 mL, équipé d'une agitation mécanique, on charge sous azote 178,2 g de PPh₃ (0,679 mol), 84,1 g d'imidazole (1,235 mol), et 2430 mL de THF anhydre, On additionne avec précaution 156,8 g d'Iode bisublimé (0,618 mol) en maintenant la température du mélange réactionnel à 30°C. On porte ce milieu au reflux (66°C) durant 1 h, puis on ajoute progressivement 81 g de **27** (0,309 mol) (qui peut être préparé selon les modes opératoires décrits dans Org. Process Res. Dev. 2003, 7(6), 856-865) à 66°C +/- 2°C. Le milieu homogène ainsi obtenu est chauffé au reflux pendant 3 h. On laisse revenir à 20°C +/- 5°C, puis on coule 1000 mL d'une solution de NaHCO₃ à 10% (effervescence, athermique) (pH 8,0-8,5). Ensuite on additionne 185,5 g de Na₂S₂O₃ jusqu'à la décoloration quasi totale (apparition d'un précipité minéral). Après agitation à 20°C +/- 5°C pendant 30 minutes, on filtre et rince avec du THF le solide. Le filtrat THF/H₂O est concentré partiellement à l'évaporateur rotatif à une température inférieure à 35°C. Le concentrat aqueux est saturé avec NaCl et extrait avec 1500ml de CH₂Cl₂. La phase organique est séchée sur MgSO₄, filtrée et évaporée à sec. Le résidu est repris dans 2000 mL d'un mélange H₂O/Acétone (75/25), les insolubles sont filtrés, et rincés avec le mélange H₂O/Acétone (75/25). Les filtrats sont concentrés à l'évaporateur rotatif à 50°C et 20 mbars, et filtrés de nouveau sur un verre fritté (porosité N°4). La phase aqueuse est saturée avec NaCl, est extraite 3 fois avec CH₂Cl₂ (1000 mL, 500 mL, et 250 mL). Les phases organiques sont réunies, séchées sur MgSO₄, filtrées et évaporées à sec pour donner 60 g de produit brut, qui est dissout dans 250 ml de CH₂Cl₂. On ajoute ensuite dans la solution 30 g de silice. Après agitation pendant 15 min, on filtre la silice, rince 2 fois avec de CH₂Cl₂ (250 mL et 100 mL). Le filtrat est concentré à sec et séché sous 1 mbar à 20°C pour donner 54,8 g de produit attendu **28** (solide blanc)
RMN ¹H (300 MHz, DMSO-d₆), δ(ppm): 5.85 (m, 1 H) ; 5.35 (d, 1 H) ; 5.25 (d, 1 H) ; 4.80 (m, 1 H) ; 4.69 (m, 1 H) ; 4.43 (d, 1 H) ; 4.22 (m, 1 H) ; 3.40 (s, 3 H) ; 1.49 (s, 3 H) ; 1.30 (s, 3 H).

### Etape 2: Préparation du (3R,4R,5S)-4-hydroxy-5-((1R)-hydroxy-allyl)-3-méthoxy-dihydro-furan-2-one (29)

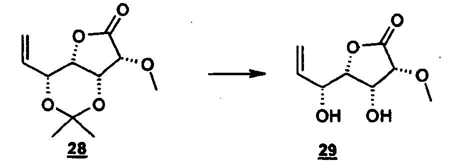

Dans un ballon de 100 mL contenant 1,0 g de **28** (4,38 mmol), 10 mL d'eau et 14 mL de THF, on ajoute à 0°C goutte à goutte 10 mL de TFA. On laisse le milieu revenir à TA et agite pendant 1 nuit. On concentre ensuite le milieu à pression réduite à TA et ajoute 50 mL d'eau, congèle et lyophilise. Le lyophilisat est empâté dans de l'heptane en présence d'un minimum de méthanol, après évaporation des solvants, on obtient 778 mg de **29** attendu (solide blanc).
MS : m/z = 211 [M+Na]⁺, 189 [M+H]⁺
RMN ¹H (400 MHz, DMSO-d₆), δ(ppm): 3,42 (s, 3H) ; 3,98 (dd, J = 2,5 et 9,0 Hz, 1H) ; de 4,25 à 4,34 (m, 3H) ; 5,22 (dm, J = 10,5 Hz, 1H) ; 5,29 (d, J = 5,0 Hz, 1H) ; 5,44 (d partiellement masqué, J = 16,5 Hz, 1H); 5,46 (m, 1H) ; 5,97 (m, 1H).

### Etape 3: Préparation du ((3R,4R,5S)-5-((2E)-(1R)-4-éthyl-1-hydroxy-héx-2-ényl)-4-hydroxy-3-méthoxy-dihydro-furan-2-one (30)

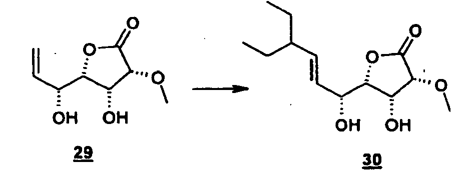

Dans un vial de 5 mL, on ajoute 100 mg de **29** (0,53 mmol), 0,52g de 3-éthyl-1-pentène (5,3 mmol), 4 mL de CH₂Cl₂ et 90 mg de catalyseur de Grubbs 2° génération (C46H65Cl2N2PRu, PM 848,98, 0,11 mmol). La solution rouge brique capsulée est chauffée pendant 10 minutes à 60 °C au micro-ondes (Smithsynthesizer, 300 Watt). On évapore le solvant, le brut est chromatographié sur une cartouche de silice (25 g) en éluant avec un mélange Heptane/AcOEt (*55*/*45)*. On obtient 54 mg de produit attendu 30 (solide marron).
RMN ¹H (300 MHz, DMSO-d₆), δ(ppm): 0,81 (t, J = 7,5 Hz, 6 H) ; 1,23 (m, 4H) ; de 1,32 à 1,49 (m, 2H) ; 1,78 (m, 1H) ; 3,42 (s, 3H) ; 3,97 (d, J = 9,0 Hz, 1H) ; 4,20 à 4,31 (m, 3H) ; 5,11 à 5,75 (m, 4 H).

### Etape 4: Préparation du (6E)-(2R,3R,4S,5R)-8-éthyl-3,4,5-trihydroxy-2-méthoxy-N-((3R)-5-méthyl-4-oxo-2,3,4,5-tetrahydro-1,5-benzothiazépin-3-yl)-déc-6-énamide (Ex8)

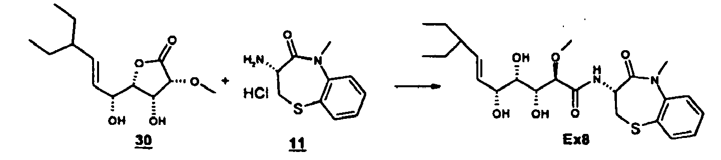

On introduit successivement dans un ballon de 30 mL, sous agitation et sous atmosphère d'argon, 50 mg de **30** (0,19 mmol), 71 mg de **11** (0,29 mmol), 64 mg de 2-éthylhexanoate de sodium (0,39 mmol) dans 2,0 mL de THF. On maintient l'agitation à TA pendant 96 h. On ajoute 4 mL de CH₂Cl₂ au milieu réactionnel. On lave successivement avec 3 mL d'une solution de HCl (0,5N), 3 mL d'eau. La phase aqueuse est réextraite avec 2 fois 4 ml de CH₂Cl₂. Les phases organiques réunies sont séchées sur sulfate de magnésium anhydre, filtrées puis évaporées à sec. Le brut est chromatographié sur une cartouche de silice (12 g, éluant, CH₂Cl₂/isopropanol : *95*/*5*). On recueille 63 mg de produit attendu **Ex8.**
ES : m/z = 465 (M-H)⁻.
RMN ¹H (400 MHz, DMSO-d₆), δ(ppm) : 0,75 à 0,82 (m, 6H) ; 1,10 à 1,37 (m, 4H) ; 1,70 (m, 1H) ; 3,06 (t, J = 11,5 Hz, 1H) ; 3,20 (s, 3H) ; 3,25 à 3,3 (m, 4H) ; 3, 44 (dd, J = 7,0 et 11, 5 Hz, 1H) ; 3,50 (m, 1H) ; 3,65 (d, J = 8,0 Hz, 1H) ; 3,92 (m, 1H) ; 4,30 (ml, 2H) ; 4,41 (m, 1H) ; 4,51 (m, 1H) ; 5,26 à 5,37 (m, 2H) ; 7,32 (m, 1H) ; 7,57 (m, 1H) ; 7,66 (d, J = 7,5Hz, 1H) ; 8,20 (dl, J = 8,0 Hz, 1H).

### Ex9: (2R,3R,4S,5R,6E)-7-cyclopentyl-3,4,5-trihydroxy-2-méthoxy-N-[(3R)-5-méthyl-4-oxo-2,3,4,5-tétrahydro-1,5-benzothiazépin-3-yl]hept-6-énamide

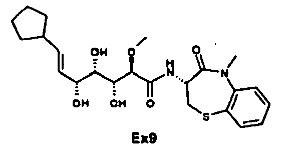

### Etape 1: Préparation du (3R,4R,5S)-5-[(1R,2E)-3-cyclopentyl-1-hydroxyprop-2-én-1-yl]-4-hydroxy-3-méthoxydihydrofuran-2(3H)-one (31)

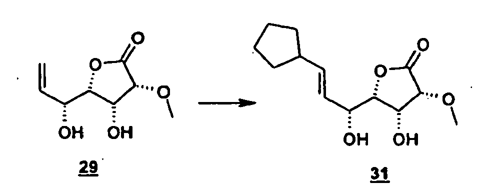

Dans un vial de 5 mL, introduire 100 mg (0,53 mmol) de **29**, 4 mL de CH₂Cl₂, 726 µL (5,3 mmol) de vinylcyclopentane puis 90,2 mg (106 µmol) de catalyseur de Grubbs de seconde génération. Chauffer la solution 10 min à 60 °C au micro-ondes. Le solvant est ensuite évaporé à sec sous pression réduite puis le résidu est purifié sur colonne de silice Biotage 12-M (éluant : Hept/AcOEt 40/60). Le produit **31** (76,3 mg, Rf = 0.35 dans les conditions d'élution utilisées) est obtenu sous forme d'un solide marron.
MS : m/z = 279 [M+Na]⁺, 257 [M+H]⁺
RMN ¹H (300 MHz, DMSO-d₆), δ(ppm): 5.82 (dd, 1 H, J=8 Hz et 16 Hz,), 5.50 (dd, 1H, J=6 Hz et 16 Hz), 5.39 (d, 1H, J=4.5 Hz), 5.15 (d, 1H, J=6 Hz), 4.27 (m, 3H), 3.95 (dd, 1H, J=3 Hz et 8 Hz), 3.41 (s, 3H), 2.43 (m, 1H), 1.73 (m, 2H), 1.56 (m, 4H), 1.26 (m, 2H).

### Etape 2: Préparation du (2R,3R,4S,5R,6E)-7-cyclopentyl-3,4,5-trihydroxy-2-méthoxy-N-[(3R)-5-méthyl-4-oxo-2,3,4,5-tétrahydro-1,5-benzothiazépin-3-yl]hept-6-énamide (Ex9)

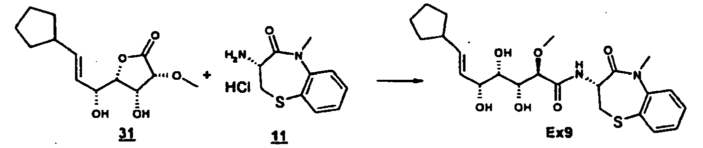

Dans un monocol de 30 mL, introduire 60 mg (234 µmol) de **31**, 68,7 mg (281 µmol) de **11**, 58,3 mg (351 µmol) de 2-éthylhexanoate de sodium dans 2,5 mL de THF. La solution est laissée sous agitation à température ambiante pendant 1 semaine. La solution est ensuite reprise avec 5 mL de CH₂Cl₂ puis lavée avec 4 mL d'HCl 0,5 N puis 4 mL d'eau. La phase aqueuse est ensuite extraite par 2*5 mL de CH₂Cl₂ puis les phases organiques sont réunies, séchées sur sulfate de sodium puis filtrées. Le solvant est ensuite évaporé à sec sous pression réduite puis le résidu est purifié sur colonne de silice Biotage 12-M (éluant : CH₂Cl₂/lsopropanol 97/3). Le produit **Ex9** (91,7 mg, Rf = 0.25 dans les conditions d'élution utilisées) est obtenu sous forme d'un solide blanc.
MS : m/z = 929 [2M+H]⁺, 465 [M+H]⁺, 447 [(M-H₂O)+H]⁺, 429 [(M-2H₂O)+H]⁺
RMN ¹H (400 MHz, DMSO-d₆) δ (ppm) = 8.24 (d, 1H, J=8 Hz), 7.67 (d, 1H, J=8Hz), 7.57 (m, 2H), 7.33 (m, 1H), 5.57 (dd, 1H, J=6.5 Hz et 15 Hz), 5.34 (dd, 1H, J=6.5 Hz et 15 Hz), 4.53 (d, 1H, J=4.5 Hz), 4.41 (m, 1H), 4.33 (d, 1H, J=4.5 Hz), 4.30 (d, 1H, J=5 Hz), 3.91 (m, 1H), 3.66 (d, 1H, J=8 Hz), 3.47 (m, 1H), 3.41 (m, 1H), 3.31 (m, 1H), 3.29 (s, 3H), 3.20 (s, 3H), 3.07 (t, 1H, J=12 Hz), 2.38 (m, 1H), 1.70 (m, 2H), 1.54 (m, 4H), 1.22 (m, 2H).

### Ex10: (2R,3R,4S,5R,6E)-3,4,5-trihydroxy-2-méthoxy-N-[(3R)-5-méthyl-4-oxo-2,3,4,5-tétrahydro-1,5-benzothiazépin-3-yl]undéc-6-énamide

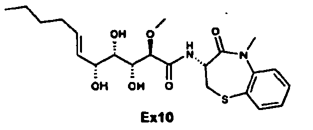

### Etape 1: Préparation du (3R,4R,5S)-4-hydroxy-5-[(1R,2E)-1-hydroxyhept-2-én-1-yl]-3-méthoxydihydrofuran-2(3H)-one (32)

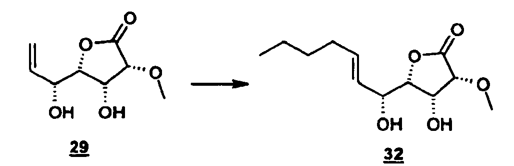

Dans un vial de 5 mL, introduire 100 mg (0,53 mmol) de **29,** 4 mL de CH₂Cl₂, 665 µL (5,3 mmol) d'hexène-1 puis 90,2 mg (106 µmol) de catalyseur de Grubbs de seconde génération. Chauffer la solution 10 min à 60 °C au micro-ondes. Le solvant est ensuite évaporé à sec sous pression réduite puis le résidu est purifié sur colonne de silice Biotage 12-S (éluant : Hept/AcOEt 40/60). Le produit **32** (57,3 mg, Rf = 0.29 dans les conditions d'élution utilisées) est obtenu sous forme d'un solide beige.
RMN ¹H (300 MHz, DMSQ-d₆) : δ (ppm) = 5.81 (m, 1H), 5.51 (dd, 1H, J=5 Hz et 15.5 Hz), 5.38 (d, 1H, J=4.2 Hz), 5.14 (d, 1H, J=4.9 Hz), 4.26 (m, 3H), 3.96 (dd, 1H, J=2.2 Hz et 8.8 Hz), 3.41 (s, 3H), 2.03 (q, 2H, J=6 Hz), 1.32 (m, 4H), 0.87 (t, 3H, J=8 Hz)

### Etape 2: Préparation du (2R,3R,4S,5R,6E)-3,4,5-trihydroxy-2-méthoxy-N-[(3R)-5-méthyl-4-oxo-2,3,4,5-tétrahydro-1,5-benzothiazépin-3-yl]undéc-6-énamide (Ex10)

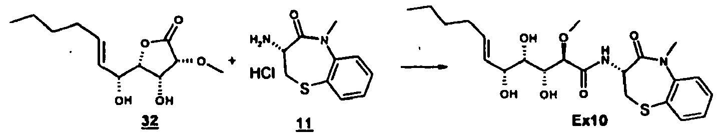

Dans un monocol de 20 mL, introduire 60 mg (246 µmol) de **32,** 72,1 mg (294 µmol) de **11**, 61,2 mg (368 µmol) de 2-éthylhexanoate de sodium dans 2,5 mL de THF. La solution est laissée sous agitation à température ambiante pendant 48 heures. La solution est ensuite reprise avec 5 mL de CH₂Cl₂ puis lavée avec 4 mL d'HCl 0,5 N puis 3 mL d'eau. La phase aqueuse est ensuite extraite par 2*5 mL de CH₂Cl₂ puis les phases organiques sont réunies, séchées sur sulfate de sodium puis filtrées. Le solvant est ensuite évaporé à sec sous pression réduite puis le résidu est purifié sur colonne de silice biotage 12-M (éluant : CH₂Cl₂/Isopropanol 98/2). Le produit **Ex10** (30,2 mg, Rf = 0.19 dans les conditions d'élution utilisées) est obtenu sous forme d'un solide blanc.
MS : m/z (ES⁺) = 926 [2M+Na]⁺, 474 [M+Na]⁺, 453 [M+H]⁺, 435 [(M-H₂O)+H]⁺ RMN ¹H (400 MHz, DMSO-d₆) : δ (ppm) = 8.24 (d, 1H, J=8 Hz), 7.66 (d, 1H, J=8Hz), 7.56 (m, 2H), 7.33 (m, 1H), 5.55 (m, 1H), 5.36 (dd, 1H, J=7.5 Hz et 16 Hz), 4.52 (d, 1H, J=4 Hz), 4.40 (m, 1H), 4.31 (d, 1H, J=4.5 Hz), 4.28 (d, 1H, J=5 Hz), 3.91 (m, 1H), 3.65 (d, 1H, J=8 Hz), 3.48 (m, 1H), 3.43 (m, 1H), 3.34 (m, 1H), 3.32 (s, 3H), 3.19 (s, 3H), 3.06 (t, 1H, J=12 Hz), 1.95 (m, 2H), 1.26 (m, 4H), 0.86 (t, 3H, J=7.5 Hz)

### Ex11: (2R,3R,4S,5R,6E)-7-cyclohexyl-3,4,5-trihydroxy-2-méthoxy-N-[(3R)-5-méthyl-4-oxo-2,3,4,5-tétrahydro-1,5-benzothiazépin-3-yl]hept-6-énamide

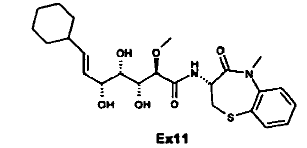

### Etape 1: Préparation du (3R,4R,5S)-5-[(1R,2E)-3-cyclohexyl-1-hydroxyprop-2-én-1-yl]-4-hydroxy-3-méthoxydihydrofuran-2(3H)-one (33)

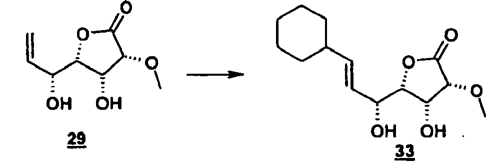

Dans un vial de 5 mL, introduire 100 mg (0,53 mmol) de **29**, 4 mL de CH₂Cl₂, 728 µL (5,3 mmol) de vinylcyclohexane puis 90,2 mg (106 µmol) de catalyseur de Grubbs de seconde génération. Chauffer la solution 10 min à 60 °C aux micro-ondes. Le solvant est ensuite évaporé à sec sous pression réduite puis le résidu est purifié sur colonne de silice Biotage 25-M (éluant : Hept/AcOEt 45/55). Le produit **33** (130,4 mg, Rf = 0.33 dans les conditions d'élution utilisées) est obtenu sous forme d'un solide beige.
MS : m/z (ES⁺) = 293 [M+Na]⁺
(ES⁻) = 315 [(M+HCOOH)-H]⁻, 269 [M-H]⁻
RMN ¹H (300 MHz, DMSO-d₆) δ (ppm) = 5.8 (dd, 1H, J=6 Hz et 16 Hz), 5.48 (dd, 1H, J=4.5 Hz et 16 Hz), 5.16 (br s, 2H), 4.29 (m, 2H), 4.25 (d, 1H, J=4.5 Hz), 3.95 (dd, 1H, J=3 Hz et 9 Hz), 3.42 (s, 3H), 1.96 (m, 1H), 1.66 (m, 4H), 1.15 (m, 6H)

### Etape 2: Préparation du (2R,3R,4S,5R,6E)-7-cyclohexyl-3,4,5-trihydroxy-2-méthoxy-N-[(3R)-5-méthyl-4-oxo-2,3,4,5-tétrahydro-1,5-benzothiazépin-3-yl]hept-6-énamide (Ex11)

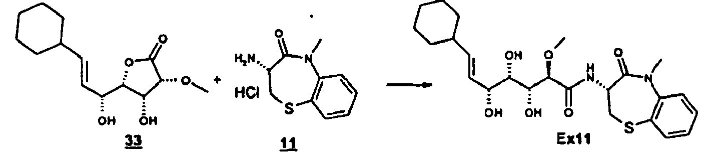

Dans un monocol de 20 mL, introduire 120 mg (444 µmol) de **33,** 130,4 mg (533 µmol) de **11**, 177 mg (1,065 mmol) de 2-éthylhexanoate de sodium dans 6 mL de THF. La solution est laissée sous agitation à température ambiante pendant 1 semaine. La solution est ensuite reprise avec 6 mL de CH₂Cl₂ puis lavée avec 2*5 mL d'HCl 0,5 N puis 5 mL d'eau. La phase aqueuse est ensuite extraite par 2*5 mL de CH₂Cl₂ puis les phases organiques sont réunies, séchées sur sulfate de sodium puis filtrées. Le solvant est ensuite évaporé à sec sous pression réduite puis le résidu est purifié sur colonne de silice Biotage 12-S (éluant : Hept/AcOEt 15/85). Le produit **Ex11** (91,1 mg, Rf = 0.24 dans les conditions d'élution utilisées) est obtenu sous forme d'un solide blanc.
MS : m/z (ES⁺) = 979 [2M+Na]⁺, 501 [M+Na]⁺, 479 [M+H]⁺,461 [(M-H₂O)+H]⁺
RMN ¹H (400 MHz, DMSO-d₆) : δ (ppm) = 8.28 (d, 1H, J=8 Hz), 7.70 (d, 1H, J=8Hz), 7.60 (m, 2H), 7.37 (m, 1H), 5.57 (dd, 1H, J=6.5 Hz et 15 Hz), 5.35 (dd, 1H, J=6.5 Hz et 15 Hz), 4.58 (br s, 1H), 4.44 (m, 1H), 4.35 (br s, 2H), 3.93 (t, 1H, J=6.5 Hz), 3.69 (d, 1H, J=8 Hz), 3.50 (m, 1H), 3.44 (m, 1H), 3.40 (m, 1H), 3.36 (s, 3H), 3.23 (s, 3H), 3.10 (t, 1H, J=12 Hz), 1.92 (m, 1H), 1.65 (m, 4H), 1.15 (m, 6H)

### Ex12: (2R,3R,4S,5R,6E)-7-(2-fluorophényl)-3,4,5-trihydroxy-2-méthoxy-N-[(3R)-5-méthyl-4-oxo-2,3,4,5-tétrahydro-1,5-benzothiazépin-3-yl]hept-6-énamide

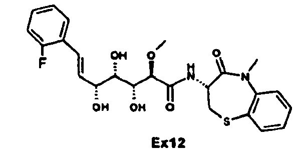

### Etape 1: Préparation du (3R,4R,5S)-5-[(1R,2E)-3-(2-fluorophényl)-1-hydroxyprop-2-én-1-yl]-4-hydroxy-3-méthoxydihydrofuran-2(3H)-one (34)

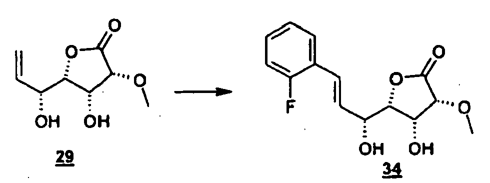

Dans un vial de 5 mL, introduire 100 mg (0,53 mmol) de **29,** 4 mL de CH₂Cl₂, 633 µL (5,3 mmol) de 2-fluorostyrène puis 90,2 mg (106 µmol) de catalyseur de Grubbs de seconde génération. Chauffer la solution 10 min à 60 °C au micro-ondes. Le solvant est ensuite évaporé à sec sous pression réduite puis le résidu est purifié sur colonne de silice biotage 25-M (éluant : Hept/AcOEt 40/60). Le produit **34** (64,4 mg, Rf = 0.24 dans les conditions d'élution utilisées) est obtenu sous forme d'un solide blanc.
MS : m/z (ES⁺) = 265 [(M-H₂O)+H]⁺
(ES⁻) = 327 [(M+HCOOH)-H]⁻, 281 [M-H]⁻
RMN ¹H (300 MHz, DMSO-d₆) : δ (ppm) = 7.61 (t, 1H, J=9 Hz), 7.31 (m, 1H), 7.19 (t, 2H, J=9 Hz), 6.89 (d, 1H, J=16.1 Hz), 6.50 (dd, 1H, J=4.9 Hz et 16.1 Hz), 5.55 (d, 2H, J=5.5 Hz), 4.49 (m, 1H), 4.42 (m, 1H), 4.28 (d, 1H, J=4.3 Hz), 4.09 (dd, 1H, J=2.8 Hz et 8.3 Hz), 3.43 (s, 3H)

### Etape 2: Préparation du (2R,3R,4S,5R,6E)-7-(2-fluorophényl)-3,4,5-trihydroxy-2-méthoxy-N-[(3R)-5-méthyl-4-oxo-2,3,4,5-tétrahydro-1,5-benzothiazépin-3-yl]hept-6-énamide (Ex12)

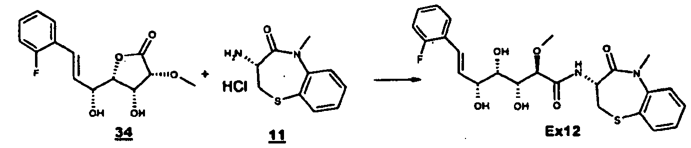

Dans un monocol de 30 mL, introduire 60 mg (212 µmol) de **34,** 62,4 mg (255 µmol) de **11,** 53 mg (319 µmol) de 2-éthylhexanoate de sodium dans 2,5 mL de THF. La solution est laissée sous agitation à température ambiante pendant 6 jours. La solution est ensuite reprise avec 5 mL de CH₂Cl₂ puis lavée avec 4 mL d'HCl 0,5 N puis 4 mL d'eau. La phase aqueuse est ensuite extraite par 2*5 mL de CH₂Cl₂ puis les phases organiques sont réunies, séchées sur sulfate de sodium puis filtrées. Le solvant est ensuite évaporé à sec sous pression réduite puis le résidu est purifié sur colonne de silice Biotage 12-M (éluant : CH₂Cl₂/iso-propanol 98/2). Le produit **Ex12** (45,7 mg, Rf = 0.37 dans les conditions d'élution utilisées) est obtenu sous forme d'un solide blanc.
MS : m/z (ES⁺) = 491 [M+H]⁺, 473 [(M-H₂O)+H]⁺
(ES⁻) = 535 [(M+HCOOH)-H]⁻, 489 [M-H]⁻
RMN ¹H (400 MHz, DMSO-d₆) : δ (ppm) = 8.27 (d, 1H, J=8 Hz), 7.65 (d, 1H, J=8Hz), 7.54 (m, 3H), 7.32 (m, 1H), 7.26 (m, 1H), 7.16 (m, 2H), 6.65 (d, 1H, J=16 H), 6.38 (dd, 1H, J=6 Hz et 16 Hz), 4.95 (d, 1H, J=4 Hz), 4.55 (d, 1H, J=6 Hz), 4.47 (d, 1H, J=6.5 Hz), 4.41 (m, 1H), 4.18 (m, 1H), 3.70 (d, 1H, J=8 Hz), 3.53 (m, 1H), 3.44 (m, 1H), 3.42 (m, 1H), 3.28 (s, 3H), 3.20 (s, 3H), 3.07 (t, 1H, J=12 Hz)

### Ex13: (2R,3R,4S,5R,6E)-7-(4-fluorophényl)-3,4,5-trihydroxy-2-méthoxy-N-[(3R)-5-méthyl-4-oxo-2,3,4,5-tétrahydro-1,5-benzothiazépin-3-yl]hept-6-énamide

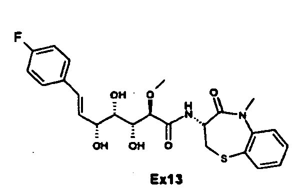

### Etape 1: Préparation du (3R,4R,5S)-5-[(1R,2E)-3-(4-fluorophényl)-1-hydroxyprop-2-én-1-yl]-4-hydroxy-3-méthoxydihydrofuran-2(3H)-one (35)

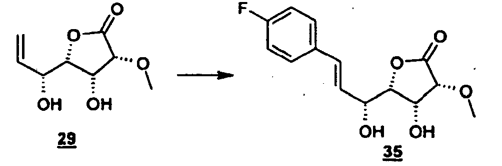

Dans un vial de 5 mL, introduire 100 mg (0,53 mmol) de **29**, 4 mL de CH₂Cl₂, 636 µL (5,3 mmol) de 4-fluorostyrène puis 90,2 mg (106 µmol) de catalyseur de Grubbs de seconde génération. Chauffer la solution 10 min à 60 °C au micro-ondes. Le solvant est ensuite évaporé à sec sous pression réduite puis le résidu est purifié sur colonne de silice Biotage 25-M (éluant : CH₂Cl₂/ iso-propanol 9/1). Le produit **35** (99,2 mg, Rf = 0.15 dans les conditions d'élution utilisées) est obtenu sous forme d'un solide blanc.
MS : m/z (%) = 282 [MH⁺], 152 (100)
RMN ¹H (300 MHz, DMSO-d₆): δ (ppm) = 7.5 (dd, 2H, J=6 Hz et 9 Hz,), 7.17 (t, 2H, J=9 Hz), 6.76 (d, 1H, J=16 Hz), 6.33 (dd, 1H, J=6 Hz et 15 Hz), 5.52 (br s, 1H), 5.46 (d, 1H, J=5.5 Hz), 4.46 (m, 1H), 4.42 (m, 1H), 4.28 (d, 1H, J=4.5 Hz), 4.08 (dd, 1H, J=3 Hz et 9 Hz), 3.42 (s, 3H)

### Etape 2: Préparation du (2R,3R,4S,5R,6E)-7-(4-fluorophényl)-3,4,5-trihydroxy-2-méthoxy-N-[(3R)-5-méthyl-4-oxo-2,3,4,5-tétrahydro-1,5-benzothiazépin-3-yl]hept-6-énamide (Ex13)

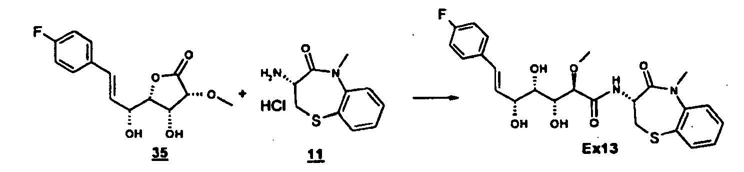

Dans un monocol de 30 mL, introduire 90 mg (319 µmol) de **35,** 93,6 mg (383 µmol) de **11**, 127,2 mg (765 µmol) de 2-éthylhexanoate de sodium dans 4 mL de THF. La solution est laissée sous agitation à température ambiante pendant 28 heures. La solution est ensuite reprise avec 6 mL de CH₂Cl₂ puis lavée avec 5 mL d'HCl 0,5 N puis 5 mL d'eau. La phase aqueuse est ensuite extraite par 2*5 mL de CH₂Cl₂ puis les phases organiques sont réunies, séchées sur sulfate de sodium puis filtrées. Le solvant est ensuite évaporé à sec sous pression réduite puis le résidu est purifié sur colonne de silice Biotage 12-M (éluant : Hept/AcOEt 15/85). Le produit **Ex13** (55,5 mg, Rf = 0.17 dans les conditions d'élution utilisées) est obtenu sous forme d'un solide blanc.
MS : m/z (ES⁺) = 513 [M+Na]⁺, 491 [M+H]⁺
(ES⁻) = 535 [(M+HCOOH)-H]⁻, 489 [M-H]⁻
RMN ¹H (300 MHz, DMSO-d₆) : δ (ppm) = 8.26 (d, 1H, J=8 Hz), 7.65 (d, 1H, J=8Hz), 7.56 (m, 2H), 7.43 (dd, 2H, J=6 Hz et 9 Hz), 7.32 (m, 1H), 7.13 (t, 2H, J=9 Hz), 6.52 (d, 1H, J=16 Hz), 6.20 (dd, 1H, J=6 Hz et 16 Hz), 4.86 (d, 1H, J=4 Hz), 4.49 (d, 1H; J=6 Hz), 4.44 (d, 1H, J=6.5 Hz), 4.40 (m, 1H), 4.15 (m, 1H), 3.70 (d, 1H, J=8 Hz), 3.53 (m, 1H), 3.47 (m, 1H), 3.45 (m, 1H), 3.28 (s, 3H), 3.19 (s, 3H), 3.07 (t, 1H, J=12 Hz)

### Ex14: (2R,3R,4S,5R,6E)-3,4,5-trihydroxy-2-méthoxy-N-[(3R)-5-méthyl-4-oxo-2,3,4,5-tétrahydro-1,5-benzothiazépin-3-yl]-7-phénylhept-6-ënamide

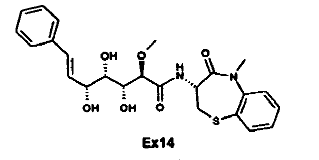

### Etape 1: Préparation du (3R,4R,5S)-4-hydroxy-5-[(1R,2E)-1-hydroxy-3-phénylprop-2-én-1-yl]-3-méthoxydihydrofuran-2(3H)-one) (36)

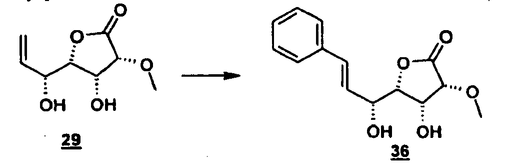

Dans un vial de 5 mL, introduire 100 mg (0,53 mmol) **29,** 4 mL de CH₂Cl₂, 615 µL (5,3 mmol) de styrène puis 90,2 mg (106 µmol) de catalyseur de Grubbs de seconde génération. Chauffer la solution 10 min à 60 °C au micro-ondes. Le solvant est ensuite évaporé à sec sous pression réduite puis le résidu est purifié sur colonne de silice Biotage 25-M (éluant : CH₂Cl₂/ Iso-propanol 98/2). Le produit **36** (79,6 mg, Rf = 0.24 dans les conditions d'élution utilisées) est obtenu sous forme d'un solide marron.
MS : m/z (ES⁻) = 527 [2M-H]⁻, 309 [(M+HCOOH)-H]⁻, 263 [M-H]⁻
RMN ¹H (300 MHz, DMSO-d₆): δ (ppm) = 7.45 (d, 2H, J=7.5 Hz), 7.35 (t, 2H, J=7.5 Hz), 7.25 (t, 1H, J=7.5 Hz), 6.77 (d, 1H, J=16 Hz), 5.88 (dd, 1H, J=5.5 Hz et 16 Hz), 5.54 (d, 1H, J=3 Hz), 5.46 (d, 1H, J=5 Hz), 4.48 (m, 1H), 4.42 (m, 1H), 4.48 (d, 1H, J=4 Hz), 4.09 (dd, 1H, J=3 Hz et 9 Hz), 3.41 (s, 3H)

### Etape 2: Préparation du (2R,3R,4S,5R,6E)-3,4,5-trihydroxy-2-méthoxy-N-[(3R)-5-méthyl-4-oxo-2,3,4,5-tétrahydro-1,5-benzothiazépin-3-yl]-7-phénylhept-6-énamide (Ex14)

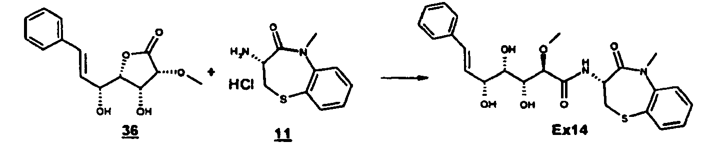

Dans un monocol de 20 mL, introduire 70 mg (265 µmol) de **36**, 77,8 mg (318 µmol) de **11,** 106 mg (636 µmol) de 2-éthylhexanoate de sodium dans 4 mL de THF. La solution est laissée sous agitation à température ambiante pendant 43 heures. La solution est ensuite reprise avec 6 mL de CH₂Cl₂ puis lavée avec 2*5 mL d'HCl 0,5 N puis 5 mL d'eau. La phase aqueuse est ensuite extraite par 2*5 mL de CH₂Cl₂ puis les phases organiques sont réunies, séchées sur sulfate de sodium puis filtrées. Le solvant est ensuite évaporé à sec sous pression réduite puis le résidu est purifié sur colonne de silice Biotage 12-S (éluant : Hept/AcOEt 15/85). Le produit **Ex14** (62,4 mg, Rf = 0.3 dans les conditions d'élution utilisées) est obtenu sous forme d'un solide blanc.
MS : m/z (ES⁺) = 495 [M+Na]⁺, 473 [M+H]⁺
(ES⁺) = 517 [(M+HCOOH)-H]⁻, 471 [M-H]⁻
RMN ¹H (300 MHz, DMSO-d₆) : δ (ppm) = 8.26 (d, 1H, J=8 Hz), 7.65 (d, 1H, J=8Hz), 7.55 (m, 2H), 7.39 (d, 2H, J= 6.5 Hz), 7.33 (m, 2H), 7.29 (m, 1H), 7.21 (t, 1H), 6.53 (d, 1H, J=16 Hz), 6.25 (dd, 1H, J=6 Hz et 16 Hz), 5.34 (d, 1H, J=4.5 Hz), 4.48 (d, 1H, J=6 Hz), 4.44 (d, 1H, J=6.5 Hz), 4.38 (m, 1H), 4.15 (m, 1H), 3.70 (d, 1H, J=7.5 Hz), 3.53 (m, 1H), 3.46 (m, 1H), 3.42 (m, 1H), 3.32 (s, 3H), 3.20 (s, 3H), 3.06 (t, 1H, J=12 Hz)

### Ex15: (2R,3R,4S,5R,6E)-3,4,5-trihydroxy-2-méthoxy-8,8-diméthyl-N-[(3R)-4-oxo-9-phényl-2,3,4,5-tétrahydro-1,5-benzothiazépin-3-yl]non-6-énamide

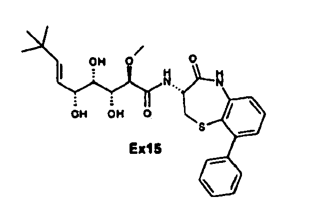

### Etape 1: Préparation du 2-fluoro-3-nitrobiphényl (37)

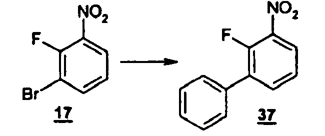

Dans un ballon de 50mL, sous agitation et atmosphère d'argon, contenant 10 mL d'eau, 3 mL de dioxane et 500 mg de **17** (2,273 mmol), on introduit 277 mg d'acide phenylboronique (2,273 mmol), 46,41 mg de chlorure de 1,1'-bis(diphenylphosphino)ferrocene palladium (C35H30C14FeP2Pd, PM 816,65, 0,057 mmol) et 2,96 g de carbonate de Césium (9,09 mmol). On chauffe à 100°C le milieu sous agitation pendant 3 h.. On ajoute ensuite 20 ml d'AcOEt et lave avec 2 fois 20 mL d'eau. La phase organique est séchée sur MgSO4, filtrée et évaporé à sec. Le brut est chromatographié sur une cartouche de silice (30 g) en éluant avec Heptane/AcOEt (*en gradient AcOEt : 10 à 50%*). On recueille 360 mg de produit attendu **37** (Huile Jaune).
EI : 217⁺
RMN ¹H (400 MHz, DMSO-d₆), δ(ppm): De 7,45 à 7,63 (m, 6H) ; 7,91 (dt, J = 2,0 et 9,0 Hz, 1H) ; 8,14 (dt, J = 2,0 et 9,0 Hz, 1H).

### Etape 2: Préparation de N-[(2,2-diméthylpropanoyl)oxy]-S-(3-nitrobiphényl-2-yl)-L-cystéine (38)

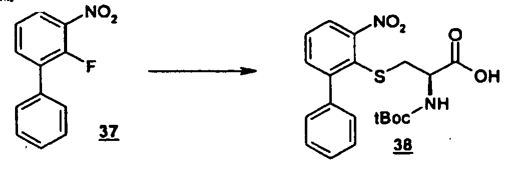

Dans un tricol de 25 mL contenant 367 mg de L-Boc-Cys-OH (1,657 mmol), 2,5 mL d'eau et 402 mg de NaHCO₃ (4,789 mmol), on introduit goutte à goutte une solution de **37** (360 mg, 1,657 mmol) dans 2,8 mL d'éthanol. On porte le milieu au reflux pendant 6 h, puis évapore l'éthanol et ajoute 5 mL d'eau distillée. On lave ensuite 2 fois la phase aqueuse avec 5 mL d'éther, une fois la phase éthérée décantée, on amène la phase aqueuse jusqu'à pH2-3 avec du HCl (1N), et extrait 2 fois avec 5 mL d'AcOEt. Les phases organiques sont réunies, séchées sur MgSO₄, filtrées et enfin évaporées à sec. On obtient 0.81 g de produit brut **38** qui est utilisé directement pour le stade suivant.
RMN ¹H (400 MHz, DMSO-d₆), δ(ppm): 1,32 (s, 9H) ; de 2,39 à 2,57 (m partiellement masqué, 2H) ; 3,57 (m, 1H) ; 6,77 (d, J = 8,0 Hz, 1H) ; de 7,40 à 7,55 (m, 5H) ; 7,59 (d large, J = 8,0 Hz, 1H) ; 7,65 (t, J = 8,0 Hz, 1H) ; 7,86 (d large, J = 8,0 Hz, 1H) ; 12,15 (m étalé, 1H).

### Etape 3: Préparation de S-(3-aminobiphényl-2-yl)-N-[(2,2-diméthylpropanoyl)oxy]-L-cystéine (39)

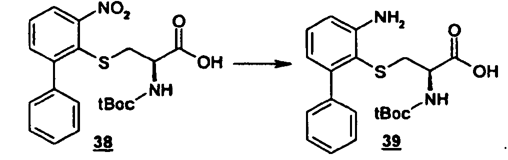

Dans un autoclave contenant 810 mg de **38** (1,936 mmol), 69,84 mg de Pd/C (10%) et 20 mL de MeOH, on hydrogène sous 5 bars pendant 10 h à 20°C. Après filtration du catalyseur sur célite, on évapore le solvant et obtient 580 mg de produit attendu **39** qui est utilisé directement pour l'étape suivante. RMN ¹H (400 MHz, DMSO-d₆), δ(ppm): 1,33 (s, 9H) ; de 2,60 à 2,75 (m, 2H) ; 3,81 (m, 1H) ; 5,60 (m très étalé, 2H) ; 6,47 (d, J = 8,0 Hz, 1H) ; 6,78 (d, J = 8,0 Hz, 1H) ; 6,89 (d, J = 8,0 Hz, 1H) ; 7,09 (t, J = 8,0 Hz, 1H) ; de 7,24, à 7,41 (m, 5H) ; 12,2 (m très étalé, 1H).

### Etape 4: Préparation du (3R)-3-{[(2,2-diméthylpropanoyl)oxy]amino}-9-phényl-2,3-dihydro-1,5-benzothiazépin.4(5H)-one (40)

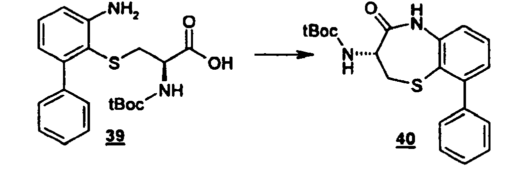

Dans un tricol de 25 mL contenant 580 mg de **39** (1,493 mmol), 7 mL de DMF, on introduit, à 0°C, 0,269 ml de Cyanophosphonate de diéthyle (289,4 mg, 1,774 mmol), et 10 minute après, 0,201 ml de TEA (1,43 mmol). On agite le milieu réactionnel à 0°C pendant 2 h. On ajoute 20 ml d'AcoEt, puis lave avec 2 fois 20 mL d'eau distillée. La phase organique est séchée sur MgSO₄. filtrée puis évaporée à sec. Le brut (600 mg d'huile jaune) est chromatographié sur une cartouche de silice (30 g) avec un éluant Heptane/AcOEt (*en gradient AcOEt : 10* à *50%*). On obtient 410 mg de produit attendu **40** (solide blanc)..
RMN ¹H (400 MHz, DMSO-d₆), δ(ppm): 1,35 (s, 9H) ; 3,02 (t, J = 12,0 Hz, 1H) ; 3,45 (dd, J = 7,5 et 12,0 Hz, 1H) ; 4,20 (m, 1H) ; de 7,12 à 7,25 (m, 3H) ; de 7,33 à 7,51 (m, 6H) ; 10,05 (s, 1H).

### Etape 4: Préparation du (3R)-3-amino-9-phényl-2,3-dihydro-1,5. benzothiazépin-4(5H)-one chlorhydrate (41)

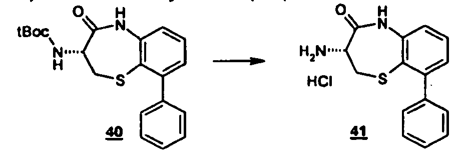

Dans un ballon de 50 mL contenant 410 mg de **40** (1,107 mmol), on ajoute 8 mL d'une solution d'acide chlorhydrique dans le dioxane (4M). On agite durant 4 h à TA sous argon. Un précipité blanc se forme, qui est essoré, lavé avec 3 mL de dioxane, puis 5 mL d'éther isopropylique. On obtient ainsi 260 mg d'amine **41** (solide blanc) sous forme de chlorhydrate.
RMN ¹H (300 MHz, DMSO-d₆), δ(ppm): 3,17 (t, J = 12,0 Hz, 1H) ; 3,65 (dd, J = 8,0 et 12,0 Hz, 1H) ; 4,08 (dd, J = 8,0 et 12,0 Hz, 1H) ; 7,21 (dd, J = 1,5 et 8,0 Hz, 1H) ; 7,28 (dd, J = 1,5 et 8,0 Hz, 1H) ; de 7,35 à 7,49 (m, 5H) ; 7,52 (t, J = 8,0 Hz, 1H) ; 8,39 (s large, 3H) ; 10,65 (s, 1H).

### Etape 5: Préparation du (2R,3R,4S,5R,6E)-3,4,5-trihydroxy-2-méthoxy-8,8-diméthyl-N-[(3R)-4-oxo-9-phényl-2,3,4,5-tétrahydro-1,5-benzothiazépin-3-yl]non-6-énamide (Ex15)

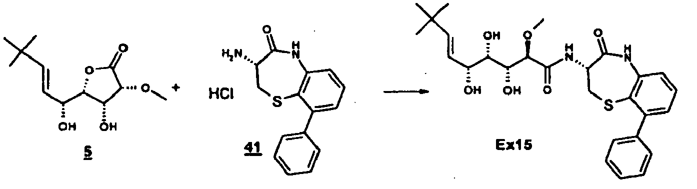

On introduit successivement dans un ballon de 25 mL, sous agitation et sous atmosphère d'argon, 100 mg de **5** (409 µmol), 151 mg de **41** (491 µmol), 102 mg de 2-éthylhexanoate de sodium (0,61 mmol) dans 2,3 mL de THF. On maintient l'agitation à TA pendant 24 h. On concentre le milieu réactionnel à sec. Les résidus sont chromatographiés sur une cartouche de silice (15 g, éluant CH₂Cl₂/MeOH *en gradient : MeOH 1* à *10%*). On recueille 145 mg de produit attendu **Ex15.**
ES: m/z = 513 (M-H⁺)
RMN ¹H (400 MHz, DMSO-d₆), δ(ppm): 0,96 (s, 9H) ; 3,10 (t, J = 12,0 Hz, 1H) ; 3,22 (s, 3H) ; 3,30 (m masqué, 1H) ; de 3,40 à 3,55 (m, 2H) ; 3,69 (d, J = 8,0 Hz, 1H) ; 3,94 (m, 1H) ; 4,29 (m, 2H) ; 4,54 (m, 2H) ; 5,30 (dd, J = 7,0 et 16,0 Hz, 1H) ; 5,63 (d, J = 16,0 Hz, 1H) ; 7,19 (d large, J = 7,5 Hz, 1H) ; 7,23 (d large, J = 7,5 Hz, 1H) ; de 7,35 à 7,46 (m, 5H) ; 7,49 (t, J = 7,5 Hz, 1H) ; 8,25 (d, J = 8,0 Hz, 1H) ; 10,2 (s, 1H).

### Ex16: (2R,3R,4S,5R,6E)-N-[(3R)-9-(3-cyanophényl)-4-oxo-2,3,4,5-tétrahydro-1,5-benzothiazépin-3-yl]-3,4,5-trihydroxy-2-méthoxy-8,8-diméthylnon-6-énamide

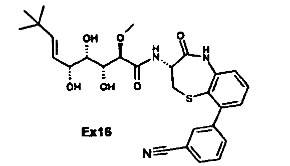

### Etape 1: Préparation du 2'-fluoro-3'-nitrobiphényl-3-carbonitrile (42)

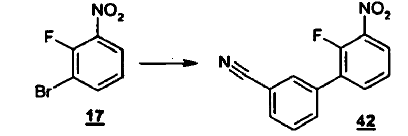

Dans un ballon de 50mL, sous agitation et atmosphère d'argon, contenant 10 mL d'eau, 3 mL de dioxane et 500 mg de **17** (2,273 mmol), on introduit 334 mg d'acide 3-cyano-phenylboronique (2,273 mmol), 46,41 mg de chlorure de 1,1'-bis(diphenylphosphino)ferrocene palladium (C35H30Cl4FeP2Pd, PM 816,65, 0,057 mmol) et 2,96 g de carbonate de Césium (9,09 mmol). On chauffe à 100°C le milieu sous agitation pendant 3 h.. On ajoute ensuite 20 ml d'AcOEt et lave avec 2 fois 20 mL d'eau. La phase organique est séchée sur MgSO4, filtrée et évaporée à sec. Le brut est chromatographié sur une cartouche de silice (30 g) en éluant avec Heptane/AcOEt (*en gradient AcOEt : 10 à 50%*). On recueille 230 mg de produit attendu **42** (solide blanc).
RMN ¹H (500 MHz, DMSO-d₆), δ(ppm): 7,59 (t, J = 8,0 Hz, 1H) ; 7,77 (t, J = 8,0 Hz, 1H) ; de 7,93 à 8,04 (m, 3H) ; 8,12 (s large, 1H) ; 8,22 (dt, J = 2,0 et 8,0 Hz, 1H).

### Etape 2: Préparation de S-(3'-cyano-3-nitrobiphényl-2-yl)-N-[(2,2-diméthylpropanoyl)oxy]-L-cystéine (43)

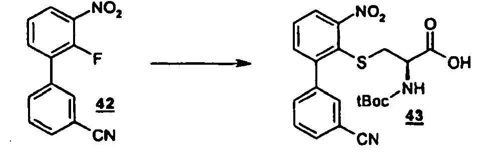

Dans un tricol de 25 mL contenant 210 mg de L-Boc-Cys-OH (0,950 mmol), 2,5 mL d'eau et 231 mg de NaHCO₃ (2,746 mmol), on introduit goutte à goutte une solution de **42** (360 mg, 1,657 mmol) dans 3,0 mL d'éthanol. On porte le milieu au reflux pendant 2 h, puis évapore l'éthanol et ajoute 5 mL d'eau distillée. On lave ensuite 2 fois la phase aqueuse avec 10 mL d'éther, une fois la phase éthérée décantée, on amène la phase aqueuse jusqu'à pH2-3 avec du HCl (1N), et extrait 2 fois avec 10 mL d'AcOEt. Les phases organiques sont réunies, séchées sur MgSO₄, filtrées et enfin évaporées à sec. On obtient 0.41 g de produit brut **43** qui est utilisé directement pour le stade suivant.
RMN ¹H (400 MHz, DMSO-d₆), δ(ppm): 1,33 (s, 9H); de 2,47 à 2,60 (m partiellement masqué, 2H) ; 3,54 (m, 1H) ; 6,79 (d, J = 8,0 Hz, 1H) ; de 7,63 à 7,73 (m, 3H) ; de 7,84 à 7,95 (m, 3H) ; 7,98 (s large, 1H) ; 12,55 (m étalé, 1H).

### Etape 3: Préparation de S-(3-amino-3'-cyanobiphényl-2-yl)-N-[(2,2-diméthylpropanoyl)oxy]-L-cystéine (44)

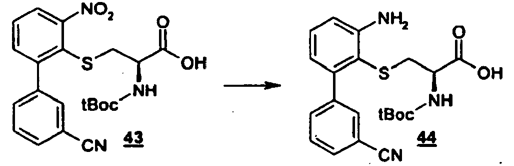

Dans un autoclave contenant 410 mg de **43** (0,925 mmol), 41 mg de Pd/C (10%) et 20 mL de MeOH, on hydrogène sous 7 bars pendant 10 h à 20°C. Après filtration du catalyseur sur célite. On évapore le solvant et obtient 350 mg de produit attendu 44 qui est utilisé directement pour l'étape suivante. RMN ¹H (400 MHz, DMSO-d₆), δ(ppm): 1,32 (s, 9H) ; de 2,60 à 2,79 (m, 2H) ; 3,72 (m, 1H) ; 5,70 (m très étalé, 2H) ; 6,48 (d large, J = 8,0 Hz, 1H) ; 6,70 (d large, J = 8,0 Hz, 1H) ; 6,80 (d large, J = 8,0 Hz, 1H) ; 7,11 (t, J = 8,0 Hz, 1H) ; de 7,52 à 7,83 (m, 4H) ; 12,5 (m très étalé, 1H).

### Etape 4: Préparation du 3-[(3R)-3-{[(2,2-diméthylpropanoyl)oxy]amino}-4-oxo-2,3,4,5-tétrahydro-1,5-benzothiazépin-9-yl]benzonitrile (45)

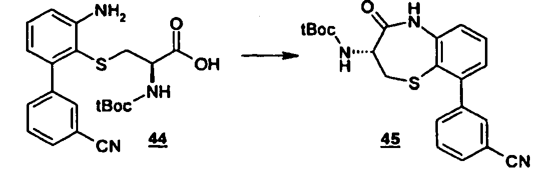

Dans un tricol de 25 mL contenant 350 mg de **41** (0,846 mmol), 4,1 mL de DMF, on introduit, à 0°C, 0,153 ml de Cyanophosphonate de diéthyle (0,164 mg, 1,01 mmol), et 10 minute après, 0,114 ml de TEA (0,811 mmol). On agite le milieu réactionnel à 0°C pendant 2 h. On ajoute 20 ml d'AcoEt, puis lave avec 2 fois 20 mL d'eau distillée. La phase organique est séchée sur MgSO₄, filtrée puis évaporée à sec. Le brut est chromatographié sur une cartouche de silice (15 g) avec un éluant CH₂Cl₂/MeOH (*en gradient MeOH : 0 à 3%*). On obtient 139 mg de produit attendu **45** (solide jaune orangé).
RMN ¹H (400 MHz, DMSO-d₆), δ(ppm): 1,34 (s, 9H) ; 3,02 (t, J = 12,0 Hz, 1H) ; 3,54 (dd, J = 7,5 et 12,0 Hz, 1H) ; 4,21 (m, 1H) ; de 7,19 à 7,30 (m, 3H) ; 7,50 (t, J = 8,0 Hz, 1H) ; 7,63 (t, J = 8,0 Hz, 1H) ; 7,79 (d large, J = 8,0 Hz, 1H) ; 7,83 (d large, J = 8,0 Hz, 1H) ; 7,98 (s large, 1H) ; 10,05 (s, 1H).

### Etape 4: Préparation du 3-[(3R)-3-amino-4-oxo-2,3,4,5-tétrahydro-1,5-benzothiazépin-9-yl]benzonitrile chlorhydrate (46)

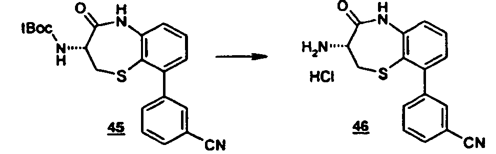

Dans un ballon de 25 mL contenant 139 mg de **45** (0,352 mmol), on ajoute 2,6 mL d'une solution d'acide chlorhydrique dans le dioxane (4M). On agite durant 4 h à TA sous argon. Un précipité blanc se forme, qui est essoré, lavé avec 3 mL de dioxane, puis 5 mL d'éther isopropylique. On obtient ainsi 72 mg d'amine **46** (solide blanc) sous forme de chlorhydrate.
RMN ¹H (300 MHz, DMSO-d₆), δ(ppm): 3,19 (t, J = 12,0 Hz, 1H) ; 3,68 (dd, J = 7,5 et 12,0 Hz, 1H) ; 4,15 (dd, J = 7,5 et 12,0 Hz, 1H) ; 7,25 (d large, J = 8,0 Hz, 1H) ; 7,32 (d large, J = 8,0 Hz, 1H) ; 7,57 (t, J = 8,0 Hz, 1H) ; 7,69 (t, J = 8,0 Hz, 1H) ; 7,77 (d large, J = 8,0 Hz, 1H) ; 7,89 (d large, J = 8,0 Hz, 1H) ; 7,92 (s large, 1H) ; 8,31 (s large, 3H) ; 10,7 (s, 1H).

### Etape 5: Préparation du (2R,3R,4S,5R,6E)-N-[(3R)-9-(3-cyanophényl)-4-oxo-2,3,4,5-tétrahydro-1,5-benzothiazépin-3-yl]-3,4,5-trihydroxy-2-méthoxy-8,8-diméthylnon-6-énamide (Ex16)

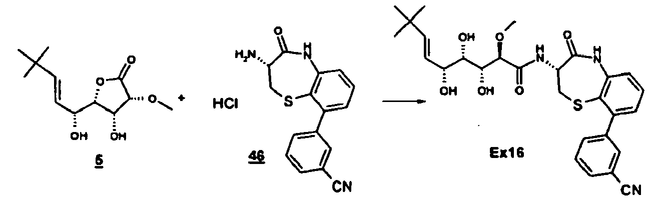

On introduit successivement dans un tube Wheatton, sous agitation et sous atmosphère d'argon, 55 mg de **5** (225 µmol), 74,7 mg de **46** (225 µmol), 74 mg de 2-éthylhexanoate de sodium (0,44 mmol) dans 1,3 mL de THF. On maintient l'agitation à TA pendant 48 h. On concentre le milieu réactionnel à sec. Les résidus sont chromatographiés sur une cartouche de silice (5 g, éluant CH₂Cl₂/MeOH *en gradient : MeOH 1 à 10%*). On recueille 100 mg de produit attendu **Ex16** (solide blanc).
RMN ¹H (400 MHz, DMSO-d₆), δ(ppm): 0,96 (s, 9H) ; 3,12 (t, J = 12,0 Hz, 1H) ; 3,22 (s, 3H) ; 3,30 (m masqué, 1H) ; de 3,53 à 3,57 (m, 2H) ; 3,69 (d, J = 8,0 Hz, 1H) ; 3,94 (m, 1H) ; 4,24 (d, J = 7,0 Hz, 1H) ; 4,29 (d large, J = 6,5 Hz, 1H) ; 4,52 (m, 2H) ; 5,30 (dd, J = 7,0 et 16,0 Hz, 1H) ; 5,63 (d, J = 16,0 Hz, 1H) ; 7,23 (d large, J = 7,5 Hz, 1H) ; 7,30 (d large, J = 7,5 Hz, 1H) ; 7,52 (t, J = 7,5 Hz, 1H) ; 7,65 (t, J = 7,5 Hz, 1H) ; 7,75 (d large, J = 7,5 Hz, 1H) ; 7,85 (d large, J = 7,5 Hz, 1H) ; 7,91 (s large, 1H) ; 8,29 (d, J = 8,0 Hz, 1H) ; 10,2 (s, 1H).

### Ex17: (2R,3R,4S,5R,6E)-N-[(3R)-6-fluoro-4-oxo-2,3,4,5-tétrahydro-1,5. benzothiazépin-3-yl]-3,4,5-trihydroxy-2-méthoxy-8,8-diméthylnon-6-énamide

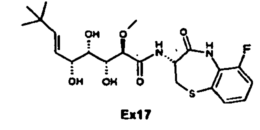

### Etape 1: Préparation N-[(2,2-diméthylpropanoyl)oxy]-S-(3-fluoro-2-nitrophényl)-L-cystéine (48)

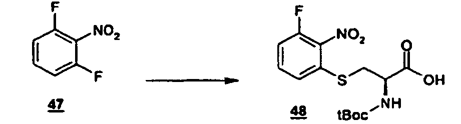

Dans un tricol de 50 mL contenant 2,78 g de L-Boc-Cys-OH (12,57 mmol), 22 mL d'eau et 3,05 g de NaHCO₃ (36,33 mmol), on introduit une solution de 2;6-difluoro-nitrobenzène **47** (2,0 g, 12,57 mmol) dans 26 mL d'éthanol. On laisse le milieu réactionnel à TA pendant 1 nuit, puis porte le milieu à 75°C pendant 1 h. On évapore l'éthanol, lave ensuite la phase aqueuse avec 20 mL d'éther, une fois la phase éthérée décantée, on amène la phase aqueuse jusqu'à pH2-3 avec du HCl (1 N), et extrait 2 fois avec 25 mL d'AcOEt. Les phases organiques sont réunies, séchées sur MgSO₄, filtrées et enfin évaporées à sec. On obtient 1,29 g de produit brut **48** (huile jaune) qui est utilisé directement pour le stade suivant.
RMN ¹H (400 MHz, DMSO-d₆), δ(ppm): 1,36 (s, 9H) ; 3,24 (dd, J = 10,0 et 13,5 Hz, 1H) ; 3,53 (dd, J = 5,0 et 13,5 Hz, 1H) ; 4,08 (m, 1H) ; 7,20 (d, J = 8,0 Hz, 1H) ; 7,44 (t, J = 8,0 Hz, 1H) ; 7,57 (t, J = 8,0 Hz, 1H) ; 7,69 (dt, J = 6,0 et 8,0 Hz, 1H) ; 12,8 (m étalé, 1H).

### Etape 2: Préparation de S-(2-amino-3-fluorophényl)-N-[(2,2-diméthylpropanoyl)oxy]-L-cystéine (49)

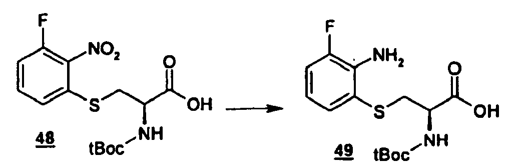

Dans un autoclave contenant 790 mg de **48** (2,192 mmol), 79 mg de Pd/C (10%) et 20 mL d'éthanol, on hydrogène sous 7 bars pendant 10 h à 20°C. Après filtration du catalyseur sur célite, on évapore le solvant et obtient 670 mg de produit attendu **49** (huile jaune) qui est utilisé directement pour l'étape suivante.
ES : m/z = 329 (M-H⁺)
RMN ¹H (400 MHz, DMSO-d₆), δ(ppm): 1,38 (s, 9H) ; 2,94 (dd, J = 10,0 et 13,5 Hz, 1H) ; 3,09 (dd, J = 5,0 et 13,5 Hz, 1H) ; 3,94 (m, 1H) ; 5,30 (s large, 2H) ; 6,52 (dt, J = 6,0 et 8,0 Hz, 1H) ; de 6,99 à 7,15 (m, 4H).

### Etape 3 Préparation du (3R)-3-{[(2,2-diméthylpropanoyl)oxy]amino}-6-fluoro-2,3-dihydro-1,5-benzothiazépin-4(5H)-one (50)

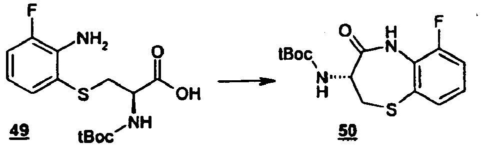

Dans un tricol de 25 mL contenant 670 mg de **49** (2,028 mmol), 10 mL de DMF, on introduit, à 0°C, 0,366 ml de Cyanophosphonate de diéthyle (0,393 mg, 2,41 mmol), et 10 minute après, 0,273 ml de TEA (1,943 mmol). On agite le milieu réactionnel à 0°C pendant 2 h. On ajoute 30 ml d'AcOEt, puis lave avec 2 fois 30 mL d'eau distillée. La phase organique est séchée sur MgS0₄, filtrée puis évaporée à sec. Le brut est chromatographié sur une cartouche de silice (70 g) avec un éluant Heptane/AcOEt (*en gradient AcOEt : 8 à 50%*). On obtient 68,4 mg de produit attendu **50** (solide blanc).
ES : m/z = 311 (M-H⁺)
RMN ¹H (400 MHz, DMSO-d₆), δ(ppm): 1,33 (s, 9H) ; 3,09 (t, J = 12,0 Hz, 1H) ; 3,52 (dd, J = 8,0 et 12,0 Hz, 1H) ; 4,09 (m, 1H) ; 7,27 (m, 2H) ; 7,39 (t, J = 9,0 Hz, 1H) ; 7,44 (d, J = 8,0 Hz, 1H) ; 9,96 (s, 1H).

### Etape 4: Préparation du (3R)-3-amino-6-fluoro-2,3-dihydro-1,5-benzothiazépin-4(5H)-one chlorhydrate (51)

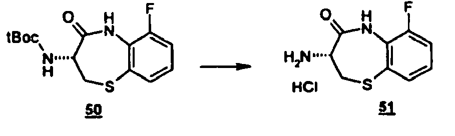

Dans un ballon de 25 mL contenant 68,4 mg de **50** (0,219 mmol), on ajoute 1,6 mL d'une solution d'acide chlorhydrique dans le dioxane (4M). On agite durant 4 h à TA sous argon. Apres avoir concentré le milieu réactionnel à sec, on obtient ainsi 51 mg d'amine **51** (solide jaune) sous forme de chlorhydrate. RMN ¹H (300 MHz, DMSO-d₆), δ(ppm): 3,23 (t, J = 12,0 Hz, 1H) ; 3,75 (dd, J = 7,5 et 12,0 Hz, 1H) ; 4,03 (dd, J = 7,5 et 12,0 Hz, 1H) ; 7,32 (dt, J = 6,0 et 8,0 Hz, 1H) ; 7,43 (t, J = 8,0 Hz, 1H) ; 7,50 (d, J = 8,0 Hz, 1H) ; 8,42 (s large, 3H) ; 10,55 (s, 1H).

### Etape 5: Préparation du (2R,3R,4S,5R,6E)-N-[(3R)-6-fluoro-4-oxo-2,3,4,5-tétrahydro-1,5-benzothiazépin-3-yl]-3,4,5-trihydroxy-2-méthoxy-8,8-diméthylnon-6-énamide (Ex17)

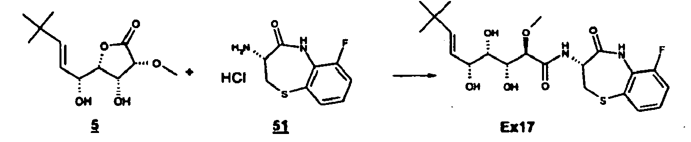

On introduit successivement dans un tube Wheatton, sous agitation et sous atmosphère d'argon, 50 mg de **5** (204 µmol), 51 mg de **51** (204 µmol), 69 mg de 2-éthylhexanoate de sodium (0,42 mmol) dans 1,2 mL de THF. On maintient l'agitation à TA pendant 48 h. On concentre le milieu réactionel à sec. Les résidus sont chromatographiés sur une cartouche de silice (5 g, éluant CH₂Cl₂/MeOH *en gradient : MeOH 1 à 10%*). On recueille 70 mg de produit attendu **Ex17** (solide blanc).
ES: m/z = 457 (MH⁺)
RMN ¹H (400 MHz, DMSO-d₆), δ(ppm): 0,96 (s, 9H) ; 3,18 (t, J = 12,0 Hz, 1H) ; 3,21 (s, 3H) ; 3,30 (m masqué, 1H) ; de 3,45 à 3,59 (m, 2H) ; 3,68 (d, J = 8,0 Hz, 1H) ; 3,92 (m, 1H) ; 4,29 (d, J = 7,5 Hz, 2H) ; 4,42 (m, 1H) ; 4,52 (d, J = 5,0 Hz, 1H) ; 5,29 (dd, J = 7,5 et 16,0 Hz, 1H) ; 5,62 (d, J = 16,0 Hz, 1H) ; 7,29 (m, 1H) ; 7,40 (t, J = 7,5 Hz, 1H) ; 7,48 (d, J = 7,5 Hz, 1H) ; 8,23 (d, J = 8,0 Hz, 1H) ; 10,1 (s, 1H).

### Ex18: (2R,3R,4S,5R,6E)-3,4,5-trihydroxy-2-méthoxy-N-[(3R)-6-méthoxy-4-oxo-2,3,4,5-tétrahydro-1,5-benzothiazépin-3-yl]-8,8-diméthylnon-6-énamide

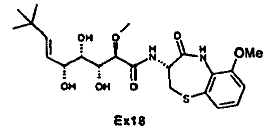

### Etape 1: Préparation de 1-fluoro-3-méthoxy-2-nitrobenzène (52)

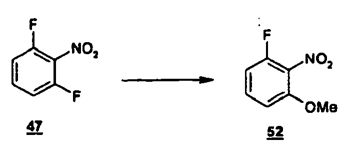

Dans un tricol de 100 mL contenant 10m de THF et 0,255 mL de MeOH, on ajoute 252 mg de d'hydrure de sodium en suspension à 60% dans l'huile (6,29 mmol). On agite à TA pendant 1h, et on introduit une solution de 2,6-difluoro-nitrobenzène **47** (1,0 g, 6,29 mmol) dans 10 mL de THF. On laisse le milieu réactionnel à TA pendant 16h. On ajoute 10 ml d'AcOEt, et lave 2 fois avec 20 mL d'eau distillée. La phase organique est séchée sur MgSO₄, filtrée et enfin évaporée à sec. On obtient 0,93 g de produit **52** (solide crème) RMN ¹H (400 MHz, DMSO-d₆), δ(ppm): 3,95 (s, 3H) ; 7,15 (t large, J = 8,0 Hz, 1H) ; 7,21 (d large, J = 8,0 Hz, 1H) ; 7,65 (dt, J = 6,0 et 8,0 Hz, 1H).

### Etape 2: Préparation de l'acide N-[(2,2-diméthylpropanoyl)oxy]-S-(3-méthoxy-2-nitrophényl)-L-cystéine (53)

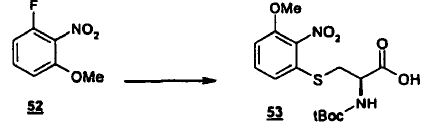

Dans un tricol de 100 mL contenant 1,422 g de L-Boc-Cys-OH (6,428 mmol), 13 mL d'eau et 1,561 g de NaHCO₃ (18,58 mmol), on introduit une solution de **52** (1,1 g, 6,428 mmol) dans 17 mL d'éthanol. On chauffe le milieu à 100°C pendant 6h. On évapore l'éthanol, lave ensuite la phase aqueuse avec 20 mL d'éther, une fois la phase éthérée décantée, on amène la phase aqueuse jusqu'à pH2-3 avec du HCl (1 N), et extrait 2 fois avec 25 mL d'AcOEt. Les phases organiques sont réunies, séchées sur MgSO₄, filtrées et enfin évaporées à sec. Les résidus sont chromatographiés sur une cartouche de silice (25 g, éluant CH₂Cl₂/MeOH *en gradient : MeOH 1 à 10%*), on obtient. 2,43 g de produit **53** (solide crème).
RMN ¹H (400 MHz, DMSO-d₆), δ(ppm): 1,39 (s, 9H) ; 3,17 (dd, J = 10,0 et 14,0 Hz, 1H) ; 3,40 (dd, J = 4,0 et 14,0 Hz, 1H) ; 3,89 (s, 3H) ; 4,03 (m, 1H) ; 7,20 (d, J = 8,0 Hz, 1H) ; 7,23 (m, 2H) ; 7,54 (t, J = 8,0 Hz, 1H) ; 12,8 (m étalé, 1H).

### Etape 3: Préparation de S-(2-amino-3-méthoxyphényl)-N-[(2,2-diméthylpropanoyi)oxy]-L-cystéine (54)

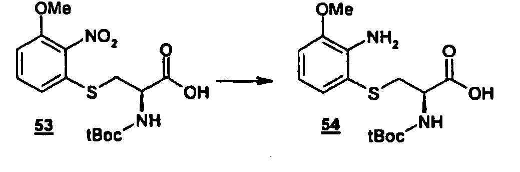

Dans un autoclave contenant 2,43 g de **53** (6,53 mmol), 285 mg de Pd/C (10%) et 174 mL de méthanol, on hydrogène sous 5 bars pendant 10 h à 20°C. Après filtration du catalyseur sur célite, on évapore le solvant et obtient 1,8 g, de produit attendu **54** (huile marron) qui est utilisé directement pour l'étape suivante.

### Etape 4 : Préparation du (3R)-3-{[(2,2-diméthylpropanoyl)oxy]amino}-6-méthoxy-2,3-dihvdro-1,5-benzothiazépin-4(5H)-one (55)

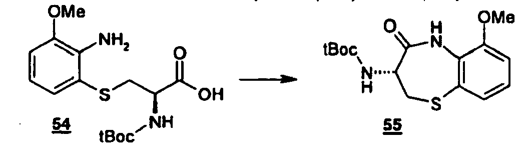

Dans un tricol de 100 mL contenant 1,80 g de **54** (5,26 mmol), 25 mL de DMF, on introduit, à 0°C, 0,948 ml de Cyanophosphonate de diéthyle (1,019 g, 6,245 mmol), et 10 minute après, 0,708 ml de TEA (5,04 mmol). On agite le milieu réactionnel à 0°C pendant 2 h. On ajoute 50 ml d'AcoEt, puis lave avec 2 fois 50 mL d'eau distillée. La phase organique est séchée sur MgSO₄, filtrée puis évaporée à sec. Le brut est chromatographié sur une cartouche de silice (150 g) avecun éluant Heptane/AcOEt (*en gradient AcOEt : 10 à 50%*). On obtient 450 mg de produit attendu **55** (solide blanc).
RMN ¹H (400 MHz, OMSO-d₆), δ(ppm): 1,32 (s, 9H) ; 3,05 (t, J = 12,0 Hz, 1H) ; 3,48 (dd, J = 7,0 et 12,0 Hz, 1H) ; 3,82 (s, 3H) ; 4,04 (m, 1H) ; de 7,10 à 7,12 (m, 4H) ; 9,40 (s, 1H).

### Etape 5: Préparation du (3R)-3-amino-6-méthoxy-2,3-dihydro-1,5-benzothiazépin-4(5H)-one chlorhydrate (56)

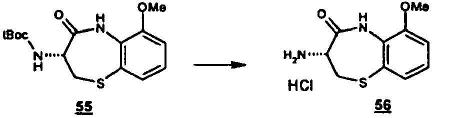

Dans un ballon de 100 mL contenant 450 mg de **55** (1,387 mmol), on ajoute 10,4 mL d'une solution d'acide chlorhydrique dans le dioxane (4M). On agite durant 3 h à TA sous argon. Un précipité blanc se forme, qui est essoré, lavé avec 3 mL de dioxane, puis 5 mL d'éther isopropylique. On obtient ainsi 259 mg d'amine **56** (solide crème) sous forme de chlorhydrate.
RMN ¹H (300 MHz, DMSO-d₆), δ(ppm): 3,19 (t, J = 12,0 Hz, 1H) ; 3,71 (m, 1H) ; 3,82 (s, 3H) ; 3,83 (m, 1H) ; de 7,17 à 7,30 (m, 3H) ; 8,39 (s, 3H) ; 10,05 (s, 1H).

### Etape 6: Préparation du (2R,3R,4S,5R,6E)-3,4,5-trihydroxy-2-méthoxy-N-[(3R)-6.méthoxy-4-oxo-2,3,4,5-tétrahydro-1,5-benzothiazépin-3-yl]-8,8-diméthylnon-6-énamide (Ex18)

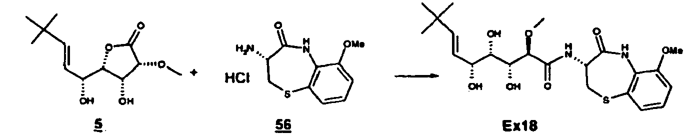

On introduit successivement dans un tube Wheatton, sous agitation et sous atmosphère d'argon, 100 mg de **5** (409 µmol), 107 mg de **56** (409 µmol), 170 mg de 2-éthythexanoate de sodium (1,02 mmol) dans 2 mL de THF. On maintient l'agitation à TA pendant 24 h. On concentre le milieu réactionel à sec. Les résidus sont chromatographiés sur une cartouche de silice (12 g, éluant AcOEt). On recueille 175 mg de produit attendu **Ex18** (meringue blanche).
ES : m/z = 467 (M-H⁺)
RMN ¹H (400 MHz, DMSO-d₆), δ(ppm): 0,96 (s, 9H) ; 3,09 (t, J = 12,0 Hz, 1H) ; 3,20 (s, 3H) ; 3,30 (m masqué, 1H) ; de 3,45 à 3,55 (m, 2H) ; 3,68 (d, J = 8,0 Hz, 1H) ; 3,82 (s, 3H) ; 3,92 (m, 1H) ; de 4,30 à 4,45 (m, 3H) ; 4,55 (s large, 1H) ; 5,29 (dd, J = 7,0 et 16,0 Hz, 1H) ; 5,62 (d, J = 16,0 Hz, 1H) ; de 7,12 à 7,26 (m, 3H) ; 8,10 (d, J = 8,0 Hz, 1H) ; 9,58 (s, 1H).

### Ex19: (2R,3R,4S,5R,6E)-3,4,5-trihydroxy-2-méthoxy-8,8-diméthyl-N-[(3R)-4-oxo-6-phénoxy-2,3,4,5-tétrahydro-1,5-benzothiazépin-3-yl]non-6-énamide

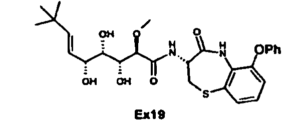

### Etape 1: Préparation de 1-fluoro-2-nitro-3-phénoxybenzène (57)

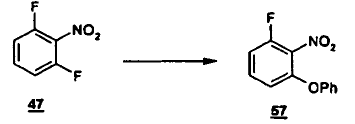

Dans un tricol de 50 mL contenant 10ml de DMF et 0,592 g de phénol, on ajoute 252 mg de d'hydrure de sodium en suspension à 60% dans l'huile (6,29 mmol). On agite à TA pendant 1 h, et on introduit une solution de 2,6-difluoro-nitrobenzène **47** (1,0 g, 6,29 mmol) dans 10 mL de DMF. On laisse le milieu réactionnel à TA pendant 24h. On ajoute 50 ml d'AcOEt, et lave 2 fois avec 20 mL d'eau distillée. La phase organique est séchée sur MgSO₄, filtrée et enfin évaporée à sec. Le brut est chromatographié sur une cartouche de silice (120 g) avecun éluant Heptane/AcOEt *(en gradient AcOEt : 0 à 50%).* On obtient 1,07 g de produit attendu **57** (huile jaune).
RMN ¹H (400 MHz, DMSO-d₆), δ(ppm): 6,92 (d large, J = 8,0 Hz, 1H) ; 7,17 (d large, J = 7,5 Hz, 2H) ; 7,29 (t, J = 7,5 Hz, 1H) ; 7,36 (t large, J = 8,0 Hz, 1H) ; 7,48 (t, J = 7,5 Hz, 2H) ; 7,65 (dt, J = 6,0 et 8,0 Hz, 1H).

### Etape 2: Préparation N-[(2,2-diméthylpropanoyl)oxy]-S-(2-nitro-3-phénoxyphényl)-L-cvstéine (58)

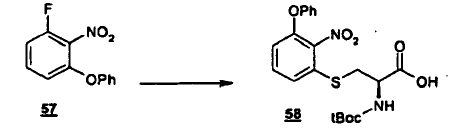

Dans un tricol de 25 mL contenant 0,693 g de L-Boc-Cys-OH (3,13 mmol), 6,3 mL d'eau et 0,76 g de NaHCO₃ (9,05 mmol), on introduit une solution de **57** (0,73 g, 3,13 mmol) dans 8 mL d'éthanol. On chauffe le milieu à 75°C pendant 6h. On évapore l'éthanol, ajoute 10 ml d'eau distillée, lave ensuite 2 fois la phase aqueuse avec 10 mL d'éther, une fois la phase éthérée décantée, on amène la phase aqueuse jusqu'à pH2-3 avec du HCl (1 N), et extrait 2 fois avec 10 mL d'AcOEt. Les phases organiques sont réunies, séchées sur MgSO₄, filtrées et enfin évaporées à sec. Les résidus sont chromatographiés sur une cartouche de silice (50 g, éluant CH₂Cl₂/MeOH en *gradient : MeOH 1 à 10%*), on obtient 760 mg de produit **58** (huile jaune). RMN ¹H (400 MHz, DMSO-d₆), δ(ppm): 1,38 (s, 9H) ; 3,22 (dd, J = 9,0 et 14,0 Hz, 1H) ; 3,50 (dd, J = 5,0 et 14,0 Hz, 1H) ; 4,08 (m, 1H) ; 6,96 (d, J = 8,0 Hz, 1H) ; 7,10 (d, J = 8,0 Hz, 2H) ; de 7,18 à 7,28 (m, 2H) ; de 7,39 à 7,49 (m, 3H) ; 7,56 (t, J = 8,0 Hz, 1H) ; 12,85 (m étalé, 1H).

### Etape 3: Préparation de S-(2-amino-3-phénoxyphényl)-N-[(2,2-diméthylpropanoyl)oxy]-L-cystéine (59)

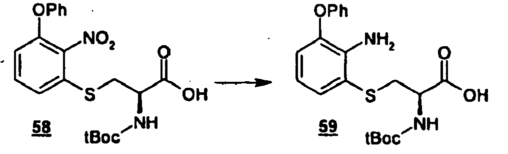

Dans un autoclave contenant 0,76 g de **58** (1,75 mmol), 76 mg de Pd/C (10%) et 34,5 mL de méthanol, on hydrogène sous 5 bars pendant 10 h à 20°C. Après filtration du catalyseur sur célite, on évapore le solvant et obtient 0,63 g de produit attendu **59** (huile marron) qui est utilisé directement pour l'étape suivante.
RMN ¹H (400 MHz, DMSO-d₆), δ(ppm): 1,39 (s, 9H) ; 2,96 (m, 1H) ; 3,11 (m, 1H) ; 3,98 (m, 1H) ; 5,10 (m étalé, 2H) ; 6,58 (t, J = 8,0 Hz, 1H) ; 6,80 (d, J = 8,0 Hz, 1H) ; 6,92 (d, J = 7,5 Hz, 2H) ; de 7,00 à 7,20 (m, 3H) ; 7,34 (t, J = 7,5 Hz, 2H) ; 12,5 (m très étalé, 1H).

### Etape 4 : Préparation du (3R)-3-{[(2,2-diméthylpropanoyl)oxy]amino}-6-phénoxy-2,3-dihydro-1,5-benzothiazépin-4(5H)-one (60)

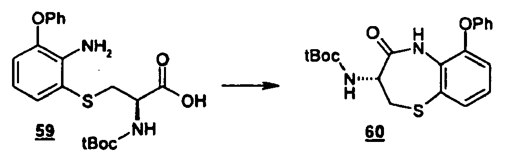

Dans un tricol de 25 mL contenant 0,63g de **59** (1,56 mmol), 7,5 mL de DMF, on introduit, à 0°C, 0,281 ml de Cyanophosphonate de diéthyle (0,302 g, 1,85 mmol), et 10 minute après, 0,21 ml de TEA (1,49 mmol). On agite le milieu réactionnel à 0°C pendant 2 h. On ajoute 15 ml d'AcoEt, puis lave avec 2 fois 15 mL d'eau distillée. La phase organique est séchée sur MgSO₄, filtrée puis évaporée à sec. Le brut est chromatographié sur une cartouche de silice (120 g) avec un éluant Heptane/AcOEt (*en gradient AcOEt : 0 à 30%*). On obtient 188 mg de produit attendu **60** (solide blanc).
RMN ¹H (400 MHz, DMSO-d₆), δ(ppm): 1,35 (s, 9H) ; 3,09 (t, J = 12,0 Hz, 1H) ; 3,52 (dd, J = 7,0 et 12,0 Hz, 1H) ; 4,21 (m, 1H) ; de 6,97 à 7,05 (m, 3H) ; de 7,08 à 7,18 (m, 2H) ; 7,21 (t, J = 7,5 Hz, 1H); 7,37 (t, J = 7,5 Hz, 2H) ; 7,42 (d, J = 7,5 Hz, 1H) ; 9,81 (s, 1H).

### Etape 5: Préparation du (3R)-3-amino-6-phénoxy-2,3-dihydro-1,5-benzothiazépin-4(5H)-one chlorhydrate (61)

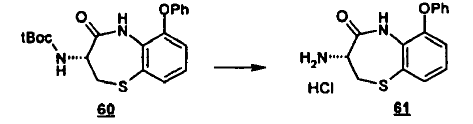

Dans un ballon de 100 mL contenant 188 mg de **60** (0,487 mmol), on ajoute 3,65 mL d'une solution d'acide chlorhydrique dans le dioxane (4M). On concentre à sec le milieu réactionnel, et reprend par 10 mL d'éther isopropylique. On obtient, après filtration, 100 mg d'amine **61** (solide blanc) sous forme de chlorhydrate.
RMN ¹H (400 MHz, DMSO-d₆), δ(ppm): 3,22 (t, J = 12,0 Hz, 1H) ; 3,79 (dd, J = 7,0 et 12,0 Hz, 1H) ; 4,08 (dd, J = 6,0 et 12,0 Hz, 1H) ; 6,97 (d large, J = 7,5 Hz, 1H) ; 7,04 (d, J = 7,5 Hz, 2H) ; 7,19 (t, J = 7,5 Hz, 1H) ; 7,23 (t, J = 8,0 Hz, 1H) ; 7,42 (m, 3H) ; 8,44 (s large, 3H) ; 10,45 (s, 1H).

### Etape 6: Préparation du (2R,3R,4S,5R,6E)-3,4,5-trihydroxy-2-méthoxy-8,8-diméthyl-N-[(3R)-4-oxo-6-phénoxy-2,3,4,5-tétrahydro-1,5-benzothiazépin-3-yl]non-6-énamide (Ex19)

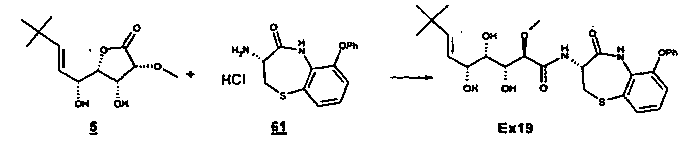

On introduit successivement dans un tube Wheatton, sous agitation et sous atmosphère d'argon, 75 mg de **5** (307 µ), 99,1 mg de **61** (307 µmol), 127 mg de 2-éthylhexanoate de sodium (0,77 mmol) dans 1,6 mL de THF. On maintient l'agitation à TA pendant 24 h. On concentre le milieu réactionnel à sec. Les résidus sont chromatographiés sur une cartouche de silice (5 g, éluant AcOEt). On recueille 56 mg de produit attendu **Ex19** (meringue blanc).
ES : m/z = 529 (M-H⁺)
RMN ¹H (400 MHz, DMSO-d₆), δ(ppm): 0,95 (s, 9H) ; 3,12 (t, J = 12,0 Hz, 1H) ; 3,19 (s, 3H) ; 3,30 (m masqué, 1H) ; 3,50 (m, 1H) ; 3,54 (dd, J = 6,5 et 12,0 Hz, 1H) ; 3,69 (d, J = 8,0 Hz, 1H) ; 3,94 (m, 1H) ; 4,32 (m, 2H) ; 4,52 (m, 1H) ; 4,58 (d, J = 4,5 Hz, 1H) ; 5,30 (dd, J = 6,5 et 16,0 Hz, 1H) ; 5,62 (d, J = 16,0 Hz, 1H) ; 7,00 (m, 3H) ; 7,12 (t, J = 7,5 Hz, 1H) ; 7,22 (t, J = 7,5 Hz, 1H) ; 7,38 (t, J = 7,5 Hz, 2H) ; 7,42 (d large, J = 7,5 Hz, 1H) ; 8,19 (d, J = 8,0 Hz, 1H) ; 9,97 (s, 1H).

### Ex20: (2R,3R,4S,5R,6E)-3,4,5-trihydroxy-2-méthoxy-8,8-diméthyl-N-[(3R)-5-méthyl-4-oxo-2,3,4,5-tétrahydro-1,5-benzothiazépin-3-yl]non-6-énamide

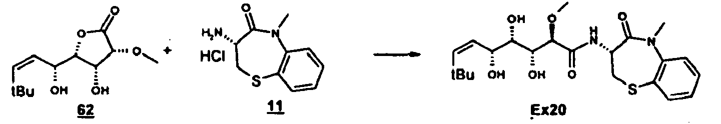

On introduit successivement dans un ballon de 5 mL, sous agitation et sous atmosphère d'argon, 50 mg de **62** (205 µmol) (préparé selon le mode opératoire décrite à l'étape 3 de **l'Ex1,** à partir de (4S,4aS,7R,7aR)-4-((Z)-3,3-diméthyl-but-1-enyl)-7-méthoxy-2,2-diméthyl-tetrahydro-furo[3,2-d]-1,3-dioxin-6-one, obtenu minoritairement lors de la préparation de **4**, selon les modes opératoires décrites dans Org. Process Res. Dev. 2003, 7(6), 856-865), 61 mg de **11** (246 µmol), 51 mg de 2-éthylhexanoate de sodium (0,31 mmol) dans 1 mL de THF. On maintient l'agitation à TA pendant 24 h. On ajoute 10 mL de CH₂Cl₂ au milieu réactionnel. On lave avec 5 mL d'une solution de HCI (1 N). La phase organique est séchée sur sulfate de magnésium anhydre, filtrée puis évaporée à sec. Le brut (0,15 g) est chromatographié sur une cartouche de silice (5 g, éluant Heptane/AcOEt : *10*/*90*). On recueille 68 mg de produit attendu **Ex20.**
ES : m/z = 451 (M-H)-.
RMN ¹H (300 MHz, DMSO-d₆), δ(ppm): 0,95 (s, 9H) ; 3,12 (t, J = 12,0 Hz, 1H) ; 3,19 (s, 3H) ; 3,30 (m masqué, 1H) ; 3,50 (m, 1H) ; 3,54 (dd, J = 6,5 et 12,0 Hz, 1H) ; 3,69 (d, J = 8,0 Hz, 1H) ; 3,94 (m, 1H) ; 4,32 (m, 2H) ; 4,52 (m, 1H) ; 4,58 (d, J = 4,5 Hz, 1H) ; 5,30 (dd, J = 6,5 et 16,0 Hz, 1H) ; 5,62 (d, J = 16,0 Hz, 1H) ; 7,00 (m, 3H) ; 7,12 (t, J = 7,5 Hz, 1H) ; 7,22 (t, J = 7,5 Hz, 1H) ; 7,38 (t, J = 7,5 Hz, 2H) ; 7,42 (d large, J = 7,5 Hz, 1H) ; 8,19 (d, J = 8,0 Hz, 1H) ; 9,97 (s, 1H).

### Activité biologique des produits préparés:

Au jour du dépôt de la demande, il a été mesuré que le Caco2-TC7 du produit de l'exemple 5 est Papp single point = 23.10⁻⁷ cm.sec⁻¹. Ce composé présente une meilleure stabilité métabolique totale (11% de métabolisation chez la souris) que le produit de l'exemple 20 (99%) décrit dans la demande de brevet WO2006/056696.

L'activité antiproliférative des produits des exemples du tableau 1 a été déterminée par mesure de l'inhibition de la prolifération cellulaire de cellules HCT116. Les cellules sont ensemencées dans un milieu de culture cellulaire à une concentration de 10 000 cellules par puits, dans 0.17 mL de milieu, et 20 µL de produit à tester, à différentes concentrations, et 10 µL de Thymidine [méthyl-14C] (100 µCi/ml - activité spécifique 47.90 mCi/mmol; NEN Technologies référence NEC568 batch 3550-001) sont ajoutés, puis les cellules sont incubées à 37°C et 5% de CO₂.

Milieu utilisé pour la culture de cellules HCT116: milieu DMEM 2 mM L-glutamine, 200 Ul/ml pénicilline, 200 µg/ml streptomycine and 10% (V/V) Sérum de veau foetal (Life Technologies).

Après 96 heures, l'incorporation de ¹⁴C-thymidine est comptée dans un compteur à scintillation liquide 1450 Microbeta Wallac Trilux. Les résultats R sont exprimés en cpm (coups par minute) et convertis en pourcentage d'inhibition de croissance Gl% en faisant premièrement la soustraction de la moyenne du nombre de cpm des puits sans cellules B et en divisant ensuite par le nombre de cpm des puits des cellules non traitées C comprenant 20µL de milieu de dilution du produit contentant 1% d'éthanol. (Gl % = (R - B) x 100 / C %).

Les valeurs d'IC50 sont calculées à l'aide de l'équation 205 du logiciel XLFit (IDBS company, UK) par analyse de régression non linéaire utilisant l'algorithme Marquardt (Donald W. MARQUARDT, J.Soc.industry.appl, vol 11, No. 2, June, 1963).

Les produits du tableau 1 présentent une IC50 sur les cellules HCT116 généralement inférieure à 30µM et de préférence inférieure à 100nM. Par exemple, le produit de l'exemple 3 a une IC50 de 10 nM et le produit de l'exemple 5 a une IC50 de 14 nM. Le produit de l'exemple 1 a une IC50 de 9 nM, le produit de l'exemple 2 a une IC50 de 20 nM, le produit de l'exemple 4 a une IC50 de 19 nM et le produit de l'exemple 9 a une IC50 de 7 nM.

**- Tableau 1 -**

| **Exemple** | **Structure** |
|---|---|
| **Ex1** | 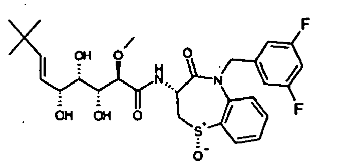 |
| **Ex2** | 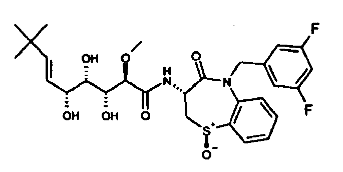 |
| **Ex3** | 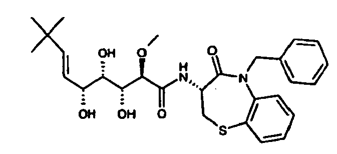 |
| **Ex4** | 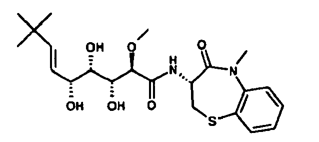 |
| **Ex5** | 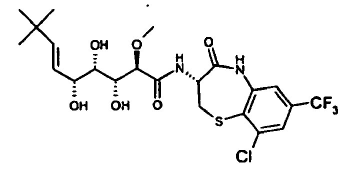 |
| **Ex6** | 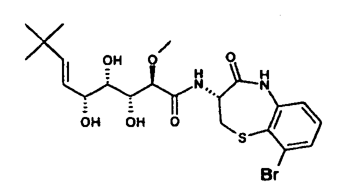 |
| **Ex7** | 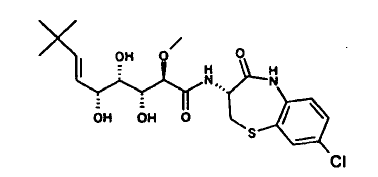 |
| **Ex8** | 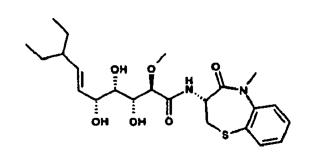 |
| **Ex9** | 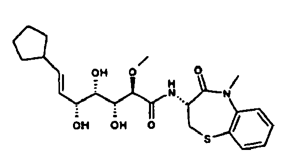 |
| **Ex10** | 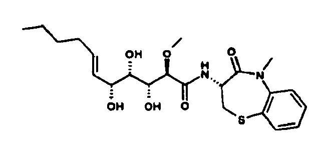 |
| **Ex11** | 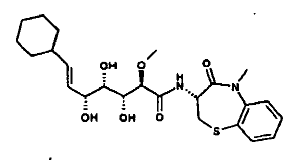 |
| **Ex12** | 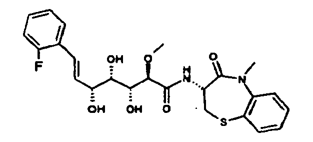 |
| **Ex13** | 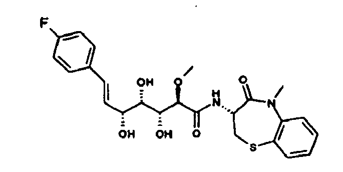 |
| **Ex14** | 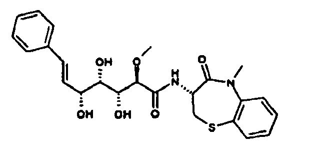 |
| **Ex15** | 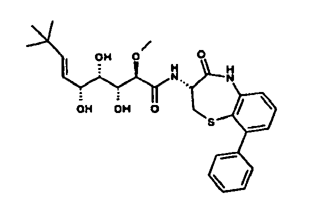 |
| **Ex16** | 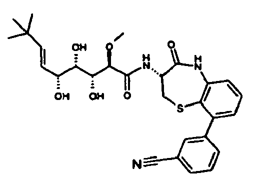 |
| **Ex17** | 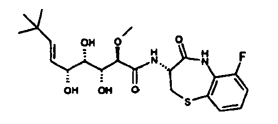 |
| **Ex18** | 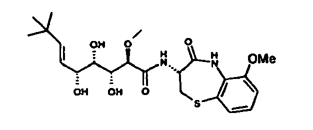 |
| **Ex19** | 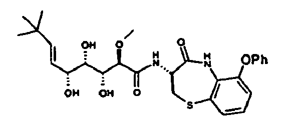 |
| **Ex20** | 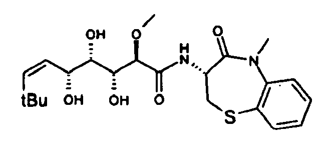 |

## Revendications

1. Produit de formule générale (I) suivante : 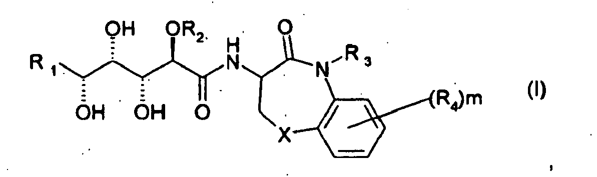 dans laquelle :
g) R₁ est le groupe -C(R₆)=C(R₇)(R₈) dans lequel R₆, R₇, et R₈ sont indépendamment sélectionnés parmi H, (C1-C6)alkyle, cycloalkyle, hétérocyclyle, aryle, hétéroaryle;
h) R₂ est méthyle;
i) R₃ est sélectionné dans le groupe constitué par H, méthyle, un groupe phénylméthyle ou un groupe (3,5-difluoro-phényl)-méthyle;
j) R₄ est indépendamment sélectionné dans le groupe constitué par H, F, CI, Br, phényle, cyanophényle, trifluorométhyle, méthoxy, phénoxy ou bien 2 substituants R₄, liés à 2 carbones adjacents du phényle, forment ensemble un cycle pyrazine.
k) m a pour valeur 0, 1, 2, 3, ou 4 ;
l) X est choisi parmi S ou SO;
à la condition que lorsque R₁ est (E) -CH=CH-C(CH₃)₃, R₂ est méthyle, X est S et m est 0, alors R₃ n'est pas un atome d'hydrogène, un groupe (3,5-difluoro-phényl)-méthyle ou un groupe -CH₂-CH=CH₂.

2. Produit selon la revendication 1, **caractérisé en ce que** R₁ est choisi parmi (E) -CH=CH-CH(CH₃)(C₂H₅), (E) -CH=CH-CH(CH₃)₂, et (E) - CH=CH-C(CH₃)₃.

3. Produit selon la revendication 1, **caractérisé en ce que** R₁ est choisi parmi (E) -C(CH₃)=CH-CH(CH₃)(C₂H₅), (E) -C(CH₃)=CH-CH(CH₃)₂, et (E) -C(CH₃)=CH-C(CH₃)₃.

4. Produit selon l'une quelconque des revendications 1 à 3, **caractérisé en ce que** X est S.

5. Produit selon l'une quelconque des revendications 1 à 3, **caractérisé en ce que** X est SO.

6. Produit selon l'une quelconque des revendications 1 à 5, **caractérisé en ce que** R₃ est H.

7. Produit selon l'une quelconque des revendications 1 à 6, **caractérisé en ce que** m est 0.

8. Produit selon l'une quelconque des revendications 1 à 7, **caractérisé en ce qu'**il est choisi parmi:
N-[(1*R*,3*R*)-5-(3,5-difluoro-benzyl)-1,4-dioxo-2,3,4,5-tetrahyd ro-1,5-benzothiazépin-3-yl]-((6E)-(2R,3R,4S,5R)-3,4,5-trihydroxy-2-méthoxy-8,8-diméthyl-non-6-énamide ;
N-[(1S,3R)-5-(3,5-difluoro-benzyl)-1,4-dioxo-2,3,4,5-tetrahydro-1,5-benzothiazépin-3-yl]-((6E)-(2R,3R,4S,5R)-3,4,5-trihydroxy-2-méthoxy-8,8-diméthyl-non-6-énamide ;
N-[(3R)-5-benzyl-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide ;
N-[(3R)-5-méthyl-4-oxo-2,3,4,5-tetrahydro-1,5-benzothiazépin-3-yl]-(6E)-(2R,3R,4S,5R)-3,4,5-trihydroxy-2-méthoxy-8,8-diméthyl-non-6-énamide ;
N-((3R)-9-chloro-4-oxo-7-trifluorométhyl-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide;
N-((3R)-9-bromo-4-oxo-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide ;
N-((3R)-8-chloro-4-oxo-2,3,4,5-tetrahydro-1,5-benzothiazépin-3-yl)-(6E)-(2R,3R,4S,5R)-3,4,5-trihydroxy-2-méthoxy-8,8-diméthyl-non-6-énamide ;
(6E)-(2R,3R,4S,5R)-8-éthyl-3,4,5-trihydroxy-2-méthoxy-N-((3R)-5-méthyl-4-oxo-2,3,4,5-tetrahydro-1,5-benzothiazépin-3-yl)-déc-6-énamide.

9. Produit selon l'une quelconque des revendications 1 à 7, **caractérisé en ce qu'**il est choisi parmi:
(2*R*,3*R*,4*S*,5*R*,6*E*)-7-cyclopentyl-3,4,5-trihydroxy-2-méthoxy-*N*-[(3*R*)-5-méthyl-4-oxo-2,3,4,5-tétrahydro-1,5-benzothiazépin-3-yl]hept-6-énamide ;
(2*R*,3*R*,4*S*,5*R*,6*E*)-3,4,5-trihydroxy-2-méthoxy-*N*-[(3*R*)-5-méthyl-4-oxo-2,3,4,5-tétrahydro-1,5-benzothiazépin-3-yl]undéc-6-énamide;
(2*R*,3*R*,4*S*,5*R*,6*E*)-7-cyclohexyl-3,4,5-trihydroxy-2-méthoxy-*N*-[(3*R*)-5-méthyl-4-oxo-2,3,4,5-tétrahydro-1,5-benzothiazépin-3-yl]hept-6-énamide ;
(2*R*,3*R*,4*S*,5*R*,6*E*)-7-(2-fluorophényl)-3,4,5-trihydroxy-2-méthoxy-*N-*[(3*R*)-5-méthyl-4-oxo-2,3,4,5-tétrahydro-1,5-benzothiazépin-3-yl]hept-6-énamide ;
(2*R*,3*R*,4*S*,5*R*,6*E*)-7-(4-fluorophényl)-3,4,5-trihydroxy-2-méthoxy-*N-*[(3*R*)-5-méthyl-4-oxo-2,3,4,5-tétrahydro-1,5-benzothiazépin-3-yl]hept-6-énamide ;
(2*R*,3*R*,4*S*,5*R*,6*E*)-3,4,5-trihydroxy-2-méthoxy-*N*-[(3*R*)-5-méthyl-4-oxo-2,3,4,5-tétrahydro-1,5-benzothiazépin-3-yl]-7-phénylhept-6-énamide ;
(2*R*,3*R*,4*S*,5*R*,6*E*)-3,4,5-trihydroxy-2-méthoxy-8,8-diméthyl-*N*-[(3*R*)-4-oxo-9-phényl-2,3,4,5-tétrahydro-1,5-benzothiazépin-3-yl]non-6-énamide ;
(2*R*,3*R*,4*S*,5*R*,6*E*)-*N*-[(3*R*)-9-(3-cyanophényl)-4-oxo-2,3,4,5-tétrahydro-1,5-benzothiazépin-3-yl]-3,4,5-trihydroxy-2-méthoxy-8,8-diméthylnon-6-énamide ;
(2*R*,3*R*,4*S*,5*R*,6*E*)-*N*-[(3*R*)-6-fluoro-4-oxo-2,3,4,5-tétrahydro-1,5-benzothiazépin-3-yl]-3,4,5-trihydroxy-2-méthoxy-8,8-diméthylnon-6-énamide ;
(2*R*,3*R*,4*S*,5*R*,6*E*)-3,4,5-trihydroxy-2-méthoxy-*N*-[(3*R*)-6-méthoxy-4-oxo-2,3,4,5-tétrahydro-1,5-benzothiazépin-3-yl]-8,8-diméthylnon-6-énamide ;
(2*R*,3*R*,4*S*,5*R*,6*E*)-3,4,5-trihydroxy-2-méthoxy-8,8-diméthyl-*N*-[(3*R*)-4-oxo-6-phénoxy-2,3,4,5-tétrahydro-1,5-benzothiazépin-3-yl]non-6-énamide ;
(2*R*,3*R*,4*S*,5*R*,6*Z*)-3,4,5-trihydroxy-2-méthoxy-8,8-diméthyl-*N*-[(3*R*)-5-méthyl-4-oxo-2,3,4,5-tétrahydro-1,5-benzothiazépin-3-yl]non-6-énamide.

10. Produit selon l'une quelconque des revendications précédentes, **caractérisé en ce qu'**il est sous forme :
a) non chirale, ou
b) racémique, ou
c) enrichie en un stéréo-isomère, ou
d) enrichie en un énantiomère ;
et **en ce qu'**il est éventuellement salifié.

11. Médicament, **caractérisé en ce qu'**il comprend un produit de formule (I) selon l'une quelconque des revendications 1 à 7, ou un sel d'addition de ce produit à un acide pharmaceutiquement acceptable, ou encore un hydrate ou un solvat du produit de formule (I).

12. Composition pharmaceutique, **caractérisée en ce qu'**elle comprend un produit de formule (I) selon l'une quelconque des revendications 1 à 7, ou un sel pharmaceutiquement acceptable, un hydrate ou un solvat de ce produit, ainsi qu'au moins un excipient pharmaceutiquement acceptable.

13. Utilisation d'un produit selon l'une quelconque des revendications 1 à 7, pour la fabrication d'un médicament utile pour prévenir ou traiter un état pathologique.

14. Utilisation selon la revendication 13, **caractérisée en ce que** l'état pathologique est le cancer.

15. Procédé de préparation d'un produit de formule générale (I) selon l'une des revendications 1 à 7, **caractérisé en ce qu'**un produit de formule générale (II) : 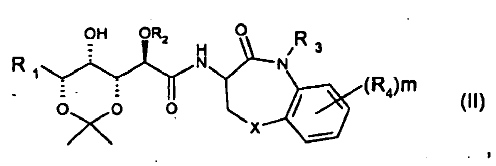 dans lequel R₁, R₂, R₃, R₄, X et m sont tels que définis précédemment, subit une hydrolyse pour obtenir un produit de formule générale (I).

16. Procédé de préparation, selon la revendication 15, d'un produit de formule générale (I) selon l'une des revendications 1 à 7, **caractérisé en que** l'on fait réagir un produit de formule générale (III) : 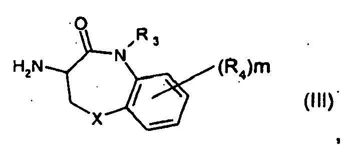 dans lequel R₃, R₄ , X et m sont tels que définis précédemment, avec un produit de formule générale (IV) : 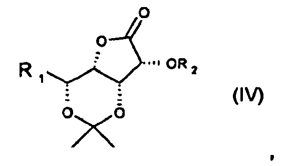 dans lequel R₁, R₂ sont tels que définis précédemment, pour obtenir un produit de formule générale (II).

17. Procédé de préparation d'un produit de formule générale (I) selon l'une des revendications 1 à 7, **caractérisé en ce que** l'on fait réagir un produit de formule générale (III) : 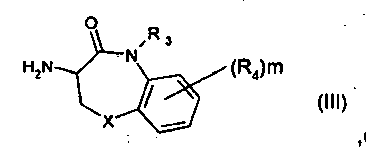 dans lequel R₃, R₄ , X et m sont tels que définis précédemment, et un produit de formule générale (V) : 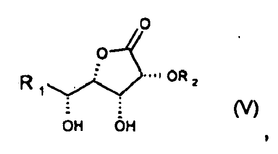 dans lequel R₁, R₂ sont tels que définis précédemment, pour obtenir un produit de formule générale (I).

18. Procédé de préparation, selon la revendication 17, d'un produit de formule générale (I) selon l'une des revendications 1 à 7, **caractérisé en ce qu'**un produit de formule générale (IV) : 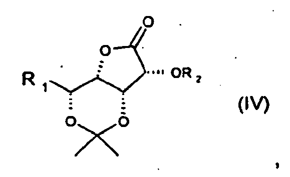 dans lequel R₁, R₂ sont tels que définis précédemment, subit une hydrolyse pour obtenir un produit formule générale (V) : 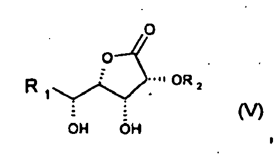 dans lequel R₁, R₂ sont tels que définis précédemment.

19. Procédé de préparation, selon la revendication 17, d'un produit de formule générale (I) selon l'une des revendications 1 à 7, **caractérisé en ce qu'**un produit de formule générale (VII) : 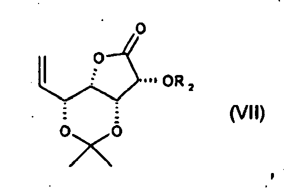 dans lequel R₂ est tel que défini précédemment, subit une hydrolyse afin d'obtenir un produit de formule générale (VI) : 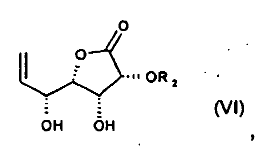 dans lequel R₂ est tel que défini précédemment, qui subit une métathèse afin d'obtenir un produit de formule générale (V) : 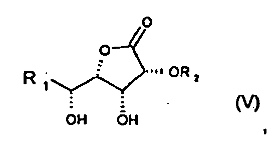 pour lesquels R₁ représente -CH=CH-R'₁ et R'₁ représente un groupe (C1-C6)alkyle, cycloalkyle, hétérocyclyle, aryle ou hétéroaryle.

20. Procédé de préparation, selon la revendication 19, d'un produit de formule générale (I) selon l'une des revendications 1 à 7, **caractérisé en ce qu'**un produit de formule (VII) : 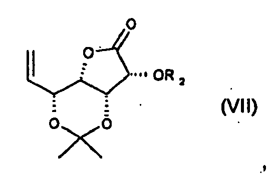 dans lequel R₂ est tel que défini précédemment, est obtenu par double déshydratation d'un produit de formule générale (VIII) : 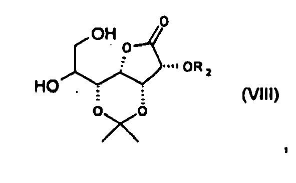 dans lequel R₂ est tel que défini précédemment.

21. Produits de formule générale (II) 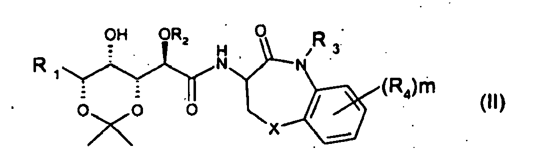 dans lequel R₁, R₂, R₃, R₄, X et m sont tels que définis précédemment,à l'exception de ceux pour lesquels R₁ est (E) -CH=CH-C(CH₃)₃, R₂ est méthyle, X est S, m est 0, et R₃ est un atome d'hydrogène, un groupe (3,5-difluoro-phényl)-méthyle ou un groupe -CH₂-CH=CH₂,

22. Produits de formule générale (III) 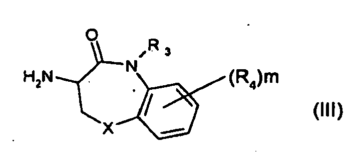 **caractérisés en ce que** X est S, R₃ est H, méthyle, phénylméthyle ou (3,5-difluorophényl)-méthyle et R₄ est F, CI, Br, phényle, cyanophényle, trifluorométhyle, méthoxy ou phénoxy, ou bien m a pour valeur 0, à l'exception de ceux pour lesquels X est S, m est 0, et R₃ est un atome d'hydrogène ou un groupe (3,5-difluoro-phényl)-méthyle et à l'exception de ceux pour lesquels X est S, R₃ est phénylméthyle et R4 est trifluorométhyle ou méthoxy.

23. Produits de formule générale (IV) et (V) 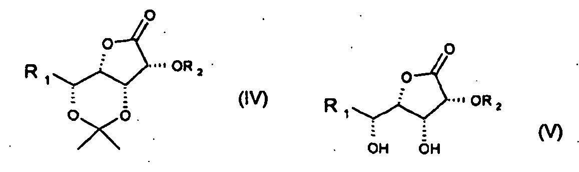 **caractérisés en ce que** R₂ est méthyle et R1 est -(E) -CH=CH-C₅H₉ ou -(E)-CH=CH-C₆H₅ où le phényle est substitué par un atome de fluor.

24. Produits de formule générale (VI) 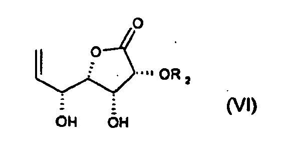 **caractérisés en ce que** R₂ est méthyle.

## Claims

1. Product of general formula (I) below: 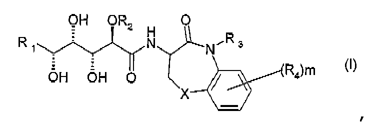 in which:
g) R₁ is the group -C(R₆)=C(R₇)(R₈) in which R₆, R₇, and R₈ are independently selected from H, (C1-C6)alkyl, cycloalkyl, heterocyclyl, aryl and heteroaryl;
h) R₂ is methyl;
i) R₃ is selected from the group consisting of H, methyl, a phenylmethyl group and a (3,5-difluorophenyl)methyl group;
j) R₄ is independently selected from the group consisting of H, F, Cl, Br, phenyl, cyanophenyl, trifluoromethyl, methoxy and phenoxy, or alternatively two substituents R₄, linked to two adjacent carbons of the phenyl, together form a pyrazine ring;
k) m has the value 0, 1, 2, 3 or 4;
1) X is chosen from S or SO;
on condition that when R₁ is (E) -CH=CH-C(CH₃)₃, R₂ is methyl, X is S and m is 0, then R₃ is not a hydrogen atom, a (3,5-difluorophenyl)methyl group or a -CH₂-CH=CH₂ group.

2. Product according to Claim 1, **characterized in that** R₁ is chosen from (E) -CH=CH-CH(CH₃)(C₂H₅), (E) -CH=CH-CH(CH₃)₂, and (E) -CH=CH-C(CH₃)₃.

3. Product according to Claim 1, **characterized in that** R₁ is chosen from (E) -C(CH₃)=CH-CH(CH₃)(C₂H₅), (E) -C(CH₃)=CH-CH(CH₃)₂, and (E) -C(CH₃)=CH-C(CH₃)₃.

4. Product according to any one of Claims 1 to 3, **characterized in that** X is S.

5. Product according to any one of Claims 1 to 3, **characterized in that** X is SO.

6. Product according to any one of Claims 1 to 5, **characterized in that** R₃ is H.

7. Product according to any one of Claims 1 to 6, **characterized in that** m is 0.

8. Product according to any one of Claims 1 to 7, **characterized in that** it is chosen from:
N-[(1R,3R)-5-(3,5-difluorobenzyl)-1,9-dioxo-2,3,4,5-tetrahydro-1,5-benzothiazepin-3-yl]-((6E)-(2R,3R,4S,5R)-3,4,5-trihydroxy-2-methoxy-8,8-dimethylnon-6-enamide;
N-[(1S,3R)-5-(3,5-difluorobenzyl)-1,4-dioxo-2,3,4,5-tetrahydro-1,5-benzothiazepin-3-yl]-((6E)-(2R,3R,4S,5R)-3,4,5-trihydroxy-2-methoxy-8,8-dimethylnon-6-enamide;
N-[(3R)-5-benzyl-4-oxo-2,3,4,5-tetrahydro-1,5-benzothiazepin-3-yl]-(6E)-(2R,3R,4S,5R)-3,4,5-trihydroxy-2-methoxy-8,8-dimethylnon-6-enamide;
N-[(3R)-5-methyl-4-oxo-2,3,4,5-tetrahydro-1,5-benzothiazepin-3-yl]-(6E)-(2R,3R,4S,5R)-3,4,5-trihydroxy-2-methoxy-8,8-dimethylnon-6-enamide;
N-((3R)-9-chloro-4-oxo-7-trifluoromethyl-2,3,4,5-tetrahydro-1,5-benzothiazepin-3-yl)-(6E)-(2R,3R,4S,5R)-3,4,5-trihydroxy-2-methoxy-8,8-dimethylnon-6-enamide;
N-((3R)-9-bromo-4-oxo-2,3,4,5-tetrahydro-1,5-benzothiazepin-3-yl)-(6E)-(2R,3R,4S,5R)-3,4,5-trihydroxy-2-methoxy-8,8-dimethylnon-6-enamide;
N-((3R)-8-chloro-4-oxo-2,3,4,5-tetrahydro-1,5-benzothiazepin-3-yl)-(6E)-(2R,3R,4S,5R)-3,4,5-trihydroxy-2-methoxy-8,8-dimethylnon-6-enamide;
(6E)-(2R,3R,4S,5R)-8-ethyl-3,4,5-trihydroxy-2-methoxy-N-((3R)-5-methyl-4-oxo-2,3,4,5-tetrahydro-1,5-benzothiazepin-3-yl)dec-6-enamide.

9. Product according to any one of Claims 1 to 7, **characterized in that** it is chosen from:
(2R,3R,4S,5R,6E)-7-cyclopentyl-3,4,5-trihydroxy-2-methoxy-N-[(3R)-5-methyl-4-oxo-2,3,4,5-tetrahydro-1,5-benzothiazepini-3-yl]hept-6-enamide;
(2R,3R,4S,5R,6E)-3,4,5-trihydroxy-2-methoxy-N-[(3R)-5-methyl-4-oxo-2,3,4,5-tetrahydro-1,5-benzothiazepin-3-yl]undec-6-enamide;
(2R,3R,4S,5R,6E)-7-cyclohexyl-3,4,5-trihydroxy-2-methoxy-N-[(3R)-5-methyl-4-oxo-2,3,4,5-tetrahydro-1,5-benzothiazepin-3-yl]hept-6-enamide;
(2R,3R,4S,5R,6E)-7-(2-fluorophenyl)-3,4,5-trihydroxy-2-methoxy-N-[(3R)-5-methyl-4-oxo-2,3,4,5-tetrahydro-1,5-benzothiazepin-3-yl]hept-6-enamide;
(2R,3R,4S,5R,6E)-7-(4-fluorophenyl)-3,4,5-trihydroxy-2-methoxy-N-[(3R)-5-methyl-4-oxo-2,3,4,5-tetrahydxo-1,5-benaothiazepin-3-yl]hept-6-enamide;
(2R,3R,4S,5R,6E)-3,4,5-trihydroxy-2-methoxy-N-[(3R)-5-methyl-4-oxo-2,3,4,5-tetrahydro-1,5-benzothiazepin-3-yl]-7-phenylhept-6-enamide;
(2R,3R,4S,5R,6E)-3,4,5-trihydroxy-2-methoxy-8,8-dimethyl-N-[(3R)-4-oxo-9-phenyl-2,3,4,5-tetrahydro-1,5-benzothiazepin-3-yl]non-6-enamide;
(2R,3R,4S,5R,6E)-N-[(3R)-9-(3-cyanophenyl)-4-oxo-2,3,4,5-tetrahydro-1,5-benzothiazepin-3-yl]-3,4,5-trihydroxy-2-methoxy-8,8-dimethylnon-6-enamide;
(2R,3R,4S,5R,6E)-N-[(3R)-6-fluoro-4-oxo-2,3,4,5-tetrahydro-1,5-benzothiazepin-3-yl]-3,4,5-trihydroxy-2-methoxy-8,8-dimethylnon-6-enamide;
(2R,3R,4S,5R,6E)-3,4,5-trihydroxy-2-methoxy-N-[(3R)-6-methoxy-4-oxo-2,3,4,5-tetrahydro-1,5-benzothiazepin-3-yl]-8,8-dimethylnon-6-enamide;
(2R,3R,45,5R,6E)-3,4,5-trihydroxy-2-methoxy-8,8-dimethyl-N-[(3R)-4-oxo-6-phenoxy-2,3,4,5-tetrahydro-1,5-benzothiazepin-3-yl]non-6-enamide;
(2R,3R,4S,5R,6Z)-3,4,5-trihydroxy-2-methoxy-8,8-dimethyl-N-[(3R)-5-methyl-4-oxo-2,3,4,5-tetrahydro-1,5-benzothiazepin-3-yl]non-6-enamide.

10. Product according to any one of the preceding claims, **characterized in that** it is:
a) in non-chiral form, or
b) in racemic form, or
c) enriched in one stereoisomer, or
d) enriched in one enantiomer;
and **in that** it is optionally salified.

11. Medicament, **characterized in that** it comprises a product of formula (I) according to any one of Claims 1 to 7, or an addition salt of this product with a pharmaceutically acceptable acid, or alternatively a hydrate or solvate of the product of formula (1).

12. Pharmaceutical composition, **characterized in that** it comprises a product of formula (I) according to any one of Claims 1 to 7, or a pharmaceutically acceptable salt, a hydrate or a solvate of this product, and also at least one pharmaceutically acceptable excipient.

13. Use of a product according to any one of Claims 1 to 7, for the manufacture of a medicament that is useful for preventing or treating a pathological condition.

14. Use according to Claim 13, **characterized in that** the pathological condition is cancer.

15. Process for preparing a product of general formula (I) according to any one of Claims 1 to 7, **characterized in that** a product of general formula (II) : 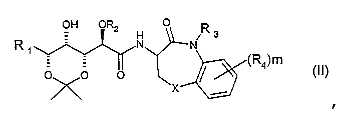 in which R₁, R₂, R₃, R₄, X and m are as defined above, undergoes a hydrolysis to give a product of general formula (I).

16. Process for preparing, according to Claim 15, a product of general formula (I) according to one of Claims 1 to 7, **characterized in that** a product of general formula (III): 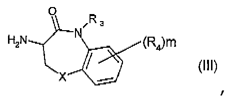 in which R₃, R₄ , X and m are as defined above, is reacted with a product of general formula (IV): 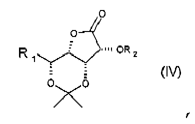 in which R₁ and R₂ are as defined above, to give a product of general formula (II).

17. Process for preparing a product of general formula (I) according to one of Claims 1 to 7, **characterized in that** a product of general formula (III): 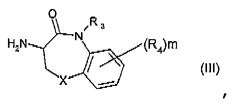 in which R₃, R₄, X and m are as defined above, and a product of general formula (V): 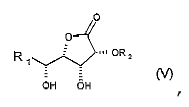 in which R₁ and R₂ are as defined above, are reacted together to give a product of general formula (I).

18. Process for preparing, according to Claim 17, a product of general formula (I) according to one of Claims 1 to 7, **characterized in that** a product of general formula (IV): 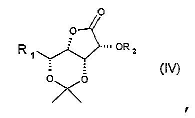 in which R₁ and R₂ are as defined above, undergoes a hydrolysis to give a product of general formula (V): 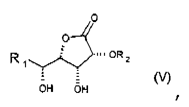 in which R₁ and R₂ are as defined above.

19. Process for preparing, according to Claim 17, a product of general formula (I) according to one of Claims 1 to 7, **characterized in that** a product of general formula (VII): 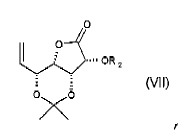 in which R₂ is as defined above, undergoes a hydrolysis in order to obtain a product of general formula (VI): 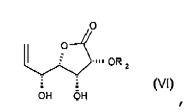 in which R₂ is as defined above, which undergoes a metathesis in order to obtain a product of general formula (V): 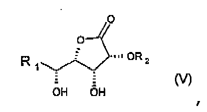 for which R₁ represents -CH=CH-R'₁ and R'₁ represents a (C1-C6)alkyl, cycloalkyl, heterocyclyl, aryl or heteroaryl group.

20. Process for preparing, according to Claim 19, a product of general formula (I) according to one of Claims 1 to 7, **characterized in that** a product of formula (VII): 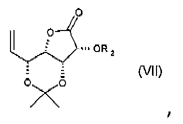 in which R₂ is as defined above, is obtained via double dehydration of a product of general formula (VIII): 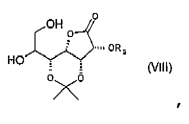 in which R₂ is as defined above.

21. Products of general formula (II) 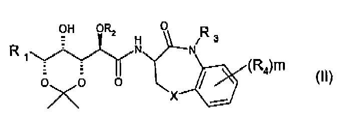 in which R₁, R₂, R₃, R₄, X and m are as defined previously, with the exception of those for which R₁ is (E)-CH=CH-C(CH₃)₃, R₂ is methyl, X is S, m is 0 and R₃ is a hydrogen atom, a (3,5-difluorophenyl)-methyl group or a group -CH₂-CH=CH₂.

22. Products of general formula (III) 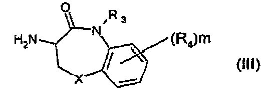 **characterized in that** X is S, R₃ is H, methyl, phenylmethyl or (3,5-difluorophenyl)methyl and R₄ is F, Cl, Br, phenyl, cyanophenyl, trifluoromethyl, methoxy or phenoxy, or alternatively m has the value 0, with the exception of those for which X is S, m is 0 and R₃ is a hydrogen atom or a (3,5-difluorophenyl)methyl group and with the exception of those for which X is S, R₃ is phenylmethyl and R₄ is trifluoromethyl or methoxy.

23. Products of general formulae (IV) and (V) 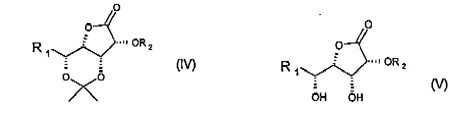 **characterized in that** R₂ is methyl and R₁ is (E) -CH=CH-C₅H₉ or (E)-CH=CH-C₆H₅ in which the phenyl is substituted with a fluorine atom.

24. Products of general formula (VI) 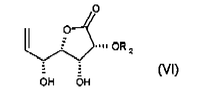 **characterized in that** R₂ is methyl.

## Patentansprüche

1. Produkt der folgenden allgemeinen Formel (I): 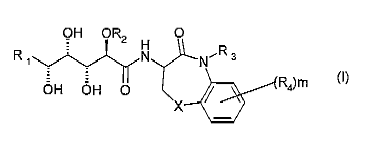 worin:
g) R₁ für -C(R₆)=C(R₇)(R₈), worin R₆, R₇ und R₈ unabhängig unter H, (C1-C6)-Alkyl, Cycloalkyl, Heterocyclyl, Aryl und Heteroaryl ausgewählt sind, steht;
h) R₂ für Methyl steht;
i) R₃ aus der Gruppe bestehend aus H, Methyl, einer Phenylmethylgruppe und einer (3,5-Difluorphenyl)methylgruppe ausgewählt ist;
j) R₄ unabhängig aus der Gruppe bestehend aus H, F, Cl, Br, Phenyl, Cyanophenyl, Trifluormethyl, Methoxy und Phenoxy ausgewählt ist oder auch zwei Substituenten R₄, die an zwei benachbarte Kohlenstoffe des Phenyls gebunden sind, zusammen einen Pyrazinring bilden;
k) m einen Wert von 0, 1, 2, 3 oder 4 hat;
1) X unter S oder SO ausgewählt ist;
mit der Maßgabe, daß R₃ nicht für ein Wasserstoffatom, eine (3,5-Difluorphenyl)methylgruppe oder eine -CH₂-CH=CH₂-Gruppe steht, wenn R₁ für (E)-CH=CH-C(CH₃)₃ steht, R₂ für Methyl steht, X für S steht und m für 0 steht.

2. Produkt nach Anspruch 1, **dadurch gekennzeichnet, daß** R₁ unter (E)-CH=CH-CH(CH₃)(C₂H₅), (E)-CH=CH-CH(CH₃)₂ und (E)-CH=CH-C(CH₃)₃ ausgewählt ist.

3. Produkt nach Anspruch 1, **dadurch gekennzeichnet, daß** R₁ unter (E)-C(CH₃)=CH-CH(CH₃) (C₂H₅), (E)-C(CH₃)=CH-CH(CH₃)₂ und (E)-C(CH₃)=CH-C(CH₃)₃ ausgewählt ist.

4. Produkt nach einem der Ansprüche 1 bis 3, **dadurch gekennzeichnet, daß** X für S steht.

5. Produkt nach einem der Ansprüche 1 bis 3, **dadurch gekennzeichnet, daß** X für SO steht.

6. Produkt nach einem der Ansprüche 1 bis 5, **dadurch gekennzeichnet, daß** R₃ für H steht.

7. Produkt nach einem der Ansprüche 1 bis 6, **dadurch gekennzeichnet, daß** m für 0 steht.

8. Produkt nach einem der Ansprüche 1 bis 7, **dadurch gekennzeichnet, daß** es unter:
N-[(1R,3R)-5-(3,5-Difluorbenzyl)-1,4-dioxo-2,3,4,5-tetrahydro-1,5-benzothiazepin-3-yl]-((6E)-(2R,3R,4S,5R)-3,4,5-trihydroxy-2-methoxy-8,8-dimethylnon-6-enamid;
N-[(1S,3R)-5-(3,5-Difluorbenzyl)-1,4-dioxo-2,3,4,5-tetrahydro-1,5-benzothiazepin-3-yl]-((6E)-(2R,3R,4S,5R)-3,4,5-trihydroxy-2-methoxy-8,8-dimethylnon-6-enamid;
N-[(3R)-5-Benzyl-4-oxo-2,3,4,5-tetrahydro-1,5-benzothiazepin-3-yl]-(6E)-(2R,3R,4S,5R)-3,4,5-trihydroxy-2-methoxy-8,8-dimethylnon-6-enamid;
N-[(3R)-5-Methyl-4-oxo-2,3,4,5-tetrahydro-1,5-benzothiazepin-3-yl]-(6E)-(2R,3R,4S,5R)-3,4,5-trihydroxy-2-methoxy-8,8-dimethylnon-6-enamid;
N-((3R)-9-Chlor-4-oxo-7-trifluormethyl-2,3,4,5-tetrahydro-1,5-benzothiazepin-3-yl)-(6E)-(2R,3R,4S,5R)-3,4,5-trihydroxy-2-methoxy-8,8-dimethylnon-6-enamid;
N-((3R)-9-Brom-4-oxo-2,3,4,5-tetrahydro-1,5-benzothiazapin-3-yl)-(6E)-(2R,3R,4S,5R)-3,4,5-trihydroxy-2-methoxy-8,8-dimethylnon-6-enamid:
N-((3R)-8-Chlor-4-oxo-2,3,4,5-tetrahydro-1,5-benzothiazepin-3-yl)-(6E)-(2R,3R,4S,5R)-3,9,5-trihydroxy-2-methoxy-8,8-dimethylnon-6-enamid und
(6E)-(2R,3R,4S,5R)-8-Ethyl-3,4,5-trihydroxy-2-methoxy-N-((3R)-5-methyl-4-oxo-2,3,4,5-tetrahydro-1,5-benzothiazepin-3-yl)dec-6-enamid ausgewählt ist.

9. Produkt nach einem der Ansprüche 1 bis 7, **dadurch gekennzeichnet, daß** es unter:
(2R,3R,4S,5R,6E)-7-Cyclopantyl-3,4,5-trihydroxy-2-methoxy-N-[(3R)-5-methyl-4-oxo-2,3,4,5-tetrahydro-1,5-benzothiazepin-3-yl]hept-6-enamid;
(2R,3R,4S,5R,6E)-3,4,5-Trihydroxy-2-methoxy-N-[(3R)-5-methyl-4-oxo-2,3,4,5-tetrahydro-1,5-banzothiazepin-3-yl]undec-6-enamid;
2R,3R,4S,5R,6E)-7-Cyclohexyl-3,4,5-trihydroxy-2-methoxy-N-[(3R)-5-methyl-4-oxo-2,3,4,5-tetrahydro-1,5-benzothiazepin-3-yl]hept-6-enamid;
(2R,3R,4S,5R,6E)-7-(2-Fluorphanyl)-3,9,5-trihydroxy-2-methoxy-N-[(3R)-5-methyl-4-oxo-2,3,4,5-tetrahydro-1,5-benzothiazepin-3-yl]hept-6-enamid;
(2R,3R,4S,5R,6E)-7-(4-Fluorphenyl)-3,4,5-trihydroxy-2-methoxy-N-[(3R)-5-methyl-4-oxo-2,3,4,5-tetrahydro-1,5-benzothiazepin-3-yl]hept-6-enamid;
(2R,3R,4S,5R,6E)-3,4,5-Trihydroxy-2-methoxy-N-[(3R)-5-methyl-4-oxo-2,3,4,5-tetrahydro-1,5-benzothiazepin-3-yl]-7-phenylhept-6-enamid;
(2R,3R,4S,5R,6E)-3,4,5-Trihydroxy-2-methoxy-8,8-dimethyl-N-[(3R)-4-oxo-9-phenyl-2,3,4,5-tetrahydro-1,5-benzothiazepin-3-yl]non-6-enamid;
(2R,3R,4S,5R,6E)-N-[(3R)-9-(3-Cyanophenyl)-4-oxo-2,3,4,5-tetrahydro-1,5-benzothiazepin-3-yl]-3,4,5-trihydroxy-2-methoxy-8,8-dimethylnon-6-enamid;
(2R,3R,4S,5R,6E)-N-[(3R)-6-Fluor-4-oxo-2,3,4,5-tetrahydro-1,5-benzothiazepin-3-yl]-3,4,5-trihydroxy-2-methoxy-8,8-dimethylnon-6-enamid;
(2R,3R,4S,5R,6E)-3,4,5-Trihydroxy-2-methoxy-N-[(3R)-6-methoxy-4-oxo-2,3,4,5-tetrahydro-1,5-benzothiazepin-3-yl]-8,8-dimethylnon-6-enamid;
(2R,3R,4S,5R,6E)-3,4,5-Trihydroxy-2-methoxy-8,8-dimethyl-N-[(3R)-4-oxo-6-phenoxy-2,3,4,5-tetrahydro-1,5-benzothiazepin-3-yl]non-6-enamid und
(2R,3R,4S,5R,6Z)-3,4,5-Trihydroxy-2-methoxy-8,8-dimethyl-N-1(3R)-5-methyl-4-oxo-2,3,4,5-tetrahydro-1,5-benzothiazepin-3-yl]non-6-enamid ausgewählt ist.

10. Produkt nach einem der vorhergehenden Ansprüche, **dadurch gekennzeichnet, daß** es
a) in achiraler Form oder
b) in racemischer Form oder
c) in mit einem Stereoisomer angereicherter Form oder
d) in mit einem Enantiomer angereicherter Form vorliegt und gegebenenfalls versalzt ist.

11. Arzneimittel, **dadurch gekennzeichnet, daß** es ein Produkt der Formel (I) nach einem der Ansprüche 1 bis 7 oder ein Additionssalz dieses Produkts mit einer pharmazeutisch unbedenklichen Säure oder auch ein Hydrat oder ein Solvat des Produkts der Formel (I) enthält.

12. Pharmazeutische Zusammensetzung, **dadurch gekennzeichnet, daß** sie ein Produkt der Formel (I) nach einem der Ansprüche 1 bis 7 oder ein pharmazeutisch unbedenkliches Salz, ein Hydrat oder ein Solvat dieses Produkts sowie mindestens einen pharmazeutisch unbedenklichen Träger enthält.

13. Verwendung eines Produkts nach einem der Ansprüche 1 bis 7 zur Herstellung eines Arzneimittels zur Prävention oder Behandlung eines pathologischen Zustands.

14. Verwendung nach Anspruch 13, **dadurch gekennzeichnet, daß** es sich bei dem pathologischen Zustand um Krebs handelt.

15. Verfahren zur Herstellung eines Produkts der allgemeinen Formel (I) nach einem der Ansprüche 1 bis 7, **dadurch gekennzeichnet, daß** man ein Produkt der allgemeinen Formel (II): 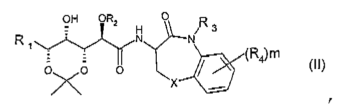 worin R₁, R₂, R₃, R₄, X und m die oben angegebene Bedeutung besitzen, zu einem Produkt der allgemeinen Formel (I) hydrolysiert.

16. verfahren nach Anspruch 15 zur Herstellung eines Produkts der allgemeinen Formel (I) nach einem der Ansprüche 1 bis 7, **dadurch gekennzeichnet, daß** man ein Produkt der allgemeinen Formel (III): 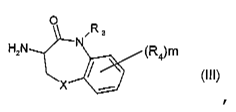 worin R₃, R₄, X und m die oben angegebene Bedeutung besitzen, mit einem Produkt der allgemeinen Formel (IV): 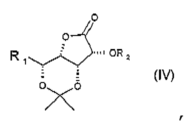 worin R₁ und R₂ die oben angegebene Bedeutung besitzen, zu einem Produkt der allgemeinen Formel (II) umsetzt.

17. Verfahren zur Herstellung eines Produkts der allgemeinen Formel (I) nach einem der Ansprüche 1 bis 7, **dadurch gekennzeichnet, daß** man ein Produkt der allgemeinen Formel (III): 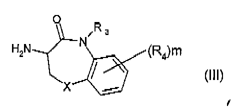 worin R₃, R₄, X und m die oben angegebene Bedeutung besitzen, und ein Produkt der allgemeinen Formel (V): 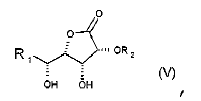 worin R₁ und R₂ die oben angegebene Bedeutung besitzen, zu einem Produkt der allgemeinen Formel (I) umsetzt.

18. Verfahren nach Anspruch 17 zur Herstellung eines Produkts der allgemeinen Formel (I) nach einem der Ansprüche 1 bis 7, **dadurch gekennzeichnet, daß** man ein Produkt der allgemeinen Formel (IV): 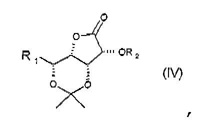 worin R₁ und R₂ die oben angegebene Bedeutung besitzen, zu einem Produkt der allgemeinen Formel (V): 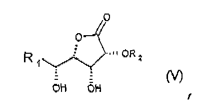 worin R₁ und R₂ die oben angegebene Bedeutung besitzen, hydrolysiert.

19. Verfahren nach Anspruch 17 zur Herstellung eines Produkts der allgemeinen Formel (I) nach einem der Ansprüche 1 bis 7, **dadurch gekennzeichnet, daß** man ein Produkt der allgemeinen Formel (VII): 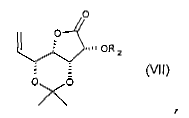 worin R₂ die oben angegebene Bedeutung besitzt, zu einem Produkt der allgemeinen Formel (VI): 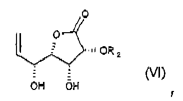 worin R₂ die oben angegebene Bedeutung besitzt, hydrolysiert und diese dann einer Metathese zu einer Verbindung der allgemeinen Formel (V): 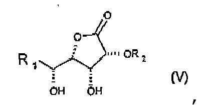 wofür R₁ für -CH=CH-R'₁ steht und R'₁ für eine (C1-C6)-Alkyl-, Cycloalkyl-, Heterocyclyl-, Aryl- oder Heteroarylgruppe steht, unterwirft.

20. Verfahren nach Anspruch 19 zur Herstellung eines Produkts der allgemeinen Formel (I) nach einem der Ansprüche 1 bis 7, **dadurch gekennzeichnet, daß** man ein Produkt der allgemeinen Formel (VII): 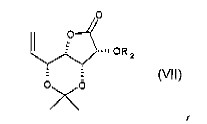 worin R₂ die oben angegebene Bedeutung besitzt, durch doppelte Dehydratisierung eines Produkts der allgemeinen Formel (VIII): 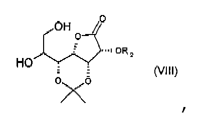 worin R₂ die oben angegebene Bedeutung besitzt, erhält.

21. Produkte der allgemeinen Formel (II) 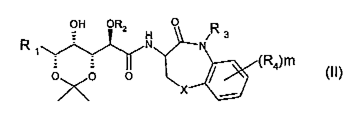 worin R₁, R₂, R₃, R₄, X und m die oben angegebene Bedeutung besitzen, mit Ausnahme derjenigen, für die R₁ für (E)-CH=CH-C(CH₃)₃ steht, R₂ für Methyl steht, X für S steht, m für 0 steht und R₃ für ein Wasserstoffatom, eine (3,5-Difluorphenyl)methylgruppe oder eine -CH₂-CH=CH₂-Gruppe steht.

22. Produkte der allgemeinen Formel (III) 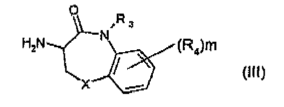 **dadurch gekennzeichnet, daß** X für S steht, R₃ für H, Methyl, Phenylmethyl oder (3,5-Difluorphenyl)-methyl steht und R₄ für F, Cl, Br, Phenyl, Cyanophenyl, Trifluormethyl, Methoxy oder Phenoxy steht oder m den Wert 0 hat, mit Ausnahme derjenigen, für die X für S steht, m für 0 steht und R₃ für ein Wasserstoffatom oder eine (3,5-Difluorphenyl)-methylgruppe steht, und mit Ausnahme derjenigen, für die X für S steht, R₃ für Phenylmethyl steht und R₄ für Trifluormethyl oder Methoxy steht.

23. Produkte der allgemeinen Formel (IV) und (V) 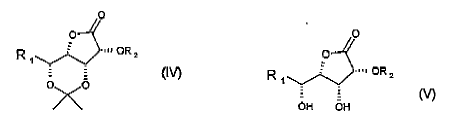 **dadurch gekennzeichnet, daß** R₂ für Methyl steht und R₁ für (E)-CH=CH-C₅H₉ oder (E)-CH=CH-C₆H₅, worin das Phenyl durch ein Fluoratom substituiert ist, steht.

24. Produkte der allgemeinen Formel (VI) 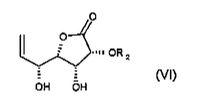 **dadurch gekennzeichnet, daß** R₂ für Methyl steht.
